(19) Europäisches Patentamt
European Patent Office
Office européen des brevets

(11) Veröffentlichungsnummer: **0 411 268 B1**

(12) # EUROPÄISCHE PATENTSCHRIFT

(45) Veröffentlichungstag der Patentschrift: **19.04.95**

(21) Anmeldenummer: **90110336.6**

(22) Anmeldetag: **31.05.90**

(51) Int. Cl.⁶: **C07D 321/12**, C07D 407/12, C07H 9/04, C07D 405/12, C07D 405/06, C07D 407/04, C07D 405/04, C07D 409/04, C07D 417/04, A61K 31/335

(54) **Dibenzo(1,5)dioxocin-5-one-Derivate, ihre Verwendung in Arzneimitteln und Verfahren zu ihrer Herstellung.**

(30) Priorität: **13.06.89 DE 3919255**

(43) Veröffentlichungstag der Anmeldung:
**06.02.91 Patentblatt 91/06**

(45) Bekanntmachung des Hinweises auf die Patenterteilung:
**19.04.95 Patentblatt 95/16**

(84) Benannte Vertragsstaaten:
**AT BE CH DE DK ES FR GB GR IT LI LU NL SE**

(56) Entgegenhaltungen:

**TETRAHEDRON LETTES, Nr. 45, November 1974, Seiten 3941-3942, Oxford, GB; T.SASSA et al.: "Structure of penicillide, a new metabolite produced by aPenicillium SP."**

(73) Patentinhaber: **BAYER AG**

**D-51368 Leverkusen (DE)**

(72) Erfinder: **Frobel, Klaus, Dr.**
**Paul-Ehrlich-Strasse 9**
**D-5600 Wuppertal 1 (DE)**
Erfinder: **Lenfers, Jan-Bernd, Dr.**
**Hubertusallee 9**
**D-5600 Wuppertal 1 (DE)**
Erfinder: **Fey, Peter, Dr.**
**Ursulastrasse 14**
**D-5600 Wuppertal 1 (DE)**
Erfinder: **Knorr, Andreas, Dr.**
**Trillser Graben 10**
**D-4006 Erkrath 2 (DE)**
Erfinder: **Stasch, Johannes-Peter, Dr.**
**Schneeewittchenweg 37**
**D-5600 Wuppertal 1 (DE)**
Erfinder: **Müller, Hartwig, Dr.**
**Steinstrasse 15**
**D-5620 Velbert 15 (DE)**
Erfinder: **Bischoff, Erwin**
**Pahlkestrasse 73**
**D-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 411 268 B1

Erfinder: **Dellweg, Hans-Georg, Dr.**
**In den Birken 46**
**D-5600 Wuppertal 1 (DE)**

## Beschreibung

Die vorliegende Erfindung betrifft 7H-Dibenzo[1,5]-dioxocin-5-one, ihre Verwendung in Arzneimitteln und Verfahren zu ihrer Herstellung, insbesondere ihre Verwendung in kreislaufwirksamen Arzneimitteln.

In Tetrahedron Letters Vol. 45, 3941-3942 (1974) ist bereits formelmäßig ein Naturstoff mit dem Namen Penicillide erwähnt, dessen Monomethylether eine keimungshemmende Wirkung auf Chinakohl besitzen soll. Als Herstellungsverfahren wird die Extraktion aus dem Mycel eines taxonomisch nicht eindeutig beschriebenen Pilzes des Spezies Penicillium angegeben. Aufgrund der unzureichenden Offenbarung dieser Publikation ist das Herstellungsverfahren nicht reproduzierbar und somit dieser Naturstoff der Öffentlichkeit nicht zugänglich.

Durch die vorliegende Erfindung werden erstmals reproduzierbare Verfahren zur Herstellung dieser Verbindungen bereitgestellt und gleichzeitig wird ihre erste pharmazeutische Verwendung beansprucht.

Die vorliegende Erfindung betrifft Verbindungen der allgemeinen Formel (I),

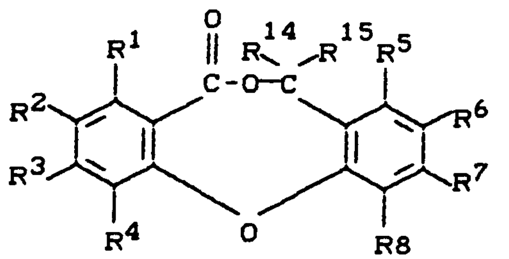

(I)

in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 12 Kohlenstoffatomen stehen, die gegebenenfalls durch Halogen, Azido, Imino, hydroxysubstituiertes Imino, Hydroxy, Cyano, Cycloalkyl mit 3 bis 8 Kohlenstoffatomen, oder durch einen 3- bis 7-gliedrigen, gesättigten oder ungesättigten Heterocyclus mit bis zu 4 Heteroatomen aus der Reihe Stickstoff, Schwefel oder Sauerstoff, oder durch Aryl oder Aryloxy mit 6 bis 10 Kohlenstoffatomen substituiert sind, die ihrerseits bis zu 3-fach gleich oder verschieden durch Halogen, Nitro, Phenyl, Phenoxy, Cyano, geradkettiges oder verzweigtes Alkyl, Alkoxy oder Alkoxycarbonyl mit jeweils bis zu 8 Kohlenstoffatomen substituiert sein können, oder durch einen Rest der Formel

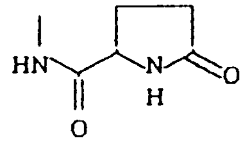

oder durch eine Gruppe der Formel $-NR^9R^{10}$, $-COR^{11}$ oder $-OR^{12}$ substituiert sind,

worin

$R^9$ und $R^{10}$ gleich oder verschieden sind und jeweils Wasserstoff, geradkettiges oder verzweigtes Alkyl mit bis zu 10 Kohlenstoffatomen oder Aryl mit 6 bis 10 Kohlenstoffatomen oder eine Gruppe der Formel $-S(O)_pR^{13}$ bedeuten,

$R^{11}$ Wasserstoff, Hydroxy, geradkettiges oder verzweigtes Alkyl oder Alkoxy mit bis zu 10 Kohlenstoffatomen, Phenoxy, Aryl mit 6 bis 10 Kohlenstoffatomen oder die Gruppe $-NR^9R^{10}$ bedeutet,

worin
$R^9$ und $R^{10}$ die oben angegebene Bedeutung haben,

$R^{12}$ Wasserstoff, Cycloalkyl mit 3 bis 8 Kohlenstoffatomen, Formyl, Acyl mit bis zu 8 Kohlenstoffatomen, Tetrahyropyranyl, Trifluormethoxy oder eine Gruppe der Formel $-S(O)_pR^{13}$ bedeutet,
worin

p eine Zahl 1 oder 2 bedeutet

$R^{13}$ - geradkettiges oder verzweigtes Alkyl mit bis zu 8 Kohlenstoffatomen oder die Gruppe $-NR^9R^{10}$ bedeutet,
worin
$R^9$ und $R^{10}$ die oben angegebene Bedeutung haben,

oder

$R^{12}$ geradkettiges oder verzweigtes Alkyl oder Alkenyl mit bis zu 10 Kohlenstoffatomen bedeutet, das gegebenenfalls durch Hydroxy, Halogen, Cycloalkyl mit 3 bis 8 Kohlenstoffatomen, Alkoxy oder Alkoxycarbonyl mit bis zu 8 Kohlenstoffatomen, oder durch Aryl mit 6 bis 10 Kohlenstoffatomen substituiert ist, das seinerseits durch Halogen, Hydroxy, Alkyl, Alkoxy oder Alkoxycarbonyl mit jeweils bis zu 8 Kohlenstoffatomen, Nitro oder Cyano substituiert sein kann,
oder durch einen 3- bis 7-gliedrigen, gesättigten oder ungesättigten Heterocyclus mit bis zu 4 Heteroatomen aus der Reihe Schwefel, Sauerstoff oder Stickstoff oder durch eine Gruppe der Formel $-NR^9R^{10}$, $-COR^{11}$ oder $-OR^{12}$ substituiert ist,
worin
$R^9$, $R^{10}$, $R^{11}$ und $R^{12}$ die oben angegebene Bedeutung haben,
oder durch einen Rest der Formel

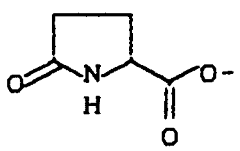 , 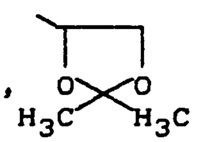 oder 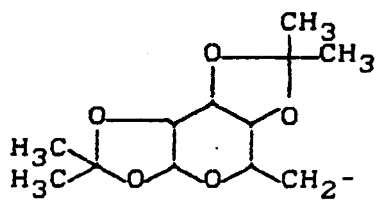

substituiert ist,
oder Aryl mit 6 bis 10 Kohlenstoffatomen bedeutet, das gegebenenfalls durch Halogen, Hydroxy, Nitro oder Cyano substituiert ist,

oder

$R^1$, $R^2$, $R^3$, $R^4$, $R^5$, $R^6$, $R^7$ und $R^8$ gleich oder verschieden sind und jeweils

- für Halogen, Cyano, Hydroxy, Nitro,
- für einen 3- bis 7-gliedrigen Heterocyclus mit bis zu 4 Heteroatomen aus der Reihe Schwefel, Sauerstoff oder Stickstoff, Pyrid-3-yl-methojodid, oder für Aryl mit 6 bis 10 Kohlenstoffatomen stehen, die ihrerseits durch Halogen, Cyano, Phenyl, Nitro, geradkettiges oder verzweigtes Alkyl oder Alkoxy mit jeweils bis zu 8 Kohlenstoffatomen oder Hydroxy substituiert sein können, oder
- für Cycloalkyenyl mit 3 bis 8 Kohlenstoffatomen stehen,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,

oder $R^1$ und $R^2$, $R^2$ und $R^3$, $R^3$ und $R^4$, $R^5$ und $R^6$, $R^6$ und $R^7$ oder $R^7$ und $R^8$ jeweils gemeinsam einen gesättigten oder ungesättigten 5- bis 7-gliedrigen Carbocyclus bilden, der gebenenfalls durch Nitro, Cyano, Hydroxy, geradkettiges oder verzweigtes Alkyl mit bis zu 8 Kohlenstoffatomen, oder durch eine Gruppe der Formel $-NR^9R^{10}$, $-COR^{11}$ oder $-OR^{12}$ substituiert ist,

$R^{14}$ und $R^{15}$ gleich oder verschieden sind und jeweils - Wasserstoff, geradkettiges oder verzweigtes Alkyl mit bis zu 10 Kohlenstoffatomen bedeuten, das gegebenenfalls durch Halogen, Nitro, Cyano, Hydroxy oder eine Gruppe der Formel $-NR^9R^{10}$, $-COR^{11}$ oder $-OR^{12}$ substituiert ist,

worin

$R^9$, $R^{10}$, $R^{11}$ und $R^{12}$ die oben angegebene Bedeutung haben,

- Aryl mit 6 bis 10 Kohlenstoffatomen bedeuten, das gegebenenfalls durch Nitro, Cyano, Halogen, Alkyl, Alkoxy oder Alkoxycarbonyl mit bis zu 8 Kohlenstoffatomen oder durch eine Gruppe der Formel $-NR^9R^{10}$ substituiert ist,

  worin

  $R^9$ und $R^{10}$ die oben angegebene Bedeutung haben

  oder $R^{14}$ und $R^{15}$ gemeinsam einen 5- bis 7-gliedrigen, gesättigten oder ungesättigten Carbocyclus bilden

und deren physiologisch unbedenklichen Salze, zur Anwendung bei der Bekämpfung von Erkrankungen.

Es wurde gefunden, daß die erfindungsgemäßen Verbindungen überraschenderweise eine antihypertensive Wirkung haben und eine starke Stimulation auf die ANP-Freisetzung ausüben und somit geeignet sind bei der Bekämpfung der Hypertonie und bei Digitalisvergiftungen, sowie bei der Behandlung von Herzinsuffiziens, Herzrythmusstörungen und Ödemen.

Auch die physiologisch unbedenklichen Säureadditionssalze der Verbindungen der allgemeinen Formel (I) sowie die Racemformen, die Antipoden und die Diastereomerengemische sind bevorzugt geeignet für die neuartige Verwendung.

Bevorzugt sind Verbindungen der allgemeinen Formel (I),
in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 10 Kohlenstoffatomen stehen, die gegebenenfalls bis zu 4-fach gleich oder verschieden durch folgende Reste wie Fluor, Chlor, Brom, Azido, Imino, hydroxysubstituiertes Imino, Hydroxy, Cyano, Cyclopropyl, Cyclopentyl, Cyclohexyl, oder durch Pyrryl, Morpholino, Piperidyl oder Oxiranyl oder durch Phenyl substituiert sind, die ihrerseits bis zu 3-fach gleich oder verschieden durch Fluor, Chlor, Brom, Jod, Nitro, Phenyl, Phenoxy, Cyano, geradkettiges oder verzweigtes Alkyl, Alkoxy oder Alkoxycarbonyl mit jeweils bis zu 6 Kohlenstoffatomen substituiert sein können, oder durch einen Rest der Formel

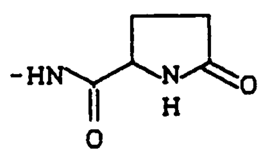

  oder durch eine Gruppe der Formel $-NR^9R^{10}$, $-COR^{11}$ oder $-OR^{12}$ substituiert sind,

  worin

$R^9$ und $R^{10}$ gleich oder verschieden sind und jeweils Wasserstoff, geradkettiges oder verzweigtes Alkyl mit bis zu 8 Kohlenstoffatomen, Phenyl oder eine Gruppe der Formel $S(O)_pR^{13}$ bedeuten,

$R^{11}$ Wasserstoff, Hydroxy, geradkettiges oder verzweigtes Alkyl oder Alkoxy mit bis zu 8 Kohlenstoffatomen, Phenoxy, Phenyl oder die Gruppe $-NR^9R^{10}$ bedeutet,

worin

$R^9$ und $R^{10}$ die oben angegebene Bedeutung haben,

$R^{12}$ Wasserstoff, Cyclopropyl, Cyclobutyl, Cyclopentyl, Cyclohexyl, Cycloheptyl, Formyl, Acyl mit bis zu 6 Kohlenstoffatomen, Tetrahydropyranyl, Trifluormethoxy oder eine Gruppe der Formel $-S(O)_p-R^{13}$ bedeutet,

worin

p eine Zahl 1 oder 2 bedeutet,

$R^{13}$ - geradkettiges oder verzweigtes Alkyl mit bis zu 6 Kohlenstoffatomen oder die Gruppe $-NR^9R^{10}$ bedeutet,

worin

$R^9$ und $R^{10}$ die oben angegebene Bedeutung haben

oder

$R^{12}$ geradkettiges oder verzweigtes Alkyl oder Alkenyl mit bis zu 8 Kohlenstoffatomen bedeutet, das gegebenenfalls bis zu 4-fach gleich oder verschieden durch folgende Reste wie Hydroxy, Fluor, Chlor, Brom, Cyclopropyl, Cyclopentyl, Cyclohexyl, Alkoxy oder Alkoxycarbonyl mit bis zu 6 Kohlenstoffatomen, oder durch Phenyl substituiert ist, das seinerseits durch Fluor, Chlor, Brom, Hydroxy, Alkyl, Alkoxy oder Alkoxycarbonyl mit jeweils bis zu 6 Kohlenstoffatomen, Nitro oder Cyano substituiert sein kann, oder durch Oxiranyl, Pyrimidyl, Pyridyl oder durch eine Gruppe der Formel $-NR^9R^{10}$, $-COR^{11}$ oder $-OR^{12}$ substituiert ist,

worin

$R^9$, $R^{10}$, $R^{11}$ und $R^{12}$ die oben angegebene Bedeutung haben,

oder durch einen Rest der Formel

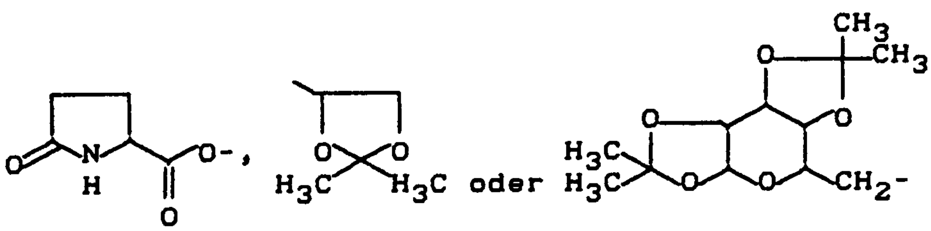

substituiert ist,

oder

$R^1$, $R^2$, $R^3$, $R^4$, $R^5$, $R^6$, $R^7$ und $R^8$ gleich oder verschieden sind und jeweils

- für Fluor, Chlor, Brom, Jod, Cyano, Hydroxy, Nitro, Pyrid-3-yl-methojodid, 2,3-Dihydrofuryl, Furyl, Pyridyl, Thienyl, Dihydropyranyl, Thiazolyl, Oxiranyl oder für Phenyl stehen, die gegebenenfalls durch Fluor, Chlor, Brom, Phenyl, geradkettiges oder verzweigtes Alkyl oder Alkoxy mit bis zu 6 Kohlenstoffatomen oder Hydroxy substituiert sind, oder
- für Cyclobutenyl, Cyclopentenyl oder Cyclohexenyl stehen,
- für eine Gruppe der Formel $-NR^9R^{10}$, $-COR^{11}$ oder $-OR^{12}$ stehen,

  worin

  $R^9$, $R^{10}$, $R^{11}$ und $R^{12}$ die oben angegebene Bedeutung haben,

  oder $R^1$ und $R^2$, $R^2$ und $R^3$, $R^3$ und $R^4$, $R^5$ und $R^6$, $R^6$ und $R^7$ oder $R^7$ und $R^8$ gemeinsam für Phenyl, Cyclopentyl oder Cyclohexyl stehen,

$R^{14}$ und $R^{15}$ gleich oder verschieden sind und jeweils

- Wasserstoff, geradkettiges oder verzweigtes Alkyl mit bis zu 8 Kohlenstoffatomen bedeuten, das gegebenenfalls durch Fluor, Chlor, Brom, Cyano, Hydroxy oder durch eine Gruppe der Formel $-NR^9R^{10}$, $-COR^{11}$ oder $-OR^{12}$ substituiert ist,

  worin

  $R^9$, $R^{10}$, $R^{11}$ und $R^{12}$ die oben angegebene Bedeutung haben,

oder

- Phenyl bedeuten, das gegebenenfalls durch Nitro, Cyano, Fluor, Chlor, Brom, Alkyl, Alkoxy oder Alkoxycarbonyl mit bis zu 6 Kohlenstoffatomen oder durch die Gruppe der Formel $-NR^9R^{10}$ substituiert ist,

  worin

  $R^9$ und $R^{10}$ die oben angegebene Bedeutung haben

und deren physiologisch unbedenklichen Salze.

Besonders bevorzugt seien Verbindungen der allgemeinen Formel (I) genannt, in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 8 Kohlenstoffatomen stehen, die gegebenenfalls bis zu 4-fach gleich oder verschieden durch folgende Reste wie Fluor, Chlor, Brom, Jod, Azido, Imino, hydroxysubstituiertes Imino, Hydroxy, Cyclopropyl, Cyclopentyl, Cyclohexyl, Morpholino, Piperidyl, Pyrryl oder durch Oxiranyl oder Phenyl substituiert sind, die ihrerseits bis zu 3-fach gleich oder verschieden durch Fluor, Chlor, Phenyl, Phenoxy oder durch geradkettiges oder verzweigtes Alkyl oder Alkoxy mit bis zu 4 Kohlenstoffatomen substituiert sein können, oder

  durch einen Rest der Formel

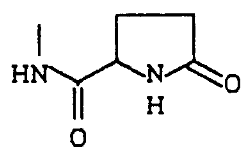

  oder durch eine Gruppe der Formel $-NR^9R^{10}$, $-COR^{11}$ oder $-OR^{12}$ substituiert sind,

worin

$R^9$ und $R^{10}$ gleich oder verschieden sind und jeweils

- Wasserstoff, geradkettiges oder verzweigtes Alkyl mit bis zu 6 Kohlenstoffatomen, Phenyl oder eine Gruppe der Formel $-S(O)_pR^{13}$ bedeuten,

$R^{11}$ Wasserstoff, Hydroxy, Trifluormethyl, geradkettiges oder verzweigtes Alkyl oder Alkoxy oder mit bis zu 6 Kohlenstoffatomen, Phenoxy, Phenyl oder die Gruppe $-NR^9R^{10}$ bedeutet,

worin

$R^9$ und $R^{10}$ die oben angegebene Bedeutung haben,

$R^{12}$ Wasserstoff, Cyclopropyl, Cyclopentyl, Cyclohexyl, Cycloheptyl, Formyl, Acyl mit bis zu 4 Kohlenstoffatomen, Tetrahydropyranyl, Trifluormethoxy oder eine Gruppe der Formel $-S(O)_p-R^{13}$ bedeutet,

worin

p eine Zahl 1 oder 2 bedeutet,

$R^{13}$ - geradkettiges oder verzweigtes Alkyl mit bis zu 4 Kohlenstoffatomen bedeutet,

oder geradkettiges oder verzweigtes Alkyl oder Alkenyl mit bis zu 6 Kohlenstoffatomen bedeutet, das gegebenenfalls bis zu 3-fach gleich oder verschieden durch folgende Reste wie Hydroxy, Fluor, Chlor, Brom, Cyclopropyl, Cyclopentyl, Cyclohexyl, Alkoxy oder Alkoxycarbonyl mit bis zu 4 Kohlenstoffatomen oder durch Phenyl substituiert ist, das seinerseits durch Fluor, Chlor, Hydroxy oder Alkoxy mit bis zu 4 Kohlenstoffatomen substituiert sein kann, oder durch Oxiranyl oder durch eine Gruppe der Formel $-NR^9R^{10}$, $-COR^{11}$ oder $-OR^{12}$ substituiert ist,

worin

$R^9$, $R^{10}$, $R^{11}$ und $R^{12}$ die oben angegebene Bedeutung haben,
oder durch einen Rest der Formel

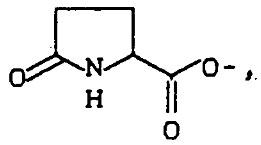,
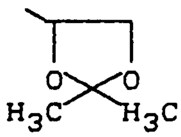 oder 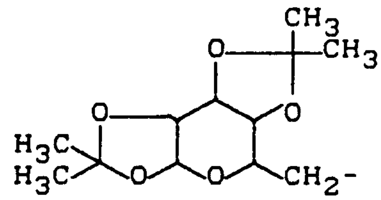

substituiert ist,
oder

$R^1$, $R^2$, $R^3$, $R^4$, $R^5$, $R^6$, $R^7$ und $R^8$ gleich oder verschieden sind und jeweils

- für Fluor, Chlor, Brom, Jod, Hydroxy oder Nitro stehen oder für Phenyl, Pyrid-3-yl-methojodid, 2,3-Dihydrofuryl, Pyrryl, Pyridyl, Thienyl, Dihydropyranyl, Thiazolyl oder Oxiranyl stehen, die ihrerseits durch Fluor, Chlor, Methyl, Isopropyl, Phenyl oder Ethoxy substituiert sein können,
- für Cyclobutenyl, Cyclopentenyl oder Cyclohexenyl stehen,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,

$R^{14}$ und $R^{15}$ gleich oder verschieden sind und Wasserstoff, geradkettiges oder verzweigtes Alkyl mit bis zu 6 Kohlenstoffatomen oder Phenyl bedeuten,

und deren physiologisch unbedenklichen Salze.

Die Erfindung betrifft insbesondere neue Verbindungen der allgemeinen Formel (Ia),

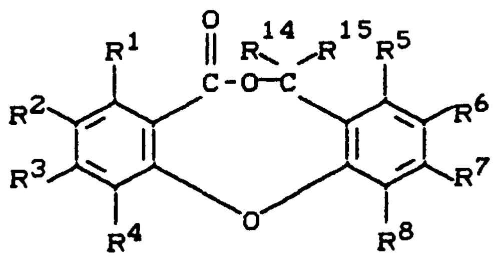

(Ia)

in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 12 Kohlenstoffatomen stehen, die gegebenenfalls durch Halogen, Azido, Imino, hydroxysubstituiertes Imino, Hydroxy, Cyano, Cycloalkyl mit 3 bis 8 Kohlenstoffatomen, oder durch einen 3- bis 7-gliedrigen, gesättigten oder ungesättigten Heterocyclus mit bis zu 4 Heteroatomen aus der Reihe Stickstoff, Schwefel oder Sauerstoff, oder durch Aryl oder Aryloxy mit 6 bis 10 Kohlenstoffatomen substituiert sind,

die ihrerseits bis zu 3-fach gleich oder verschieden durch Halogen, Nitro, Phenyl, Phenoxy, Cyano, geradkettiges oder verzweigtes Alkyl, Alkoxy oder Alkoxycarbonyl mit jeweils bis zu 8 Kohlenstoffatomen substituiert sein können, oder durch einen Rest der Formel

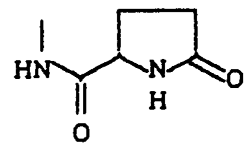

oder durch eine Gruppe der Formel $-NR^9R^{10}$, $-COR^{11}$ oder $-OR^{12}$ substituiert sind,
worin

$R^9$ und $R^{10}$ gleich oder verschieden sind und jeweils Wasserstoff, geradkettiges oder verzweigtes Alkyl mit bis zu 10 Kohlenstoffatomen oder Aryl mit 6 bis 10 Kohlenstoffatomen oder eine Gruppe der Formel $-S(O)_pR^{13}$ bedeuten,

$R^{11}$ Wasserstoff, Hydroxy, geradkettiges oder verzweigtes Alkyl oder Alkoxy mit bis zu 10 Kohlenstoffatomen, Phenoxy, Aryl mit 6 bis 10 Kohlenstoffatomen oder die Gruppe $-NR^9R^{10}$ bedeutet,
worin
$R^9$ und $R^{10}$ die oben angegebene Bedeutung haben,

$R^{12}$ Wasserstoff, Cycloalkyl mit 3 bis 8 Kohlenstoffatomen, Formyl, Acyl mit bis zu 8 Kohlenstoffatomen, Tetrahyropyranyl, Trifluormethoxy oder eine Gruppe der Formel $-S(O)_pR^{13}$ bedeutet,
worin

p eine Zahl 1 oder 2 bedeutet

$R^{13}$ - geradkettiges oder verzweigtes Alkyl mit bis zu 8 Kohlenstoffatomen oder die Gruppe $-NR^9R^{10}$ bedeutet,
worin
$R^9$ und $R^{10}$ die oben angegebene Bedeutung haben,

oder

$R^{12}$ geradkettiges oder verzweigtes Alkyl oder Alkenyl mit bis zu 10 Kohlenstoffatomen bedeutet, das gegebenenfalls durch Hydroxy, Halogen, Cycloalkyl mit 3 bis 8 Kohlenstoffatomen, Alkoxy oder Alkoxycarbonyl mit bis zu 8 Kohlenstoffatomen, oder durch Aryl mit 6 bis 10 Kohlenstoffatomen substituiert ist, das seinerseits durch Halogen, Hydroxy, Alkyl, Alkoxy oder Alkoxycarbonyl mit jeweils bis zu 8 Kohlenstoffatomen, Nitro oder Cyano substituiert sein kann,
oder durch einen 3- bis 7-gliedrigen gesättigten oder ungesättigten Heterocyclus mit bis zu 4 Heteroatomen aus der Reihe Schwefel, Sauerstoff oder Stickstoff oder durch eine Gruppe der Formel $-NR^9R^{10}$, $-COR^{11}$ oder $-OR^{12}$ substituiert ist,

worin
$R^9$, $R^{10}$, $R^{11}$ und $R^{12}$ die oben angegebene Bedeutung haben,
oder durch einen Rest der Formel

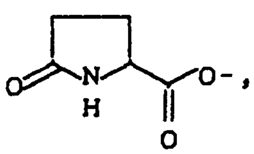 , 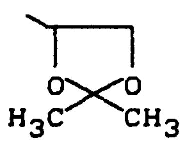 oder 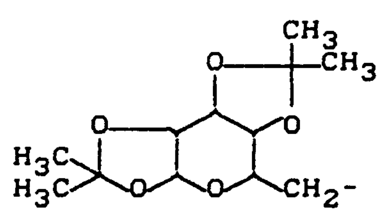

substituiert ist,
oder Aryl mit 6 bis 10 Kohlenstoffatomen bedeutet, das gegebenenfalls durch Halogen, Hydroxy, Nitro oder Cyano substituiert ist,
oder

$R^1$, $R^2$, $R^3$, $R^4$, $R^5$, $R^6$, $R^7$ und $R^8$ gleich oder verschieden sind und

- für Halogen, Cyano, Hydroxy, Nitro, oder
- für einen 3- bis 7-gliedrigen Heterocyclus mit bis zu 4 Heteroatomen aus der Reihe Schwefel, Sauerstoff oder Stickstoff,

Pyrid-3-yl-methojodid, oder für Aryl mit 6 bis 10 Kohlenstoffatomen stehen die ihrerseits durch Halogen, Cyano, Phenyl, Nitro, geradkettiges oder verzweigtes Alkyl oder Alkoxy mit jeweils bis zu 8 Kohlenstoffatomen oder Hydroxy substituiert sein können, oder

- für Cycloalkenyl mit 3 bis 8 Kohlenstoffatomen stehen,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,

oder

$R^1$ und $R^2$, $R^2$ und $R^3$, $R^3$ und $R^4$, $R^5$ und $R^6$, $R^6$ und $R^7$ oder $R^7$ und $R^8$ jeweils gemeinsam einen gesättigten oder ungesättigten 5- bis 7-gliedrigen Carbocyclus bilden, der gegebenenfalls durch Nitro, Cyano, Hydroxy, geradkettiges oder verzweigtes Alkyl mit bis zu 8 Kohlenstoffatomen, oder durch eine Gruppe der Formel $-NR^9R^{10}$, $-COR^{11}$ oder $-OR^{12}$ substituiert ist,

$R^{14}$ und $R^{15}$ gleich oder verschieden sind und jeweils - Wasserstoff, geradkettiges oder verzweigtes Alkyl mit bis zu 10 Kohlenstoffatomen bedeuten, das gegebenenfalls durch Halogen, Nitro, Cyano, Hydroxy oder eine Gruppe der Formel $-NR^9R^{10}$, $-COR^{11}$ oder $-OR^{12}$ substituiert ist,
worin
$R^9$, $R^{10}$, $R^{11}$ und $R^{12}$ die oben angegebene Bedeutung haben,

- oder Aryl mit 6 bis 10 Kohlenstoffatomen bedeuten, das gegebenenfalls durch Nitro, Cyano, Halogen, Alkyl, Alkoxy oder Alkoxycarbonyl mit bis zu 8 Kohlenstoffatomen oder durch eine Gruppe der Formel $-NR^9R^{10}$ substituiert ist,
  worin
  $R^9$ und $R^{10}$ die oben angegebene Bedeutung haben,

oder $R^{14}$ und $R^{15}$ gemeinsam einen 5- bis 7-gliedrigen, gesättigten oder ungesättigten Carbocyclus bilden,

mit der Maßgabe, daß

a) $R^8$ nicht Hydroxy, Methoxy oder Acetyl bedeuten darf, wenn $R^1$ für Methoxy, $R^3$, $R^4$, $R^5$, $R^7$, $R^{14}$ und $R^{15}$ für Wasserstoff, $R^6$ für Methyl und $R^2$ für die Gruppe

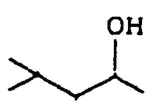

steht,

b) $R^5$ und $R^7$ nicht Brom und $R^8$ nicht Hydroxy bedeuten dürfen, wenn $R^1$ für Methoxy, $R^3$, $R^4$, $R^{14}$ und $R^{15}$ für Wasserstoff, $R^6$ für Methyl und $R^2$ für die Gruppe

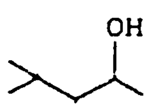

steht,

oder

c) $R^8$ nicht Methoxy bedeuten darf, wenn $R^1$ für Methoxy, $R^3$, $R^4$, $R^5$, $R^7$, $R^{14}$ und $R^{15}$ für Wasserstoff, $R^6$ für Methyl und $R^2$ für die Gruppe

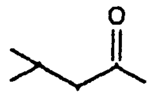

steht,
oder
d) $R^1$ nicht Methoxy bedeuten darf, wenn
$R^3$, $R^4$, $R^5$, $R^6$, $R^{14}$ und $R^{15}$ für Wasserstoff und $R^2$ für die Gruppe der Formel

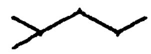

steht.

Die neuen erfindundunggemäßen Verbindungen der Formel (I) und der Formel (Ia) zeigen ein nicht vorhersehbares, wertvolles pharmakologisches Wirkspektrum. Sie beeinflussen die ANP-Freisetzung, die Kontraktionskraft des Herzens, den Tonus der glatten Muskulatur sowie den Elektrolyt- und Flüssigkeitshaushalt und wirken außerdem entweder partiell oder vollständig digitalisantagonistisch oder digitalisagonistisch.

Sie können deshalb in Arzneimitteln des pathologisch veränderten Blutdrucks, insbesondere als Antihypertensiva, zur Behandlung der Herzinsuffizienz, sowie als Koronartherapeutika oder Therapeutikum bei Digitalisvergiftungen eingesetzt werden.

Darüberhinaus können sie zur Behandlung von Herzrhythmusstörungen, Niereninsuffizienz, Leberzirrhose, Aszites, Lungenödem, Hirnödem, Schwangerschaftsödem oder Glaukom eingesetzt werden.

Die erfindungsgemäßen Verbindungen der allgemeinen Formeln (I) und (Ia) existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Physiologisch unbedenkliche Salze können Salze der erfindungsgemäßen Verbindungen der allgemeinen Formeln (I) und (Ia) mit anorganischen oder organischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen der allgemeinen Formel (I) bzw. (Ia) erhält man, indem man
[A] Verbindungen der allgemeinen Formel (II)

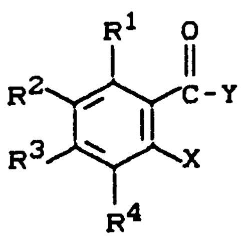

(II)

in welcher
$R^1$, $R^2$, $R^3$ und $R^4$ die oben angegebene Bedeutung haben,
X - für Fluor, Chlor, Brom oder Jod steht
und
Y - für ($C_1$-$C_6$)-Alkoxy oder Aryloxy mit 6 bis 10 Kohlenstoffatomen steht,
mit Verbindungen der allgemeinen Formel (III)

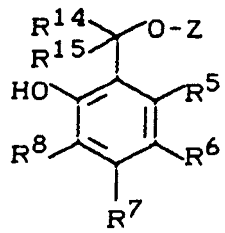

(III)

in welcher
$R^5$, $R^6$, $R^7$, $R^8$, $R^{14}$ und $R^{15}$ die oben angegebene Bedeutung haben
und

Z - für eine typische Hydroxylschutzgruppe, wie beispielsweise Tetrahydropyranyl steht,

oder Verbindungen der allgemeinen Formel (IIa)

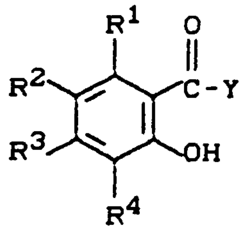

(IIa)

in welcher $R^1$, $R^2$, $R^3$, $R^4$ und Y die oben angegebene Bedeutung haben,
mit Verbindungen der allgemeinen Formel (IIIa)

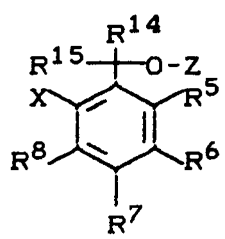

(IIIa)

in welcher
$R^5$, $R^6$, $R^7$, $R^8$, $R^{14}$, $R^{15}$, X und Z die oben angegebene Bedeutung haben,
unter Abspaltung von Halogenwasserstoffsäuren, wie beispielsweise Bromwasserstoff, zu Verbindungen der allgemeinen Formel (IV)

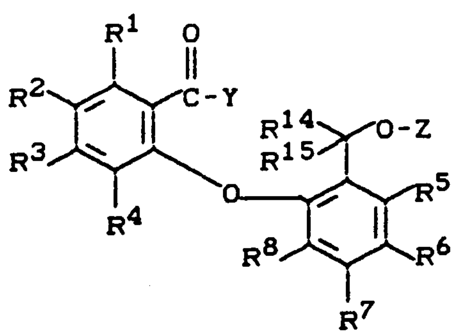

(IV)

in welcher
$R^1$, $R^2$, $R^3$, $R^4$, $R^5$, $R^6$, $R^7$, $R^8$, $R^{14}$, $R^{15}$, Y und Z die oben angegebene Bedeutung haben,
in inerten Lösemitteln kondensiert, anschließend die Hydroxylgruppe nach üblicher Methode deblockiert und unter Wasserabspaltung cyclisiert,

wobei die Substituenten $R^1$, $R^2$, $R^3$, $R^4$, $R^5$, $R^6$, $R^7$, $R^8$, $R^{14}$ und $R^{15}$ sowohl vor der Kondensation in die Verbindungen der allgemeinen Formeln (II), (IIa), (III) und (IIIa) als auch nach der Cyclisierung in die Verbindungen der allgemeinen Formel (IV) nach bekannten Methoden, wie beispielsweise durch Alkylierung, Acylierung, Substitution, Addition, Eliminierung, Umlagerung, Oxidation, Radikalreaktion oder Reduktion eingeführt und gegebenenfalls anschließend in andere funktionelle Gruppen überführt werden können,

oder indem man

[B] ausgehend von dem Naturstoff der Formel (Ib)

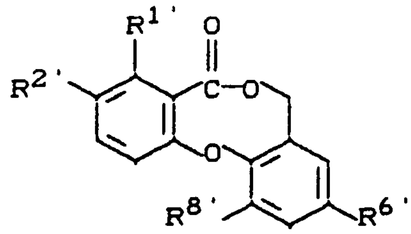

(Ib)

(nachfolgend "Penicillide" genannt) in welcher

$R^{1'}$ für Methoxy steht,

$R^{2'}$ für die Gruppe

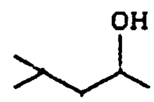

steht,

$R^{6'}$ für Methyl steht,

und

$R^{8'}$ für Hydroxy steht

nach den unter Verfahren [A] erwähnten und im folgenden beispielhaft aufgeführten üblichen Methoden, wie beispielsweise Umlagerung, Alkylierung, Acylierung, Addition, Eliminierung, Oxidation, Radikalreaktion oder Reduktion, in inerten Lösemitteln, gegebenenfalls in Anwesenheit von Hilfsstoffen, wie beispielsweise Basen, Säuren, Katalysatoren oder aktivierenden Reagenzien, die Substituenten $R^1$, $R^2$, $R^3$, $R^4$, $R^5$, $R^6$, $R^7$, $R^8$, $R^{14}$ und $R^{15}$ einführt und diese wie auch die Substituenten $R^{1'}$, $R^{2'}$, $R^{6'}$ und $R^{8'}$ in andere funktionelle Gruppen überführt.

Als geeignete übliche Methoden seien folgende Reaktionen beispielhaft angeführt:

a) der Naturstoff der Formel (Ib) oder geeignete Derivate, die durch die in Verfahren [A] beschriebene Methode dargestellt wurden, werden einfach oder mehrfach mit Verbindungen der allgemeinen Formel (V)

R-D (V)

in welcher

R dem Bedeutungsumfang von einem der oben aufgeführten Substituenten $R^1$ - $R^{15}$ entspricht, aber nicht für Wasserstoff steht,

und

D eine Abgangsgruppe wie beispielsweise Chlor, Brom, Jod, $-SO_2-CH_3$ oder $-SO_2-(C_6H_5)-p-CH_3$ bedeutet,

in inerten Lösemitteln, gegebenenfalls in Anwesenheit von Hilfsstoffen wie beispielsweise Basen, Säuren oder Katalysatoren umgesetzt, oder

b) Verbindungen der Formel (Ib) oder geeignete Derivate werden beispielsweise mit Aminen, Stickstoffwasserstoffsäure und Azodicarbonsäurediethylester, Essigsäure, Essigsäureanhydrid, Tetrahydropyranyl, Thionylchlorid, Methansulfonsäurechlorid, 2-Pyrrilidon-5-carbonsäure oder Hydroxylamin in inerten Lösemitteln, gegebenenfalls in Anwesenheit von Hilfsstoffen wie Basen oder Katalysatoren umgesetzt, oder

c) mit Grignard-Reagenzien der allgemeinen Formel (VI)

R-Mg-Br (VI)

in welcher
R die oben angegebene Bedeutung hat,
in inerten Lösemitteln umgesetzt, oder
d) mit Verbindungen der allgemeinen Formel (VII)

E-Hal (VII)

in welcher
Hal für Fluor, Chlor, Brom oder Jod steht
und
E - für einen der oben aufgeführten Substituenten $R^1$ - $R^{15}$ mit der Bedeutung Fluor, Chlor, Brom, Jod oder für den Rest $-CH_2-NO_2$ steht,
in inerten Lösemitteln halogeniert und gegebenenfalls anschließend entweder durch Eliminierung nach bekannter Methode Doppelbindungen eingeführt, gegebenenfalls eine Epoxidierung durchgeführt und gegebenenfalls eine Reduktion, Oxidation oder Hydrolyse nach üblicher Methode anschließt,
und somit die Substituenten $R^1$ - $R^{15}$ in die Verbindungen der allgemeinen Formel (Ib) und geeignete Derivate einführt, oder die Substituenten $R^{1'}$, $R^{2'}$, $R^{6'}$ und $R^{8'}$ in andere funktionelle Gruppen überführt.

Je nach Art der verwendeten Ausgangsstoffe können die Synthesevariationen für die erfindungsgemäßen Verbindungen durch folgende Formelschemata wiedergegeben werden:

[A]

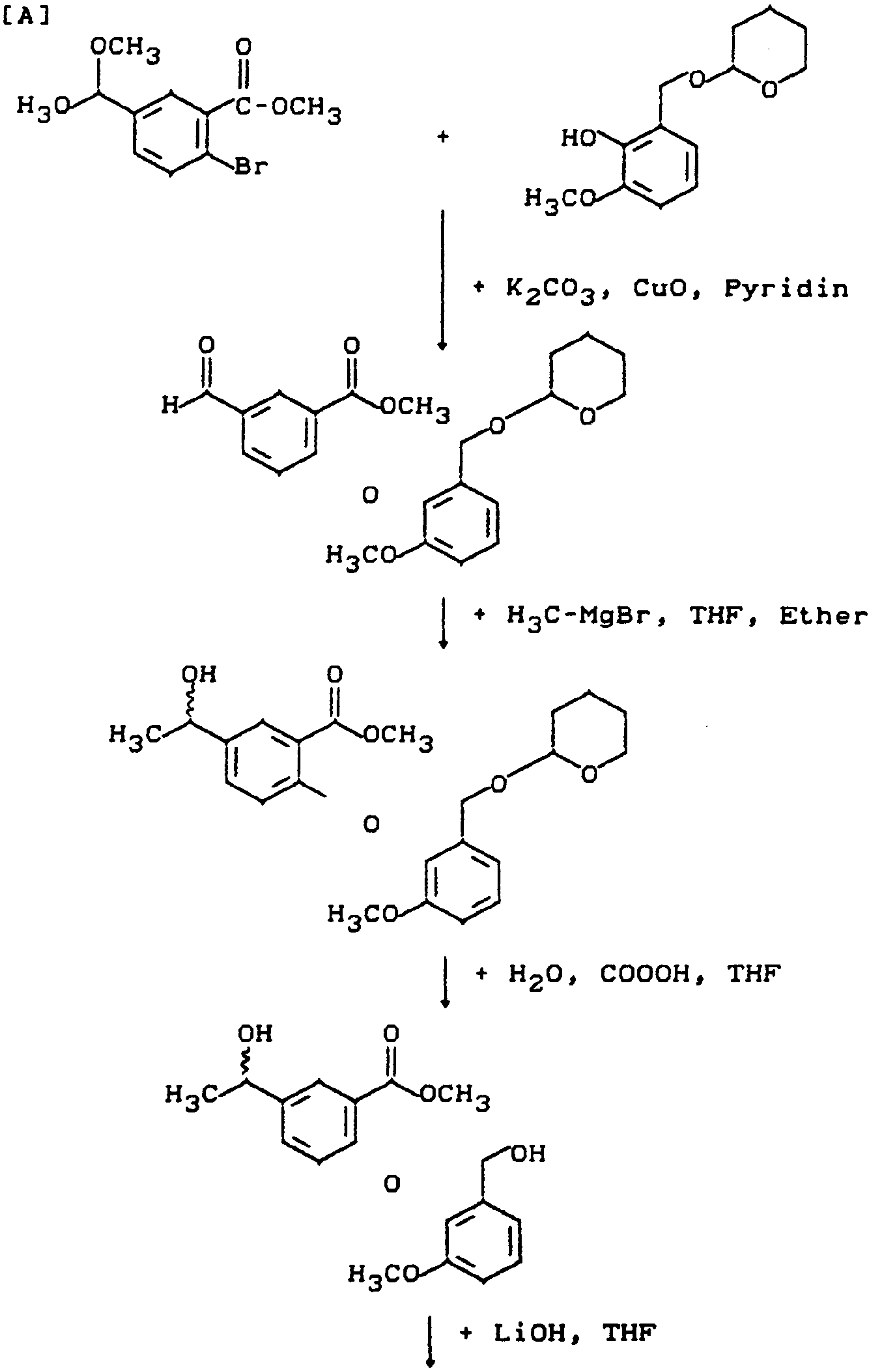

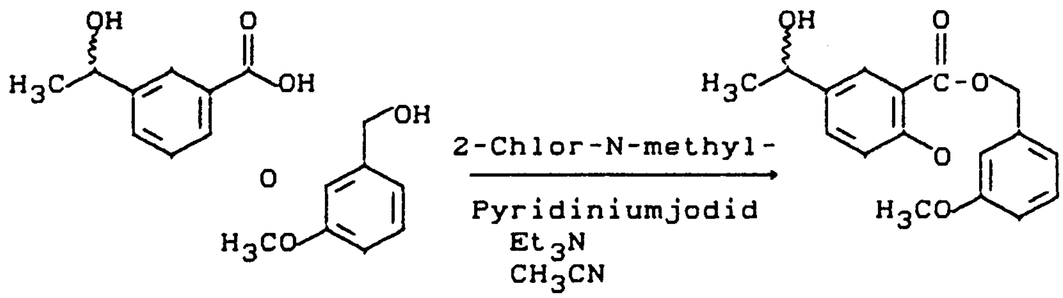
2-Chlor-N-methyl-
Pyridiniumjodid
$Et_3N$
$CH_3CN$

[B]

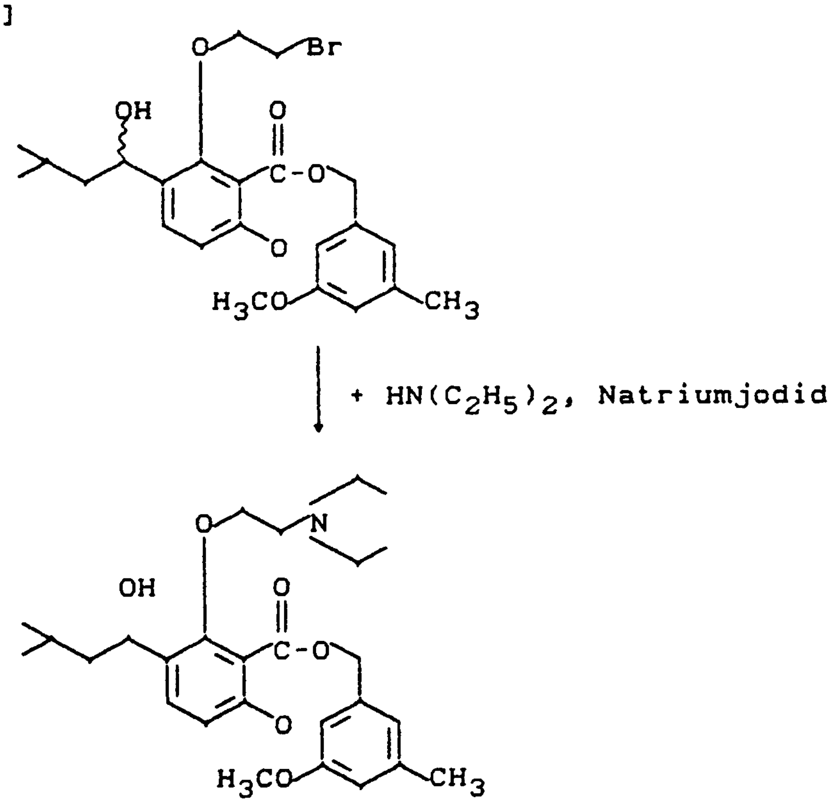
+ $HN(C_2H_5)_2$, Natriumjodid

[B]

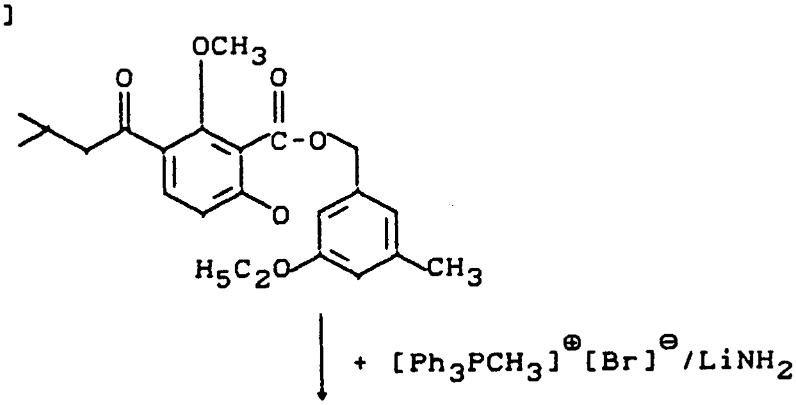
+ $[Ph_3PCH_3]^{\oplus}[Br]^{\ominus}/LiNH_2$

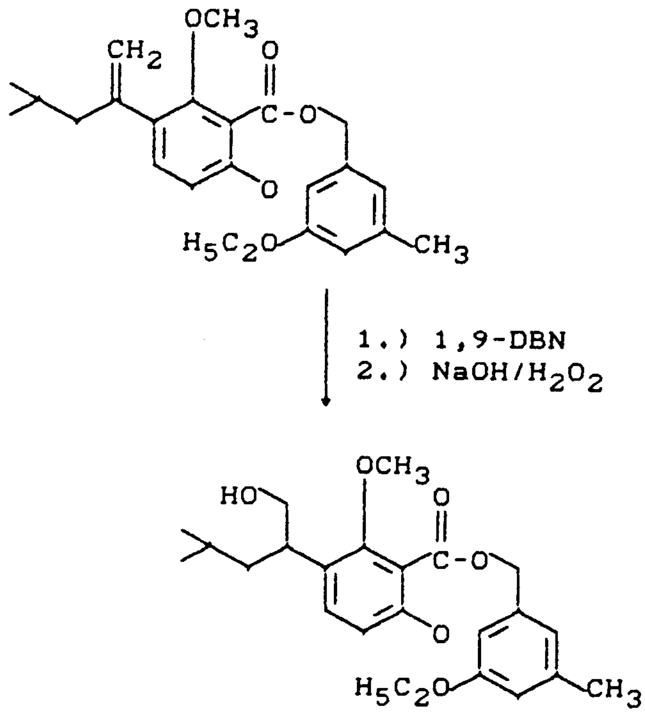
1.) 1,9-DBN
2.) NaOH/$H_2O_2$

]

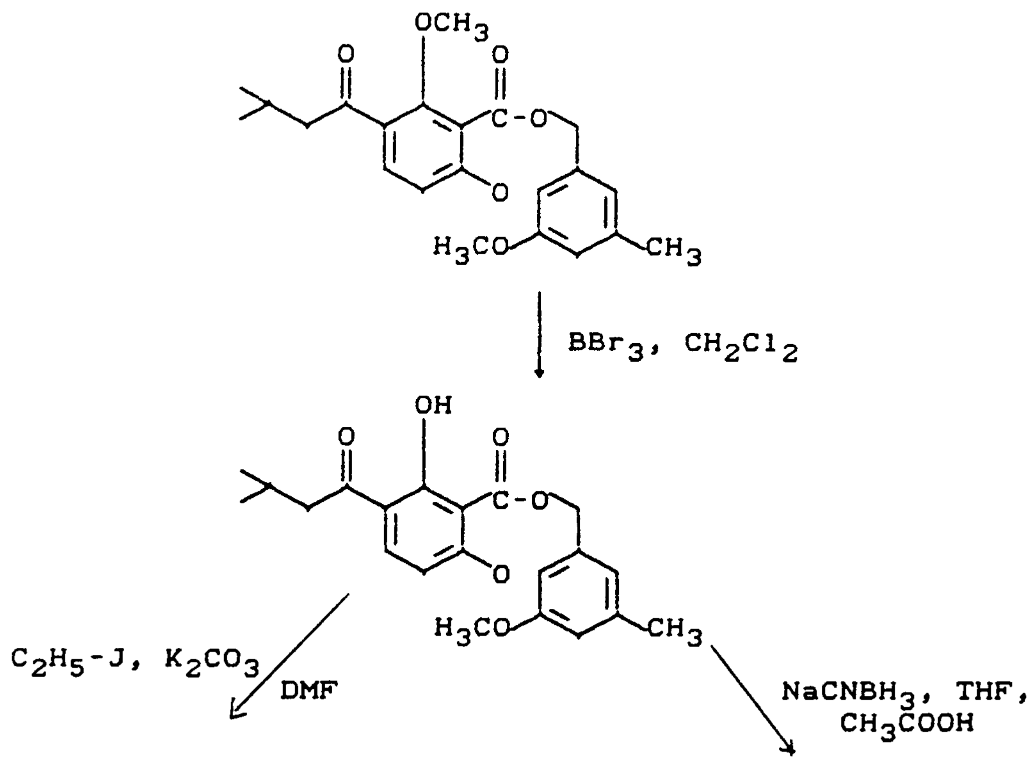
$BBr_3$, $CH_2Cl_2$
$C_2H_5$-J, $K_2CO_3$
DMF
$NaCNBH_3$, THF,
$CH_3COOH$

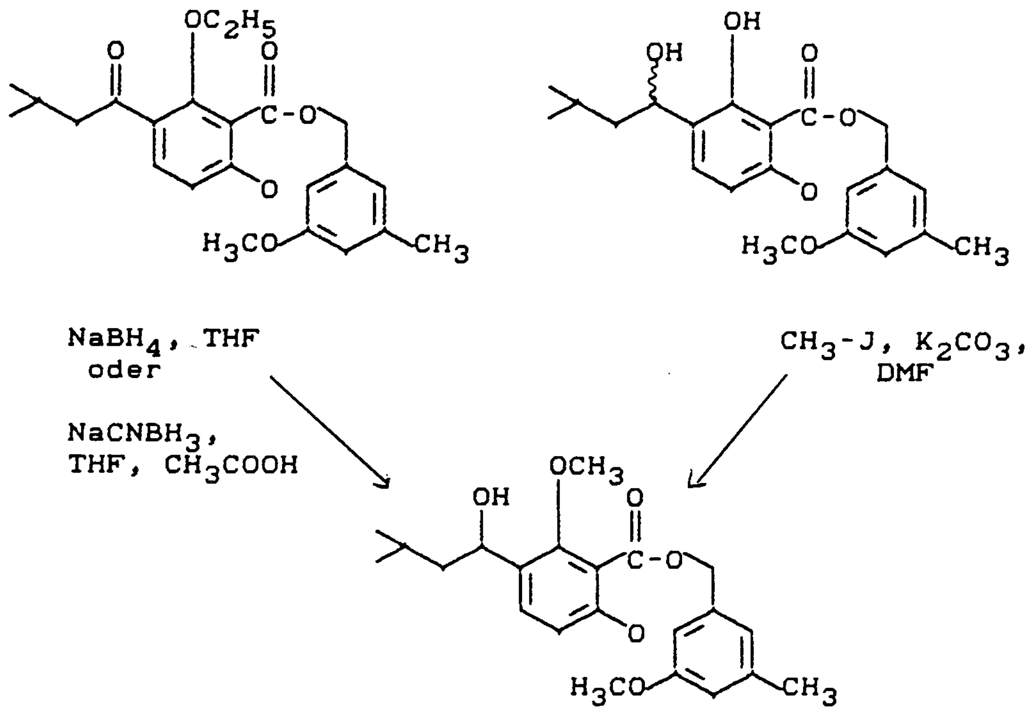


Verfahren [A] - [B]

Als Lösemittel für die Verfahren [A] und [B] können hier die üblichen organischen Lösemittel verwendet werden, die sich unter den Reaktionsbedindungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Carbonsäuren wie Essigsäure oder Propionsäure, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Die Reaktionstemperaturen können bei allen Verfahren in einem größeren Bereich variiert werden. Im allgemeinen arbeitet man zwischen -20 °C und +200 °C, vorzugsweise zwischen +20 °C und +100 °C. Insbesondere bei der Siedetemperatur des jeweiligen Lösemittels.

Die Umsetzungen können bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A und B ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristalliert. In einigen Fällen kann es erforderlich sein, die erfindungsgemäßen Verbindungen durch Chromatographie zu reinigen.

Als Basen eignen sich die üblichen anorganischen oder organischen Basen. Hierzu gehören bevorzugt Akalihydroxide wie beispielsweise Natrium-, Lithium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder 2-Chlor-N-methylpyridiniumjodid, oder Amide wie Natriumamid, Lithiumamid, Lithiumisopropylamid, oder metallorganische Verbindungen wie Butyllithium oder Phenyllithium.

Als Katalysatoren werden für einzelne Verfahrensvarianten beispielsweise Kupfersalze oder -oxide, bevorzugt Kupferoxid und Kupfer(II)acetat, oder Alkalimetalljodide wie Natriumjodid oder Kaliumjodid eingesetzt, die dem Reaktionsansatz in einer Menge von 0,5 und 150 Mol, bevorzugt mit 5 bis 50 Mol, zugesetzt werden.

Als aktivierende Reagenzien können beispielsweise Azodicarbonsäuren oder Triphenylphosphin in molaren Verhältnissen oder im Überschuß zugesetzt werden.

Die in Verfahren [A] beschriebene Kondensation wird in einem der oben aufgeführten inerten Lösemittel unter Baseneinwirkung, bevorzugt in Pyridin mit Kaliumcarbonat durchgeführt, während für die Cyclisierung bevorzugt Acetonitril, Triethylamin und 2-Chlor-N-methylpyridiniumjodid eingesetzt werden.

Als Hilfsstoffe werden bevorzugt Kondensationsmittel eingesetzt, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N-'Dipropyl-, N,N'-Diisopropyl-, N,N'-Dicyclohexylcarbodiimid, N-(3-Dimethylaminoisopropyl)-N'-ethylcarbodiimid-Hydrochlorid oder 2-Chlor-N-methylpyridiniumjodid.

Die Einführung und Abspaltung der Hydroxyschutzgruppe erfolgt nach bekannten Methoden [Th. Greene, "Protective Groups in Organic Synthesis", 1. Auflage, J. Wiley & Sons, New York, 1981]. Die Schutzgruppen können beispielsweise durch saure oder basische Hydrolyse oder durch Hydrogenolyse abgespalten werden.

Die Alkylierung erfolgt in einem der oben aufgeführten inerten Lösemitteln, bevorzugt in Dimethylformamid in Anwesenheit von Kaliumcarbonat.

Die Reduktion erfolgt im allgemeinen mit Metallhydriden oder Borhydriden, bevorzugt sind Natriumborhydrid und Natriumcyanoborhydrid in inerten Lösemitteln wie Ethern, bevorzugt in Tetrahydrofuran, Diethylether oder Dioxan in einem Temperaturbereich von -20 °C bis +100 °C, bevorzugt von 0 °C bis +50 °C bei Normaldruck.

Die Reduktion ist außerdem durch Hydrierung in inerten Lösemitteln wie Alkoholen z.B. Methanol, Ethanol, Propanol oder Isopropanol in Gegenwart eines Edelmetallkatalysators wie Platin, Palladium, Palladium auf Tierkohle oder Raney-Nickel, in einem Temperaturbereich von 0 °C bis +150 °C, bevorzugt von Raumtemperatur bis +100 °C bei Normaldruck oder Überdruck möglich.

Die Reduktion von Carbonylgruppen zu Kohlenwasserstoffen verläuft im allgemeinen mit Reduktionsmitteln wie Zinkamalgam und Säuren wie Salzsäure oder mit Hydrazinhydrat und Basen wie Natrium- oder Kaliumhydroxid, in den oben aufgeführten Lösemitteln, bevorzugt in Ethern wie Tetrahydrofuran oder Diethylether. Aldoxime und Ketoxime werden im allgemeinen mit den oben aufgeführten Metallhydriden, bevorzugt mit Lithiumaluminiumhydrid, oder mit Zink und Essigsäure, Borwasserstoffsäure, Natrium in Alkoholen oder durch die oben erwähnte katalytische Hydrierung zu den entsprechenden Aminen reduziert.

Die Reduktion von Alkoxycarbonylgruppen in Alkoholgruppen erfolgt im allgemeinen mit Hydriden, bevorzugt mit Lithiumaluminiumhydrid in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan in einem Temperaturbereich von 0 °C bis +150 °C, bevorzugt von +20 °C bis +100 °C bei Normaldruck.

Die Oxidation von Alkoholen zu Aldehyden und Ketonen erfolgt im allgemeinen mit Oxidationsmitteln wie Dichromat, Kaliumpermanganat, Brom, Mangandioxid, Dipyridinchrom(VI)oxid, Pyridindichromat, Dimethylpyrazol-$CrO_3$ Komplex, Silbercarbonat auf Zelit, Jodosobenzol, Bleitetraacetat-pyridin, Pyridiniumchlorochromat oder dem Jones-Reagenz, bevorzugt mit Pyridiniumchlorochromat in den oben erwähnten Lösemitteln, bevorzugt in einem Temperaturbereich von -20 °C bis +100 °C, bevorzugt von 0 °C bis +50 °C bei Normaldruck.

Die Wittig-Reaktionen verlaufen im allgemeinen durch Umsetzung mit Tetraalkyl- oder -arylsubstituierten Phosphoniumhalgeniden, bevorzugt mit Triphenylmethylphosphoniumbromid, in inerten Lösemitteln wie Ethern, bevorzugt mit Tetrahydrofuran, in Anwesenheit einer Base bevorzugt Lithiumamid, in einem Temperaturbereich von -10 °C bis +100 °C, bevorzugt bei Raumtemperatur bei Normaldruck.

Die Substitutionsreaktionen verlaufen im allgemeinen in den oben erwähnten inerten Lösemitteln oder in Wasser, bevorzugt in Wasser, Ameisensäure, Methanol, Ethanol, Dimethylformamid oder deren Gemische, gegebenenfalls in Anwesenheit einer der oben aufgeführten Basen oder Katalysatoren in einem Temperaturbereich von -60 °C bis +200 °C, bevorzugt von 0 °C bis +100 °C bei Normaldruck.

Die Halogenierung erfolgt in einem der oben aufgeführten inerten Lösemitteln, bevorzugt in Dimethylformamid, in einem Temperaturbereich von -10 °C bis +150 °C, bevorzugt von +25 °C bis +80 °C, bei Normaldruck.

Die im einzelnen nicht aufgeführten Reaktionen zur Einführung der Substituenten $R^1$ - $R^{15}$ wie beispielsweise Acylierungen, nucleophile oder elektrophile Substitutionen, Radikalreaktionen, Eliminierungen und Umlagerungen erfolgen nach literaturbekannten Methoden [vgl. beispielsweise C. Ferri, Reaktionen der organischen Synthese, Georg Thieme Verlag, Stuttgart 1978 und J. March, Advanced Organic Chemistry, second edition, McGraw Hill, Book Company].

Die Verbindungen der allgemeinen Formeln (II), (IIa), (III), (IIIa) und (IV) sind an sich bekannt oder können nach üblicher Methode hergestellt werden [Tietze und Eicher, Reaktionen und Synthesen im

organisch chemischen Praktikum, Georg Thieme Verlag, Stuttgart, New York, 1981; W. Fuerer, H.W. Gschwend, J. Org. Chem. 44, 1133 - 1136 (1979); F.W. Vierhapper, E. Trengler, K. Kratzl, Monatshefte für Chemie, 106, 1191 - 1201 (1975); John A. Elix und Vilas Jayanthi, Aust. J. Chem. 1987, 40 1841 -1850].

Die Verbindungen der Formel (Ib) sind nach der Publikation in Tetrahedron Letters, 45, pp. 3941 - 3947, (1974) nicht herstellbar. Sie konnten aber über den neuen Stamm Penicillium funiculosum Thom nach üblichen Methoden isoliert werden [vgl. Bodenwaschtechnik zur Isolierung von Boden-und Rhezosphärenpilzen, Methoden des mykologischen Laboratoriums, H. Kreisel, F. Schauer, Gustav Fischer Verlag, Stuttgart, New York, 1987]. Eine Kultur dieses Stammes wurde bei der Deutschen Sammlung für Mikroorganismen in Braunschweig am 08.03.1989 unter der Nummer DSM 5249 hinterlegt.

Die Verbindungen der allgemeinen Formeln (V), (VI) und (VII) sind bekannt oder können nach bekannten Methoden hergestellt werden [z.B. J. March, "Advanced Organic Chemistry", Second edition).

Die neuen und die bekannten Verbindungen der Formel (I) und (Ia) zeigen ein nicht vorhersehbares, wertvolles pharmakologisches Wirkspektrum. Sie beeinflussen die ANP-Freisetzung, die Kontraktionskraft des Herzens, den Tonus der glatten Muskulatur sowie den Elektrolyt- und Flüssigkeitshaushalt und wirken sowohl partiell oder vollständig digitalisantagonistisch oder digitalisagonistisch.

Sie können deshalb in Arzneimitteln zur Behandlung des pathologisch veränderten Blutdrucks, der Herzinsuffizienz, sowie als Koronartherapeutika oder als Therapeutikum bei Digitalisvergiftungen eingesetzt werden.

Darüberhinaus können sie zur Behandlung von Herzrhythmusstörungen, Niereninsuffizienz, Leberzirrhose, Aszites, Lungenödem, Hirnödem, Schwangerschaftsödem oder Glaukom eingesetzt werden.

Die antihypertensive Wirkung von 3-(1-Hydroxy-3-methylbutyl)-4,11-dimethoxy-9-methyl-7H-dibenzo-[b,g][1,5]dioxocin-5-on wurde an Ratten mit "reduced renal mass"-Hypertonie untersucht. Die "reduced renal mass" (RRM)-Hypertonie wurde durch 5/6-Nephrektomie mit Verabreichung einer 0,5%igen Kochsalzlösung anstelle von Trinkwasser in Anlehnung an das von Hvart et al. (1983) beschriebene Verfahren hervorgerufen.

Die Verbindung aus Beispiel 39 wirkte bei dieser Hypertonieform mit einer oralen $ED_{20}$ (20% Senkung des systolischen Blutdrucks bei indirekter Messung an wachen Ratten) von 100 mg/kg.

Die Verbindungen aus den Beispielen 12, 34, 74 und 76 wiederum stimulieren am isolierten Rattenvorhof die ANP-Freisetzung um 292, 265, 196 und 192 Prozent. Die ANP-Konzentration in der Badflüssigkeit wurde radioimmunologisch bestimmt [J.P. Stasch, H. Grote, S. Kazda, C. Hirth, Dynorphin stimulates the release of ANP from isolated rat atria, Eur. J. Pharmacol. 159, 101 (1989)].

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate, Arylsulfonate), Detergentien (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellung von Ausgangsverbindungen

Beispiel I

5-Formyl-2-[2-methoxy-6-(2-tetrahydropyranyloxymethyl)]phenoxybenzoesäuremethylester

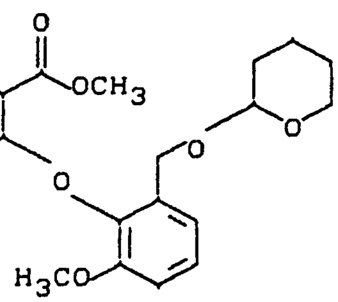

Zu einer Lösung von 10 g (34,6 mmol) 2-Brom-5-dimethoxymethyl-benzoesäuremethylester in 25 ml absolutem Pyridin werden 8,2 g (34,6 mmol) 2-Methoxy-6-(2-tetrahydropyranyloxymethyl)phenol, 5,3 g (38 mmol) Kaliumcarbonat und 3,0 g (38 mmol) Kupferoxid zugegeben. Es wird auf 130 °C erwärmt. Nach 3 h werden weitere 3,0 g (38 mmol) Kupferoxid zugegeben. Nach 12 h Reaktionszeit wird das Lösemittel abdestilliert, der Rückstand in Dichlormethan aufgenommen und mit verdünnter Salzsäure extrahiert. Die organische Phase wird mit Wasser gewaschen, über Magnesiumsulfat getrocknet, im Vakuum eingedampft und säulenchromatographisch an Kieselgel mit Petrolether/Ether 1:1 gereinigt.
Ausbeute: 6,5 g (48% der Theorie)
MS: EI: 400, 315, 300, 255, 239
SF: $C_{22}H_{24}O_7$ (400)

Beispiel II

5-Formyl-2-[2-methoxy-4-methyl-6-(2-tetrahydropyranyloxymethyl)]-3-phenoxybenzoesäuremethylester

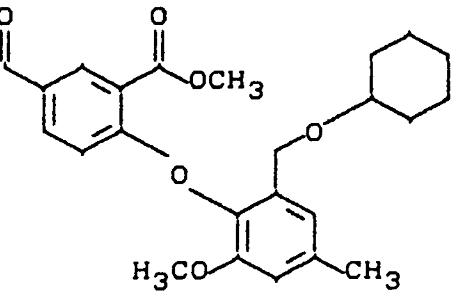

Die Titelverbindung wurde analog der Vorschrift für Beispiel I hergestellt.
Ausbeute: 78% der Theorie
MS: EI: 414, 314, 269, 135
SF: $C_{23}H_{26}O_7$ (414)

Beispiel III

5-Formyl-2-(6-hydroxymethyl-2-methoxy)phenoxybenzoesäuremethylester

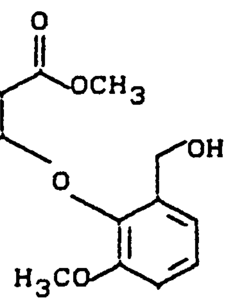

100 mg (0,25 mmol) der Verbindung aus Beispiel I werden in einem Gemisch aus 2 ml Essigsäure, 1 ml Tetrahydrofuran und 0,5 ml Wasser gelöst und 4 h auf 50 °C erwärmt. Nach weiteren 12 h bei Raumtemperatur wird im Vakuum eingeengt, der Rückstand dreimal mit Toluol codestilliert und säulenchromatographisch an Kieselgel mit Petrolether/Essigester 2:1 gereinigt.
Ausbeute: 60,5 mg (76% der Theorie)
MS: EI: 316, 255, 180, 148, 136
SF: $C_{17}H_{16}O_6$ (316)
Analog der Vorschrift für Beispiel III werden die in der folgenden Tabelle 1 aufgeführten Verbindungen hergestellt:

Tabelle 1

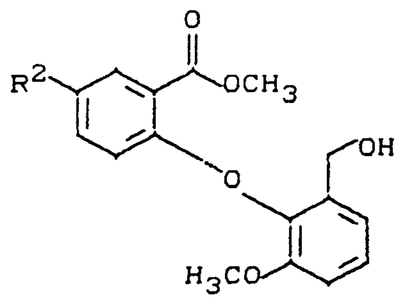

| Bsp. Nr. | $R^2$ | MS: (EI) | SF |
|---|---|---|---|
| IV | 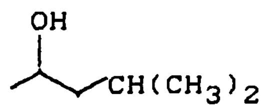 | 374, 255, 239, 220, 205 | $C_{21}H_{26}O_6$ (374) |
| V | 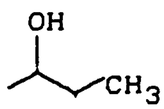 | 346, 285, 269, 255, 239 | $C_{19}H_{22}O_6$ (346) |

Fortsetzung Tabelle 1:

| Bsp. Nr. | $R^2$ | MS: (EI) | SF |
|---|---|---|---|
| VI | 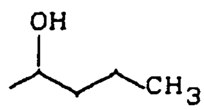 | 360, 255, 239, 181 | $C_{20}H_{24}O_6$ (360) |
| VII | 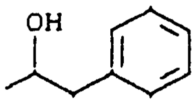 | 408, 317, 137 | $C_{24}H_{24}O_6$ (408) |
| VIII | 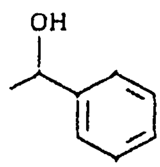 | 394, 317, 269, 226, | $C_{23}H_{22}O_6$ (394) |
| IX | 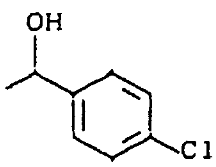 | 428, 351, 179, 153, | $C_{23}H_{21}ClO_6$ (428) |
| X | 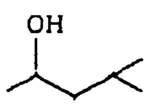 | 388, 181, 151 | $C_{22}H_{28}O_6$ (388) |

Beispiel XI

5-Formyl-2-(6-hydroxymethyl-2-methoxy)phenoxybenzoesäure

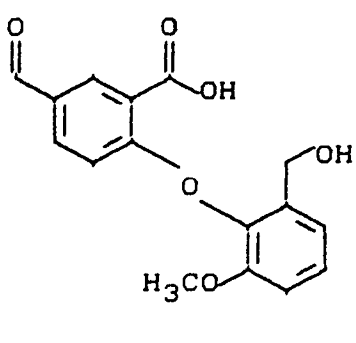

300 g (0,95 mmol) der Verbindung aus Beispiel 3 werden in 15 ml Tetrahyrofuran gelöst und mit 7,3 ml einer 0,2 N Lithiumhydroxidlösung in Wasser versetzt. Nach 16 h bei 6 ° C gibt man 6 ml 32%ige Salzsäure zu der Lösung. Die Substanz kristalliert aus. Nach 2 h wird die Flüssigkeit abgesaugt, der Rückstand mit etwas Wasser gewaschen und über Phosphorpentoxid getrocknet.

Ausbeute: 192 mg (67% der Theorie)

MS: EI: 302, 166, 148, 136

SF: $C_{16}H_{14}O_6$ (302)

Analog der Vorschrift für Beispiel XI wurden die in der folgenden Tabelle 2 aufgeführten Verbindungen

hergestellt:

Tabelle 2

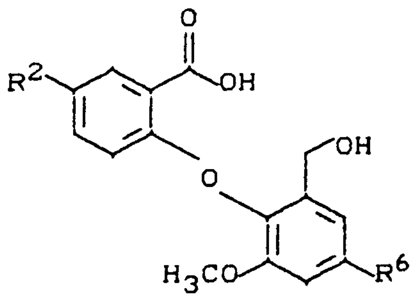

| Bsp. Nr. | $R^2$ | $R^6$ | MS: (EI) | SF |
|---|---|---|---|---|
| XII | 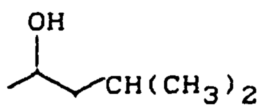 | H | 360, 342, 255, 206, 167 | $C_{20}H_{24}O_6$ (360) |
| XIII | 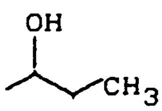 | H | 332, 167, 137 | $C_{18}H_{20}O_6$ (332) |

Fortsetzung Tabelle 2:

| Bsp. Nr. | $R^2$ | $R^6$ | MS: (EI) | SF |
|---|---|---|---|---|
| XIV | 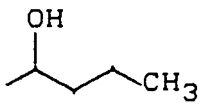 | H | 346, 167, 149, 137 | $C_{19}H_{22}O_6$ (346) |
| XV | 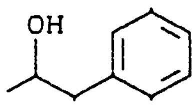 | H | 394, 376, 303, 137 | $C_{23}H_{22}O_6$ (394) |
| XVI | 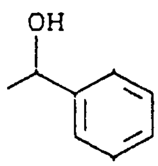 | H | 380, 227, 137 | $C_{22}H_{20}O_6$ (380) |
| XVII | 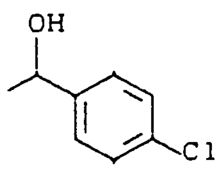 | H | 414, 257, 223 | $C_{22}H_{19}ClO_6$ (414) |
| XVIII | 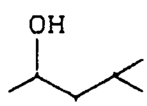 | $CH_3$ | 374, 356 206, 188 151 | $C_{21}H_{26}O_6$ (374) |

Beispiel XIX

5-(1-Hydroxy-3-methylbutyl)-2-[2-methoxy-4-methyl-6-(2-tetrahydropyranyloxymethyl]]-phenoxybenzoesäuremethylester

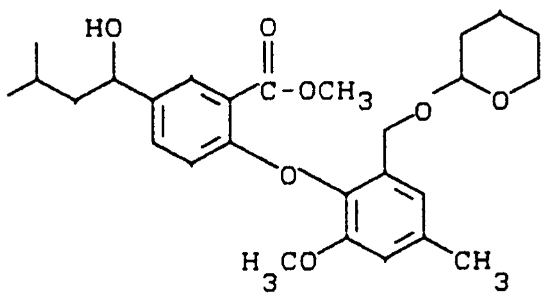

Die Titelverbindung wird analog der Vorschrift für Beispiel 140 erhalten.
Ausbeute: 50% der Theorie
MS (EI): 472, 327, 315, 285, 253
SF: $C_{27}H_{36}O_7$ (472)
Die in der nachfolgenden Tabelle 2a aufgeführten Verbindungen wurden analog der Vorschrift zu Beispiel 140 erhalten.

Tabelle 2a

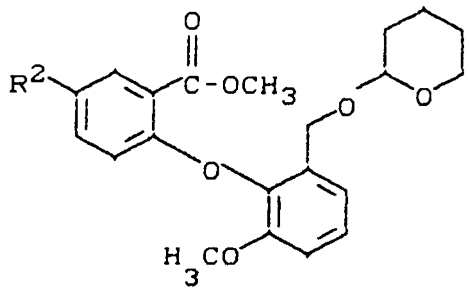

| Bsp. Nr. | $R^2$ | MS: (EI) | SF |
|---|---|---|---|
| XX | 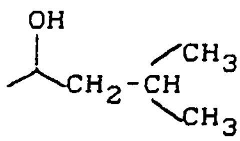 | 458, 313 301, 239, 181 | $C_{26}H_{34}O_7$ (458) |
| XXI | 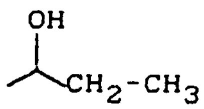 | 430, 344, 301, 285, 239 | $C_{24}H_{30}O_7$ (430) |
| XXII | 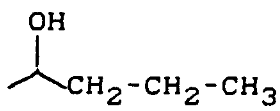 | DCI: 462(M+NH$_4$), 444, 427, 361, 343 | $C_{25}H_{32}O_7$ (444) |
| XXIII | 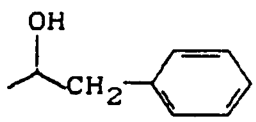 | DCI: 493 (M+H$^+$), 409, 334, 316, 299 | $C_{29}H_{32}O_7$ (492) |

Fortsetzung Tabelle 2a

| Bsp. Nr. | $R^{12}$ | MS: (EI) | SF |
|---|---|---|---|
| XXIV | 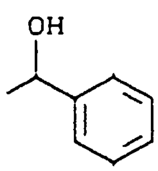 | 478, 393, 333, 239 | $C_{28}H_{30}O_7$ (478) |
| XXV | 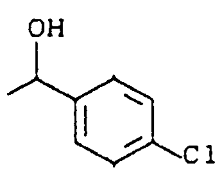 | 512, 394, 275 | $C_{28}H_{29}ClO_7$ (512) |

Die in Tabelle 2b aufgeführten Beispiele wurden in Analogie zur Vorschrift des Beispiels I hergestellt:

Tabelle 2b:

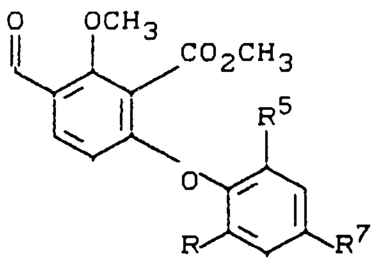

| Bsp. Nr | $R^5$ | $R^7$ | R | MS (EI) | SF |
|---|---|---|---|---|---|
| XXVI | 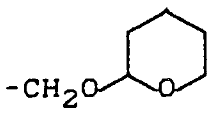 | H | $OCH_3$ | 430, 330 285, 269 | $C_{23}H_{26}O_8$ (430) |

Fortsetzung Tabelle 2b

| Bsp. Nr | $R^5$ | $R^7$ | R | MS (EI) | SF |
|---|---|---|---|---|---|
| XXVII | 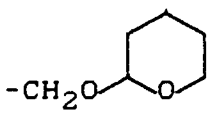 | $CH_3$ | H | 414, 269, 253, | $C_{23}H_{26}O_7$ (414) |
| XXVIII | 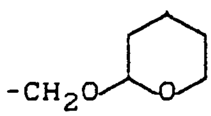 | Br | $OCH_3$ | 510, 508, 410, 408, 378 | $C_{23}H_{25}BrO_3$ (509) |
| XXIX | -CH-CH$_3$ <br> \| <br> OH | $CH_3$ | $OCH_3$ | 374, 324, 299, 256, 164 | $C_{20}H_{22}O_7$ (374) |

Die in Tabelle 2 c aufgeführten Beispiele wurden in Analogie zur Vorschrift des Beispiels III hergestellt:

Tabelle 2c:

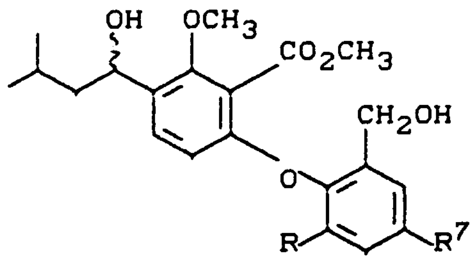

| Bsp. Nr. | $R^7$ | R | MS (EI) | SF |
|---|---|---|---|---|
| XXX | H | $OCH_3$ | 404, 347<br>285, 269<br>242 | $C_{22}H_{28}O_7$ (404) |

Fortsetzung Tabelle 2c

| Bsp. Nr. | $R^7$ | R | MS (EI) | SF |
|---|---|---|---|---|
| XXXI | $CH_3$ | H | 398, 342 | $C_{22}H_{28}O_6$ (388) |
| XXXII | Br | $OCH_3$ | 484, 482 | $C_{22}H_{27}BrO_7$ (483) |

Die in Tabelle 2d aufgeführten Beispiele wurden in Analogie zur Vorschrift des Beispiels 140 hergestellt.

Tabelle 2d:

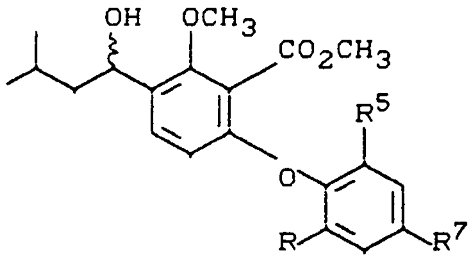

| Bsp. | $R^5$ | $R^7$ | R | MS (EI) | SF |
|---|---|---|---|---|---|
| XXXIII | $-CH_2O-$ 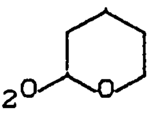 | H | $OCH_3$ | 488, 347, 331, 299, 269 | $C_{27}H_{36}O_6$ (488) |
| XXXIV | $-CH_2O-$ 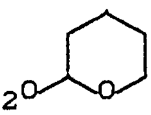 | $CH_3$ | H | 490, $(M+NH_4)^+$, 455, 388, 371, 253 | $C_{27}H_{36}O_7$ (472) |

Fortsetzung Tabelle 2d:

| Bsp. | $R^5$ | $R^7$ | R | MS (EI) | SF |
|---|---|---|---|---|---|
| XXXV | $-CH_2O-$ 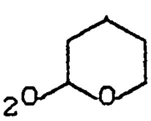 | Br | $OCH_3$ | 568, 566, 425, 409, 349 | $C_{27}H_{35}BrO_8$ (567) |
| XXXVI | $-CH-CH_3$<br>OH | $CH_3$ | $OCH_3$ | 432, 375, 326, 297, 165 | $C_{24}H_{32}O_4$ (432) |

Beispiel XXXVII

5-(1-Hydroxy-3-methylbutyl)-2-(6-hydroxymethyl-2-methoxy)phenoxy-6-methoxy-benzoesäure

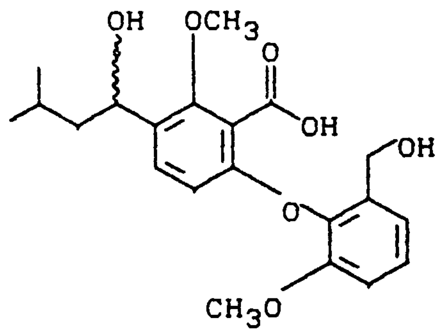

105 mg (0,26 mmol) der Verbindung aus Beispiel XXX werden in 2 ml Methanol gelöst und mit 145 mg (2,6 mmol) KOH versetzt. Nach 12 h bei Raumtemperatur wird der Ansatz i.V. eingeengt, in Wasser aufgenommen und mit verdünnter Salzsäure angesäuert. Die wäßrige Phase wird mit $CH_2Cl_2$ extrahiert, die vereinigten organischen Phasen über $MgSO_4$ getrocknet und i.V. eingeengt. Die so erhaltene Substanz wird ohne Charakterisierung weiter verarbeitet.

Die in Tabelle 2e aufgeführten Beispiele wurden in Analogie zur Vorschrift des Beispiels XXXVII hergestellt:

Tabelle 2e:

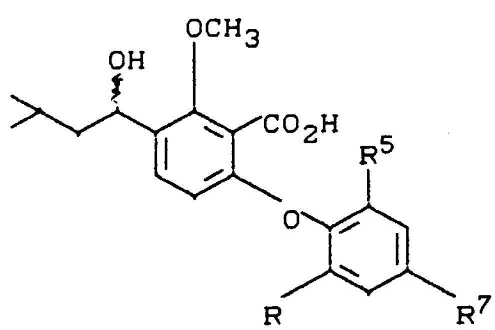

| Bsp. Nr. | $R^5$ | $R^7$ | R | MS (EI) | SF |
|---|---|---|---|---|---|
| XXXVIII | $-CH_2OH$ | $CH_3$ | H | 374 | $C_{21}H_{26}O_6$ (374) |
| XXXIX | $-CH_2OH$ | Br | $OCH_3$ | 469 | $C_{21}H_{25}BrO_7$ (469) |
| XL | $-CH(OH)-CH_3$ | $CH_3$ | $OCH_3$ | 418 | $C_{23}H_{30}O_7$ (418) |

Ausführungsbeispiele

Beispiel 1

3-Formyl-11-methoxy-9-methyl-7H-dibenzo[b,g][1,5]dioxocin-5-on

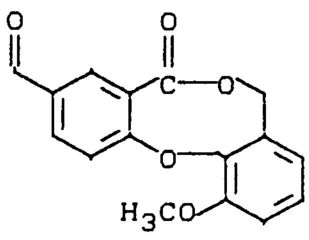

Zu einer Lösung von 609 mg (2,4 mmol) 2-Chlor-1-methylpyridiniumiodid in 40 ml absolutem Acetonitril wird bei 80 °C eine Lösung von 180 mg (0,6 mmol) der Verbindung aus Beispiel XI und 663 µl (4,8 mmol) Triethylamin in 40 ml absolutem Acetonitril über einen Zeitraum von 6 h zugetropft. Nach einer weiteren Stunde bei 80 °C wird im Vakuum eingeengt, der Rückstand in Dichlormethan aufgenommen, mit Wasser gewaschen, über Magnesiumsulfat getrocknet und eingeengt. Die Reinigung erfolgt säulenchromatographisch an Kieselgel mit Petrolether/Essigester 4:1. Im Verlauf der Trennung wird das Laufmittel auf Petrolether/Essigester 1:2 umgewandelt.

Ausbeute: 73 mg (43% der Theorie)

MS: EI: 284, 239, 136

SF: $C_{16}H_{12}O_5$ (284)

Analog der Vorschrift für Beispiel 1 wurden die in der folgenden Tabelle 3 aufgeführten Verbindungen hergestellt.

Tabelle 3:

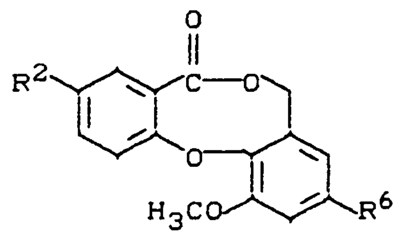

| Bsp. Nr. | $R^2$ | $R^6$ | MS: (EI) | SF |
|---|---|---|---|---|
| 2 | 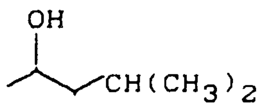 | H | 342, 285, 149, 137, | $C_{20}H_{22}O_5$ (342) |
| 3 | 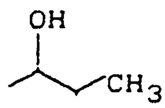 | H | 314, 285, 149, 137 | $C_{18}H_{18}O_5$ (314) |
| 4 | 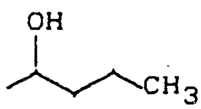 | H | 328, 285, 149, 137 | $C_{19}H_{20}O_5$ (328) |
| 5 | 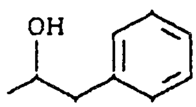 | H | 376, 285, 149, 137 | $C_{23}H_{20}O_5$ (376) |
| 6 | 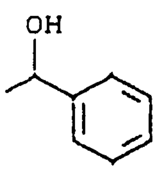 | H | 362, 283, 227, 213 | $C_{22}H_{18}O_5$ (362) |
| 7 | 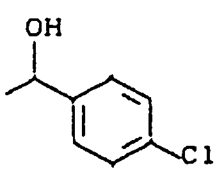 | H | 396, 282, 139 | $C_{22}H_{17}ClO_5$ (396) |
| 8 | 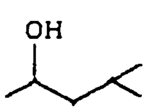 | $CH_3$ | 356, 299 151 | $C_{21}H_{24}O_5$ (356) |

Beispiel 9

3-(1-Hydroxy-3-methylbutyl)-11-(1-methyl-ethoxy)-4-methoxy-9-methyl-7H-dibenzo[b,g][1,5]dioxocin-5-on

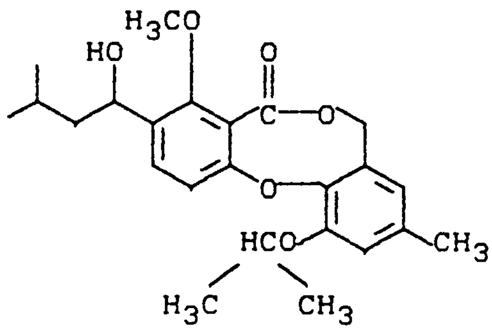

200 mg (0,54 mmol) Penicillide (Ib) werden in 2 ml Dimethylformamid mit 0,5 ml (50 mmol) Isopropyljodid und 160 mg (1,14 mmol) wasserfreiem Kaliumcarbonat 12 h unter Argon bei 25 °C gerührt. Nach Eindampfen im Vakuum wird mit 1 N wäßriger Salzsäure aufgenommen und erschöpfend mit Diethylether extrahiert. Nach Trocknen der organischen Phase wird der Verdampfungsrückstand an Kiesel mit Petrolether/Ether = 1/1 chromatographiert. Dünnschichtchromatographisch reine Fraktionen werden vereinigt und eingedampft.
Ausbeute: 169 mg (72% der Theorie) farbloser Feststoff
MS (EI): 414, 357, 315, 297, 269, 243, 219
SF: $C_{24}H_{30}O_6$ (414)
Analog der Vorschrift für Beispiel 9 werden die in den folgenden Tabellen 4, 5, 6 und 7 aufgeführten Verbindungen hergestellt.

Tabelle 4

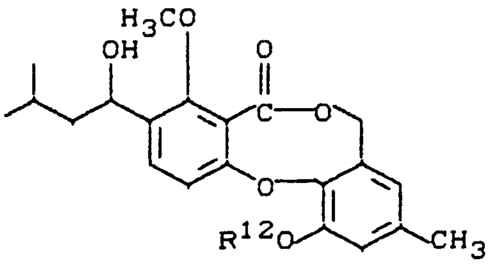

| Bsp. Nr. | $R^{12}$ | MS: (EI) | SF |
|---|---|---|---|
| 10 | $-(CH_2)_4-Br$ | 508, 506, 490, 488 451, 449 269, 137, 135 | $C_{25}H_{31}BrO_6$ (507) |
| 11 | 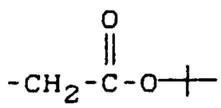 | DCI: 504, 486, 469, 448, 413 | $C_{27}H_{34}O_8$ (486) |
| 12 | 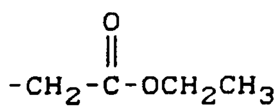 | 458, 401, 283, 255 | $C_{25}H_{30}O_8$ (458) |
| 13 | 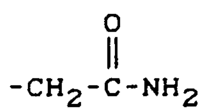 | 429, 372, 326, 297, 274 | $C_{23}H_{27}NO_7$ (429) |
| 14 | 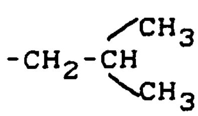 | 428, 371, 269, 243 | $C_{25}H_{32}O_6$ (428) |
| 15 | 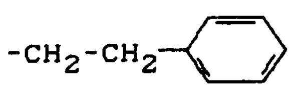 | 476, 419, 401, 105 | $C_{29}H_{32}O_6$ (476) |

Fortsetzung Tabelle 4

| Bsp. Nr. | $R^{12}$ | MS: (EI) | SF |
|---|---|---|---|
| 16 | $-CH_2$ 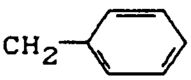 | 462, 405, 91 | $C_{28}H_{30}O_6$ (462) |
| 17 | $-CH_2$ 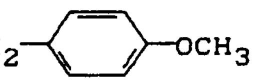 | 492, 354, 315, 269 | $C_{29}H_{32}O_7$ (492) |
| 18 | $-(CH_2)_5-CH_3$ | 456, 399 369, 353 269 | $C_{27}H_{36}O_6$ (456) |
| 19 | $-(CH_2)_2-CH(CH_3)_2$ | 442, 385, 357, 339 | $C_{26}H_{34}O_6$ (442) |
| 20 | $-CH_2-CH=CH_3$ | 412, 355, 309 | $C_{24}H_{28}O_6$ (412) |
| 21 | $-CH_2-CH_2-CH_3$ | 414, 357, 329, 311 | $C_{24}H_{30}O_6$ (414) |
| 22 | $-CH_2$ 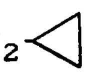 | 426, 369, 269, 179 | $C_{25}H_{30}O_6$ (426) |
| 23 | $-CH_2-CH_3$ | 400, 343, 313, 297 | $C_{23}H_{28}O_6$ (400) |
| 24 | $-CH_2$ 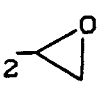 | 428, 371 341, 335 | $C_{24}H_{28}O_7$ (428) |

Fortsetzung Tabelle 4

| Bsp. Nr. | $R^{12}$ | MS: (EI) | SF |
|---|---|---|---|
| 25 | 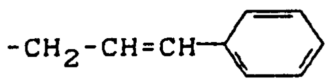 | 488, 431, 413 | $C_{30}H_{32}O_6$ (488) |
| 26 | 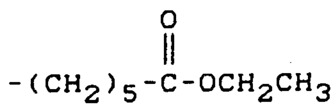 | 514, 457, 439, 427, 297 | $C_{29}H_{38}O_8$ (514) |
| 27 | $-(CH_2)_6-OH$ | 472, 397 369, 297 | $C_{27}H_{36}O_7$ (472) |
| 28 | $-(CH_2)_3-OH$ | 430, 373, 355, 343, 337 | $C_{24}H_{30}O_7$ (430) |
| 29 | 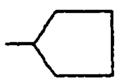 | 440, 383, 315, 269 | $C_{26}H_{32}O_6$ (440) |
| 30 | 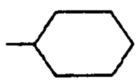 | 454, 315, 269, 243, 219 | $C_{27}H_{34}O_6$ (454) |
| 31 | 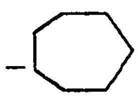 | 468, 411, 315, 269 | $C_{28}H_{36}O_6$ (468) |
| 32 | 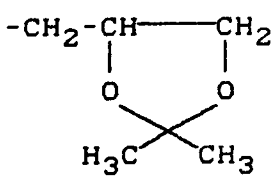 | 486, 471, 429, 353, 341, 115 | $C_{27}H_{34}O_8$ (486) |

Fortsetzung Tabelle 4

| Bsp. Nr. | $R^{12}$ | MS: (EI) | SF |
|---|---|---|---|
| 33 | 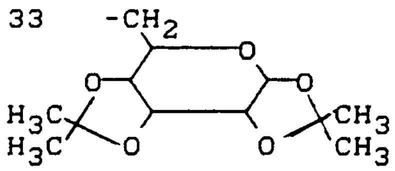 | FAB: 614, 597, 269 | $C_{33}H_{42}O_{11}$ (614) |
| 34 | 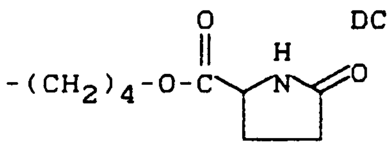 | DCI: 556, 538, 462, 444, 427 | $C_{30}H_{37}NO_9$ (555) |
| 35 | $-CH_2-CH=CH-CH_3$ | 426, 372, 315, 269 | $C_{25}H_{30}O_6$ (426) |

Tabelle 5

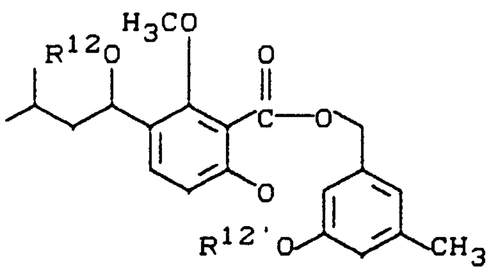

| Bsp. Nr. | $R^{12}/R^{12'}$ | MS: (EI) | SF |
|---|---|---|---|
| 36 | $R^{12} = R^{12'}$ = 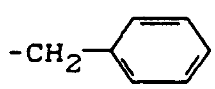 | DCI: 445, 354, 298 | $C_{35}H_{36}O_6$ (552) |

Fortsetzung Tabelle 5:

| Bsp. Nr. | $R^{12}/R^{12'}$ | MS: (EI) | SF |
|---|---|---|---|
| 37 | $R^{12}$ = 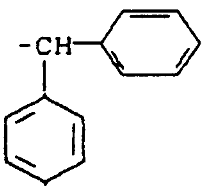 <br> $R^{12'}$ = H | 538, 400, 252, 168 | $C_{34}H_{34}O_6$ (538) |
| 38 | $R^{12'}$ = H <br> $R^{12}$ = $-CH_2O-(CH_2)_2-OCH_3$ | 460, 354, 89 | $C_{25}H_{32}O_8$ (460) |

<u>Tabelle 6</u>

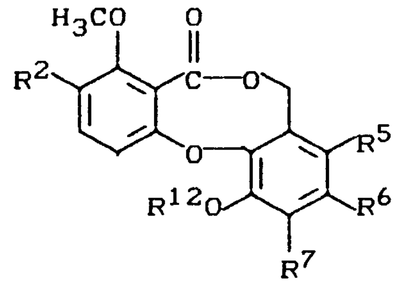

| Bsp. Nr. | $R^2$ | $R^5$ | $R^6$ | $R^7$ | $R^{12}$ | MS (EI) | SF |
|---|---|---|---|---|---|---|---|
| 39 | 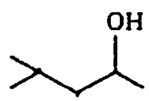 | Br | $CH_3$ | H | $CH_3$ | 466, 464 409, 407 363, 361, 322, 320 | $C_{22}H_{25}BrO_6$ (465) |
| 40 | 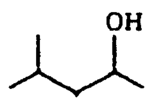 | H | $CH_3$ | Br | $CH_3$ | 466, 464, 409, 407 362, 360 | $C_{21}H_{25}BrO_6$ (465) |
| 41 | 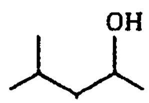 | Br | $CH_3$ | Br | $CH_3$ | 546, 544, 542, 489, 487, 485, 443, 441, 439 | $C_{22}H_{24}Br_2O_6$ (544) |
| 42 | 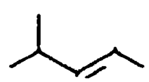 | H | $CH_3$ | H | H | 354, 343, 269, 163 | $C_{21}H_{22}O_5$ (354) |
| 43 | 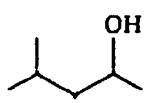 | H | $CH_3$ | 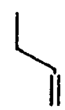 | $CH_3$ | 426, 369 | $C_{25}H_{30}O_6$ (426) |

Tabelle 7

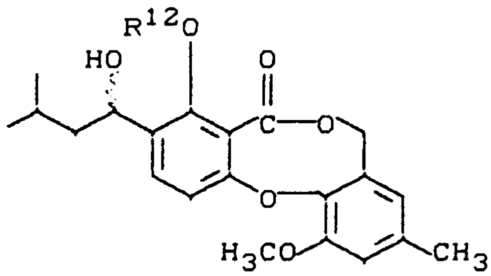

| Bsp. Nr. | $R^{12}$ | MS: (EI) | SF |
| --- | --- | --- | --- |
| 44 | 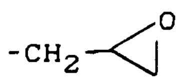 | 428, 371, 297, 269 | $C_{24}H_{28}O_7$ (428) |
| 45 | $-CH_2-CH_2-OH$ | 416, 209 151 | $C_{23}H_{28}O_7$ (416) |

Tabelle 8

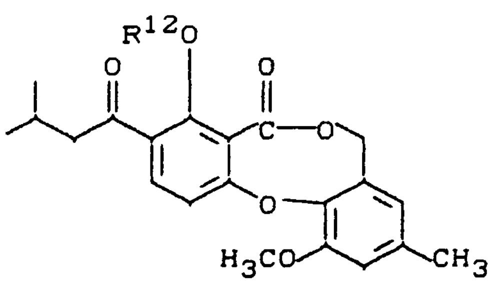

| Bsp. Nr. | $R^{12}$ | MS: (EI) | SF |
| --- | --- | --- | --- |
| 46 | 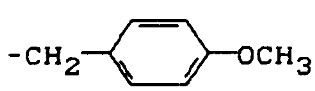 | FAB: 491 $(M+H^+)$ 154, 121 | $C_{29}H_{30}O_7$ (490) |

Fortsetzung Tabelle 8

| Bsp. Nr. | $R^{12}$ | MS: (EI) | SF |
|---|---|---|---|
| 47 | 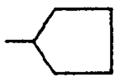 | 438, 370 150 | $C_{26}H_{30}O_6$ (438) |
| 48 | 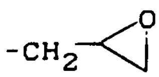 | 426, 369, 151, 132 | $C_{24}H_{26}O_7$ (426) |
| 49 | $-CH_2-CH_2-Br$ | 478, 476, 420, 418, 353 | $C_{23}H_{25}BrO_6$ |
| 50 | 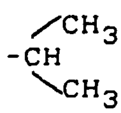 | 412, 370 150 | $C_{24}H_{28}O_6$ (412) |
| 51 | $-CH_2-CH_3$ | 398, 341, 312, 297 151 | $C_{23}H_{26}O_6$ (398) |
| 52 | 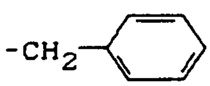 | 460, 403, 384, 354, 327 | $C_{28}H_{28}O_6$ (460) |
| 53 | $-CH_2-CH=CH_2$ | 410, 297, 240, 177 | $C_{24}H_{26}O_6$ (410) |
| 54 | 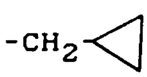 | 424, 297, 283, 269 | $C_{25}H_{28}O_6$ (424) |
| 55 | 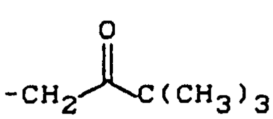 | 484, 428, 384, 327, 177 | $C_{27}H_{32}O_8$ (484) |

Fortsetzung Tabelle 8

| Bsp. Nr. | $R^{12}$ | MS: (EI) | SF |
|---|---|---|---|
| 56 | $-CH_2-CH_2-CH_3$ | 412, 312, 297, 151 | $C_{24}H_{28}O_6$ (412) |
| 57 | 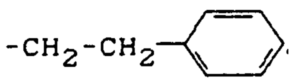 | 474, 432, 417, 370, 150 | $C_{29}H_{30}O_6$ (474) |
| 58 | $-CH-(C_6H_5)_2$ | DCI: 554($M+NH_4^+$) 537, 453, 371, 167 | $C_{34}H_{32}O_6$ (536) |
| 59 | 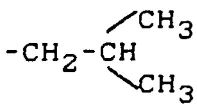 | 426, 370, 297, 151 | $C_{25}H_{30}O_6$ (426) |
| 60 | $-(CH_2)_4-Br$ | 506, 504, 297, 177 | $C_{25}H_{29}O_6Br$ |
| 61 | 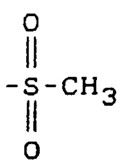 | 448, 391, 283, 151 | $C_{22}H_{24}O_8S$ (448) |
| 62 | 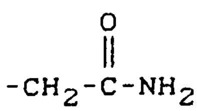 | 427, 370, 312, 295, 269 | $C_{23}H_{25}NO_7$ (447) |
| 63 | 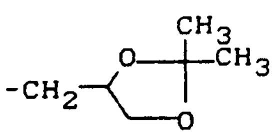 | 484, 469, 370, 353, 269 | $C_{27}H_{32}O_8$ (484) |

Beispiel 64

3-(1-Acetoxy-3-methylbutyl)-11-(1-acetoxy)-4-methoxy-9-methyl-7H-dibenzo[b,g][1,5]dioxocin-5-on

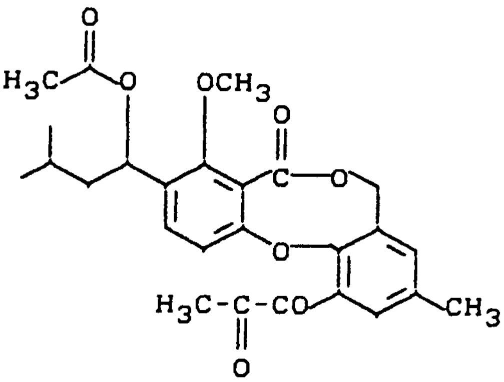

100 mg (0,27 mmol) Penicillide (Ib) werden in 2 ml Pyridin und 1 ml Essigsäureanhydrid 14 h bei 25 °C unter Feuchtigkeitsausschluß gerührt. Die Reaktionsmischung wird im Vakuum eingedampft, der Rückstand in Methylenchlorid aufgenommen und mit Wasser gewaschen. Der Verdampfungsrückstand der getrockneten organischen Phase wird an Kieselgel mit Hexan/Diethylether chromatographiert.
Ausbeute: 61 mg (50% der Theorie) farbloser Feststoff
MS (EI): 456, 414, 354, 315, 298, 285, 269, 219
SF: $C_{25}H_{28}O_8$ (456)

Beispiel 65

11-Hydroxy-4-methoxy-9-methyl-3-(3-methylbutan-1-onyl)-7H-dibenzo[b,g][1,5]dioxocin-5-on

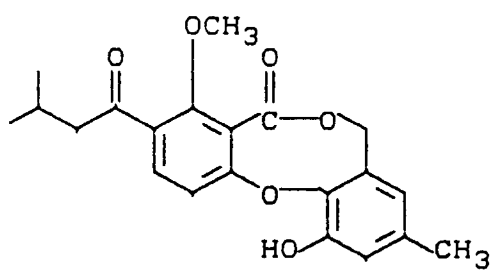

800 mg (2,2 mmol) Penicillide (Ib) werden in 15 ml Methylenchlorid und 880 mg (4,1 mmol) Pyridiniumchlorochromat 3 h bei 25 °C unter Argon gerührt. Nach Zugabe von 200 ml Diethylether wird über Kieselgel Si60 filtriert. Der Verdampfungsrückstand des Filtrats wird an Kieselgel mit Petrolether/Ether = 1:1 chromatographiert.
Ausbeute: 363 mg (45% der Theorie) farbloser Feststoff
MS (EI): 370, 313, 177
SF: $C_{21}H_{22}O_6$ (370)
Analog der Vorschrift für Beispiel 65 wurden die in der folgenden Tabelle 9 aufgeführten Verbindungen hergestellt:

Tabelle 9

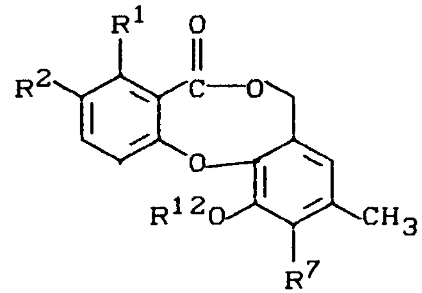

| Bsp. Nr. | $R^1$ | $R^2$ | $R^7$ | $R^{12}$ | MS (EI) | SF |
|---|---|---|---|---|---|---|
| 66 | $OCH_3$ | 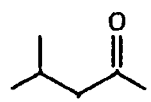 | H | $-CH_2-CH_3$ | 398, 341, 177 | $C_{23}H_{26}O_6$ (398) |
| 67 | $OCH_3$ | 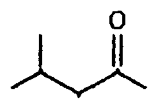 | H | $-CH_2-CH$ $CH_3$ $CH_3$ | 426, 335, 169, 150 | $C_{25}H_{30}O_6$ (426) |
| 68 | $OCH_3$ | 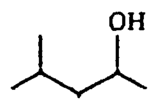 | 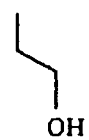 | $CH_3$ | 430, 373 | $C_{24}H_{30}O_7$ (430) |
| 69 | $-O-(CH_2)_2-N$ $C_2H_5$ $C_2H_5$ | 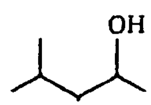 | H | $CH_3$ | 471, 414 | $C_{27}H_{37}NO_6$ (471) |

Beispiel 70

11-Hydroxy-4-methoxy-9-methyl-3-(1-O-tetrahydropyranyl-3-methylbutyl)-7H-dibenzo[b,g][1,5]dioxocin-5-on

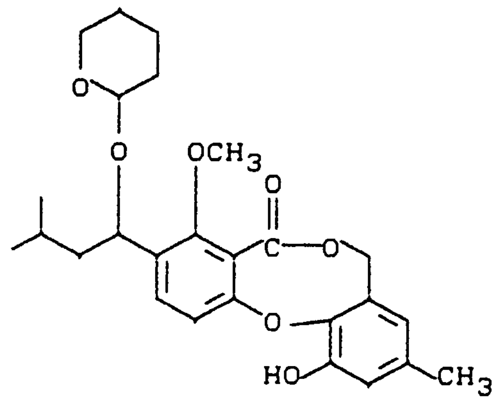

100 mg (0,27 mmol) Penicillide (Ib) werden in 1 ml absolutem Methylenchlorid mit 72 µl (0,8 mmol) Dihydropyran und katalytischen Mengen p-Toluolsulfonsäure 12 h bei 25 °C gerührt. Nach Zugabe von 10 ml Methylenchlorid wird mit wäßrigem Bicarbonat gewaschen, neutral gewaschen, getrocknet und eingedampft. Chromatographie des Rückstands an Kieselgel Si60 in Petrolether/Ether = 2:1 mit fraktionierter Elution liefert 65 mg (53% der Theorie) farblosen Feststoff.
MS (EI): 456, 372, 354, 315, 269
SF: $C_{26}H_{32}O_7$ (456)

Beispiel 71

4,11-Dimethoxy-9-methyl-3-(1-hydroximino-3-methylbutyl-7H-dibenzo[b,g][1,5]dioxocin-5-on

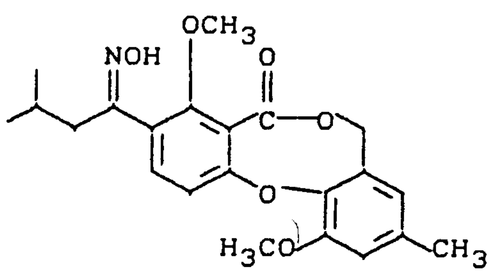

100 mg (0,26 mmol) 4,11-Dimethoxy-9-methyl-3-(1-oxo-3-methylbutyl)-7H-dibenzo[b,g][1,5]dioxocin-5-on werden in 3 ml Eisessig, 36 mg (0,52 mmol) Hydroxylamin-hydrochlorid und 43 mg (0,52 mmol) Natriumacetat 60 h bei 25 °C gerührt. Nach Eindampfen im Hochvakuum wird in Methylenchlorid aufgenommen, mit Wasser gewaschen, getrocknet und der Verdampfungsrückstand an Kiesegel Si60 in Ether/Petrolether = 2:1 chromatographiert.
Ausbeute: 40 mg (39% der Theorie) farbloser Feststoff
MS (EI): 399, 367, 325
SF: $C_{22}H_{25}NO_6$ (399)

Beispiel 72

3-(1-Azido-2-methylbutyl)-11-hydroxy-4-methoxy-9-methyl-7H-dibenzo[b,g][1,5]dioxocin-5-on

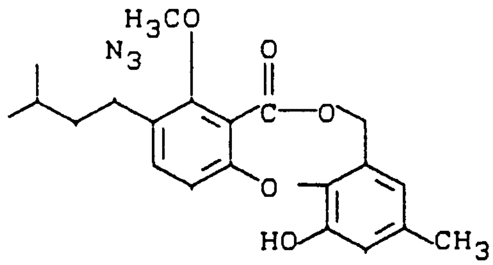

6 g (16,1 mmol) Penicillide (Ib) werden in 100 ml Tetrahydrofuran mit 4,6 g (17,6 mmol) Triphenylphosphin und 29,3 ml einer frisch bereiteten 0.6 n Lösung von Stickstoffwassersäure in Toluol versetzt. Hierzu werden bei 0 °C unter Argon 2,7 ml (17,6 mmol) Azodicarbonsäurediethylester hinzugetropft. Anschließend wird 12 h bei 25 °C gerührt. Nach Verdünnen mit Methylenchlorid wird mit wäßrigem Bicarbonat gewaschen, getrocknet und eingedampft. Chromatographie an Kieselgel Si60 in Diethylether/Petrolether = 2:1, liefert 5,6 g (88% der Theorie) farblosen Feststoff.
MS( EI): 397, 355, 299, 176, 163
SF: $C_{21}H_{23}N_3O_5$ (397)

Beispiel 73

3-(1-Azido-2-methylbutyl)-4,11-dimethoxy-9-methyl-7H-dibenzo[b,g][1,5]dioxocin-5-on

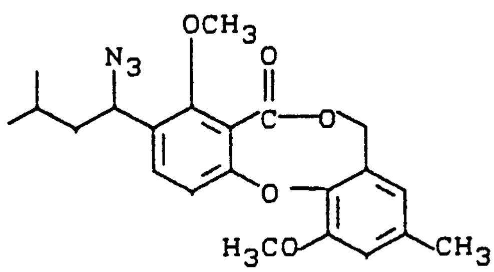

Die Titelverbindung wurde analog der Vorschrift für Beispiel 72 dargestellt.
MS (EI): 411, 369, 314, 383, 163
SF: $C_{22}H_{25}N_3O_5$ (411)

Beispiel 74

3-(1-Amino-3-methylbutyl)-11-hydroxy-4-methoxy-9-methyl-7H-dibenzo[b,g][1,5]dioxocin-5-on

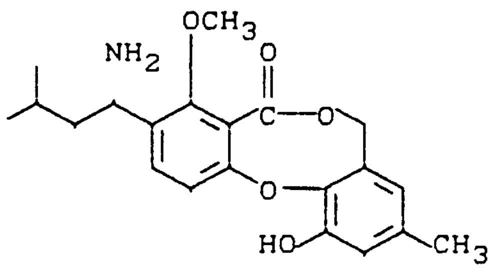

Zu 5,63 g (14 mmol) der Verbindung aus Beispiel 72 werden 63,3 ml einer Lösung von 20 g Borsäure und 20 g Nickeldichloridhexahydrat in 500 ml Ethanol hinzugefügt. Dazu werden 1,1 g (28,4 mmol) Natriumboranat in 20 ml Ethanol schnell hinzugetropft. Nach Rühren bei 25 °C für 12 h wird im Vakuum eingedampft, mit Methylenchlorid aufgenommen und mit Wasser gewaschen. Nach Trocknen und Eindampfen wird an Kieselgel Si60 in Methylenchlorid/Methanol/konzentriertem wäßrigem Ammoniak = 200:10:1 chromatographisch gereinigt.
Ausbeute: 3,9 g (76% der Theorie) farbloser Feststoff.
MS (DCI): 372, 355
SF: $C_{21}H_{25}NO_5$ (371)

Beispiel 75

3-(1-Amino-2-methylbutyl)-4,11-dimethoxy-9-methyl-7H-dibenzo[b,g][1,5]dioxocin-5-on

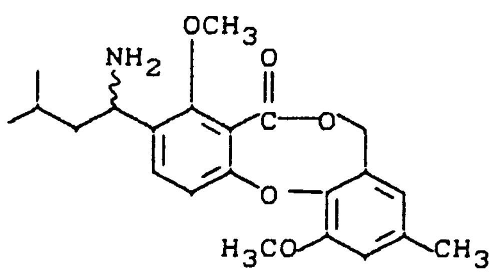

Die Verbindung wurde analog der Vorschrift für Beispiel 74 hergestellt.
MS (FAB): 386, 369, 355
SF: $C_{22}H_{27}NO_5$ (385)

Beispiel 76

11-(4-Diethylaminobutyloxy)-3-(1-hydroxy-3-methylbutyl)-4-methoxy-9-methyl-7H-dibenzo[b,g][1,5]dioxocin-5-on

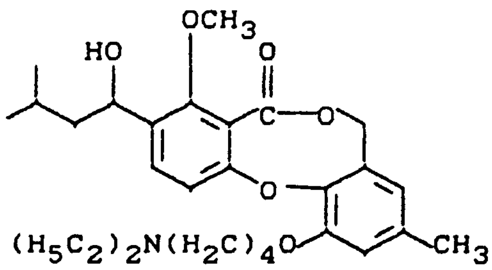

118 mg (0,23 mmol) der Verbindung aus Beispiel 10 werden mit 1 ml Dimethylformamid, 48 µl (0,46 mmol) Diethylamin und katalytischen Mengen an Natriumjodid 12 h bei 70 °C unter Argon gerührt. Nach Eindampfen im Hochvakuum wird in Methylenchlorid aufgenommen, mit Wasser gewaschen, getrocknet und eingedampft. Chromatographie an Kieselgel Si60 in Methylenchlorid/Methanol/konzentriertem wäßrigem Ammoniak liefert 86 mg (74% der Theorie) farblosen Feststoff.
MS (DCI): 500, 484
SF: $C_{29}H_{41}NO_6$ (499)

Beispiel 77

3-(1-Hydroxy-3-methylbutyryl)-4-methoxy-9-methyl-11-O-methylsulfonyl-7H-dibenzo[b,g][1,5]dioxocin-5-on

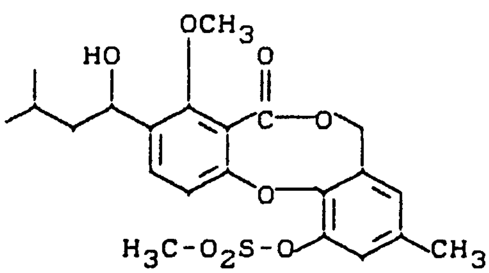

150 mg (0,4 mmol) Penicillide (Ib) in 3 ml Methylenchlorid werden bei 0 °C unter Argon mit 0,14 ml (1,0 mmol) Triethylamin und 86 µl (1,1 mmol) Methansulfonsäurechlorid versetzt und 12 h bei 25 °C gerührt. Ansäuern auf pH 2, Extraktion mit Methylenchlorid, Waschen, Trocknen und Eindampfen liefert nach Chromatographie an Kieselgel Si60 in Ether/Petrolether 2:1 75 mg (41% der Theorie) farblosen Feststoff.
MS (EI): 450, 393, 365, 347
SF: $C_{22}H_{26}O_8S$ (450)

Beispiel 78

4,11 Dimethoxy-3-(1-hydroxypropyl)-9-methyl-7H-dibenzo[b,g][1,5]dioxocin-5-on

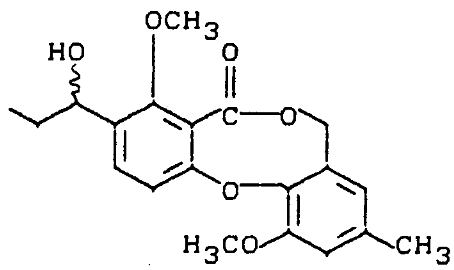

53 mg (0,15 mmol) der Verbindung aus Beispiel 101 in 4 ml absolutem Tetrahydrofuran werden unter Argon bei 0 °C mit 0,7 ml (1,4 mmol) einer 2 M Ethylmagnesiumbromid-Lösung in Tetrahydrofuran versetzt und 1 h bei 25 °C gerührt. Nach Zugabe von 2 ml 1 N Salzsäure wird mit Methylenchlorid verdünnt, mit Wasser gewaschen, getrocknet und eingedampft. Nach chromatographischer Reinigung an Kieselgel Si60 (Petrolether/Ether-Gradient) fällt das Hauptprodukt 36 mg (62% der Theorie) als farbloser Feststoff an.
MS (EI): 358, 329, 311, 299, 283, 242
SF: $C_{25}H_{32}O_8$ (358)

Die in Tabelle 10 aufgeführten Beispiele wurden analog der Vorschrift für Beispiel 78 hergestellt.

Tabelle 10

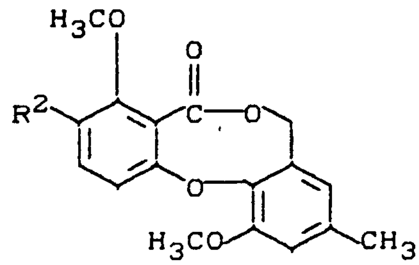

| Bsp. Nr. | $R^2$ | MS: (EI) | SF |
|---|---|---|---|
| 79 | 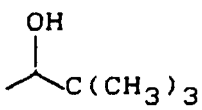 | 386, 329, 311, 299, 283, 242 | $C_{22}H_{26}O_6$ (386) |
| 80 | 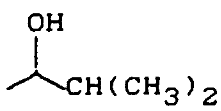 | 372, 329, 311, 299, 283 | $C_{21}H_{24}O_6$ (372) |
| 81 | 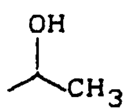 | 344, 283 | $C_{19}H_{20}O_6$ (344) |
| 82 | 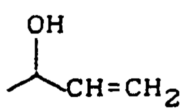 | 356, | $C_{20}H_{20}O_6$ (356) |
| 83 | 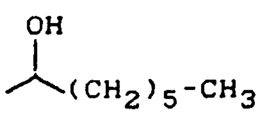 | 414, 329, 283, 242 | $C_{24}H_{30}O_6$ (414) |
| 84 | 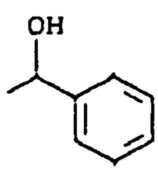 | 406, 325, 262 | $C_{24}H_{22}O_6$ (406) |
| 85 | 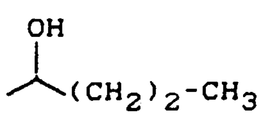 | 372, 329, 311, 299, 283, 242 | $C_{21}H_{24}O_6$ (372) |

Fortsetzung Tabelle 10

| Bsp. Nr. | $R^2$ | MS: (EI) | SF |
|---|---|---|---|
| 86 | 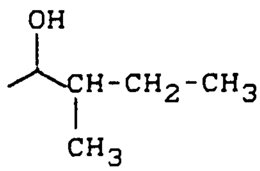 | 386, 311, 299, 283, 242 | $C_{22}H_{26}O_6$ (386) |
| 87 | 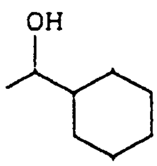 | 412, 329, 283 | $C_{24}H_{28}O_6$ (412) |
| 88 | 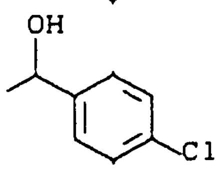 | 440, 283, 167 | $C_{24}H_{21}ClO_6$ (440.5) |
| 89 | 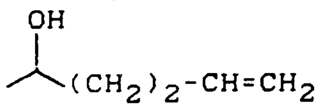 | 384, 329, 283, 242, | $C_{22}H_{24}O_6$ (384) |
| 90 | 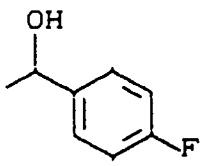 | 424, 135 123, 109 | $C_{24}H_{21}O_6F$ (424) |
| 91 | 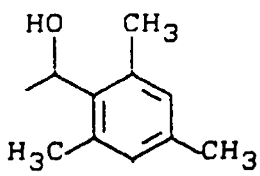 | 448, 399, 301, 271, 242 | $C_{27}H_{28}O_6$ (448) |
| 92 | 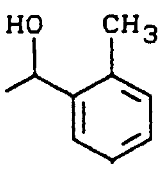 | 420, 402, 269, 119, 44 | $C_{25}H_{24}O_5$ (420) |
| 93 | 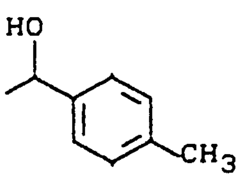 | 420, 402, 143, 119 | $C_{25}H_{24}O_6$ (420) |

Beispiel 94

3-(1-Chloro-3-methylbutyl)-11-hydroxy-4-methoxy-9-methyl-7H-dibenzo[b,g][1,5]dioxocin-5-on

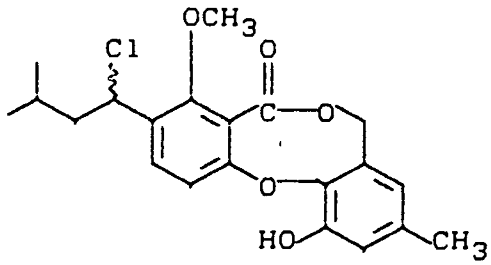

200 mg (0,54 mmol) Penicillide (Ib) in 1 ml Dioxan und 0,38 ml (5,2 mmol) Thionylchlorid werden 5 h unter Rückfluß gekocht (Feuchtigkeitsausschluß). Nach Eindampfen im Vakuum wird in Methylenchlorid aufgenommen, mit wäßrigem Bicarbonat, dann mit Wasser gewaschen, getrocknet und eingedampft. Chromatographie an Kieselgel Si60 in Ether/Petrolether = 2:1 liefert 187 mg (93% der Theorie) farblosen Feststoff.

$^1$H-NMR ($CDCl_3$): δ = 7,63 (1H, d); 6,91 (1H, d); 6,87 (1H, d); 6,40 (1H, d); 6,09 (1H, s); 5,40 (1H, mc); 5,09 (1H, mc); 4,03 (3H, s); 2,26 (3H, s); 2,05 (1H, mc); 1,75 (2H, mc); 0,95 (6H, d).

SF: $C_{21}H_{23}ClO_5$ (390.4)

Beispiel 95

3-(1-Chloro-3-methylbutyl)-4,11-dimethoxy-9-methyl-7H-dibenzo[b,g][1,5]dioxocin-5-on

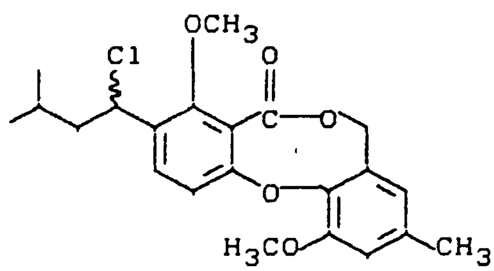

Die Titelverbindung wurde analog der Vorschrift für Beispiel 94 hergestellt.
MS (EI): 406, 404, 313, 163
SF: $C_{22}N_{25}ClO_5$ (404,4)

Beispiel 96

4,11-Dimethoxy-9-methyl-3-(3-methylbutyl)-7H-dibenzo[b,g][1,5]-dioxocin-5-on

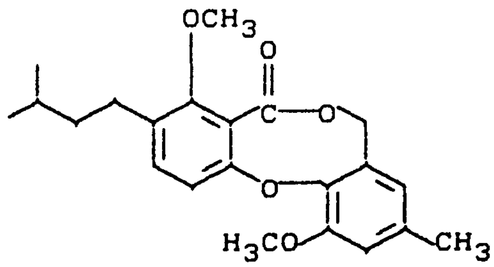

Die Titelverbindung wurde analog der Vorschrift für Beispiel 97 hergestellt.
MS (EI): 370, 342, 325, 313, 299
SF: $C_{22}H_{26}O_5$ (370)

Beispiel 97

11-Hydroxy-4-methoxy-9-methyl-3-(3-methylbutyl)-7H-dibenzo[b,g][1,5]dioxocin-5-on

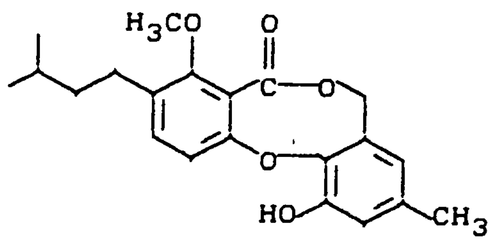

131 mg (0,3 mmol) der Verbindung aus Beispiel 94 in 2,5 ml Toluol und 200 µl (0,74 mmol) Tributylzinnhydrid werden unter Rückfluß erhitzt. Nach Zugabe von 29 mg (0,18 mmol) 2,2'-Azodiisobuttersäurenitril wird noch 2 h unter Rückfluß gekocht, im Vakuum eingedampft und Rückstand an Kieselgel Si60 mit Petrolether/Ether = 5:1 chromatographisch gereinigt.
Ausbeute: 128 mg (90% der Theorie) farblose Kristalle aus Diethylether
MS (EI): 356, 299, 220, 163
SF: $C_{21}H_{24}O_5$ (356)

Beispiel 98

4,11-Dimethoxy-9-methyl-3-(3-methyl-1-butenyl)-7H-dibenzo[b,g][1,5]dioxocin-5-on

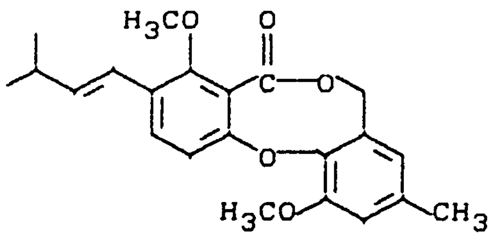

1,1 g (3,0 mmol) der Verbindung aus Beispiel 95 in 10 ml Dimethylformamid, 2,2 ml (15,3 mmol) 7-Methyl-1,5,7-triazabicyclo[4.4.0]dec-5-en und 0,65 g Kupfer-I-oxid werden in einer Argonatmosphäre 12 h bei 80 °C gerührt. Nach Filtration wird im Vakuum eingedampft, mit Methylenchlorid aufgenommen, 1 N wäßrige Salzsäure zugegeben, dann mit Wasser gewaschen, getrocknet und eingedampft. Chromatographie an Kieselgel Si60 in Petrolether/Ether = 3:1 liefert 730 mg (65% der Theorie) glasartigen Feststoff.
MS (EI): 368, 353, 335, 323, 218, 203
SF: $C_{22}H_{25}O_5$ (368)

Beispiel 99

11-Hydroxy-4-methoxy-9-methyl-3-(1-N-((S)-2-pyrrolidon-5-carbonamido)-3-methylbutyl)-7H-dibenzo[b,g]-[1,5]-dioxocin-5-on

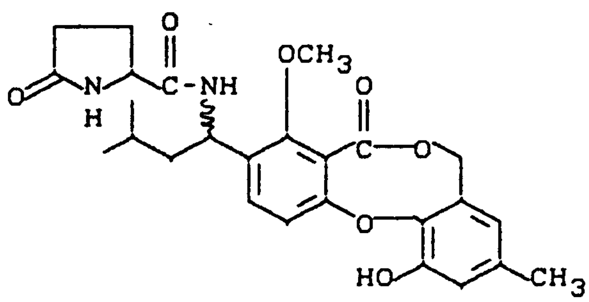

63 mg (0,49 mmol) (S)-2-Pyrrolidon-5-carbonsäure wird unter Argon bei -55 °C mit 85 µl (0,49 mmol) Diisopropylethylamin in 1 ml Dimethylformamid versetzt. Nach Zugabe von 38 µl (0,54 mmol) Methansulfonsäurechlorid wird 30 Minuten bei -55 °C gerührt. Danach werden 166 µl (1,2 mmol) Triethylamin und 0,2 g (0,54 mmol) der Verbindung aus Beispiel 74 in 1 ml Dimethylformamid hinzugetropft. Nach 30 Minuten Rühren bei 25 °C wird mit Methylenchlorid verdünnt und bei pH 3-4 mit Wasser gewaschen, getrocknet und eingedampft. Chromatographie an Kieselgel Si60 in Methylenchlorid/Methanol 20:1 ergibt 92 mg (35% der Theorie) farblosen Feststoff.
MS (DCI): 483, 425, 220, 186
SF: $C_{26}H_{30}N_2O_7$ (482)

Beispiel 100

4,11-Dimethoxy-3-(1,2-epoxy-3-methylbutyl)-9-methyl-7H-dibenzo[b,g][1,5]dioxocin-5-on

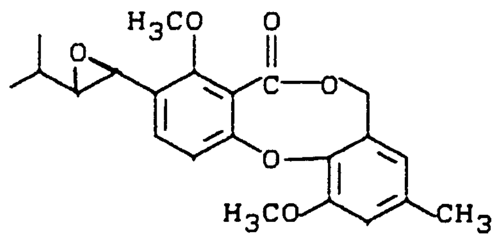

43 mg (0,12 mmol) der Verbindung aus Beispiel 98 werden in 2 ml Dichlormethan mit einem Überschuß an meta-Chlorperbenzoesäure 3 h bei 25 °C gerührt. Nach Waschen mit wäßrigem Bicarbonat, Trocknen und Eindampfen wird an Kieselgel Si60 in Petrolether/Ether 1:1 chromatographiert. In der Hauptfraktion fallen 12 mg (27% der Theorie) farbloser Feststoff an.
MS (EI): 384, 267, 239, 234, 219, 191
SF: $C_{22}H_{24}O_6$ (384)

Beispiel 101

4,11-Dimethoxy-9-methyl-3-formyl-7H-dibenzo[b,g][1,5]dioxocin-5-on

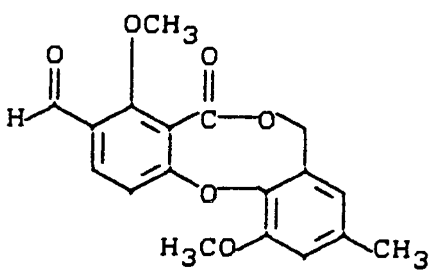

5,6 g (15,2 mmol) der Verbindung aus Beispiel 98 in 100 ml Methylenchlorid und 10 ml Methanol wird bei -60 °C in einer Standardapparatur (Organikum, 9. Auflage, Seite 298 ff., VEB Deutscher Verlag der Wissenschaften] ein Ozon-/Sauerstoffgemisch bis zur Sättigung eingeleitet. Nach Zugabe von 19 ml Tributylphosphin wird unter Rühren auf 25 °C aufgewärmt, mit Hexan gewaschen und an Kiesel Si60 in Ether/Petrolether 1:1 chromatographiert.
Ausbeute: 2,0 g (40% der Theorie) farblose Kristalle
MS (EI): 328, 300, 299, 283, 269
SF: $C_{18}H_{16}O_6$ (328)

Beispiel 102

3-(1,4-Dihydroxybutyryl)-4,11-dimethoxy-9-methyl-7H-dibenzo[b,g][1,5]dioxocin-5-on

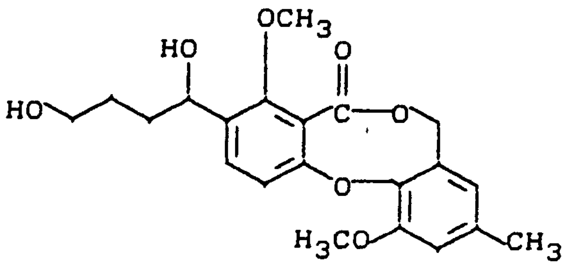

Die nach dem Verfahren von Beispiel 101 aus 100 mg (0,26 mmol) der Verbindung aus Beispiel 89 dargestellten Primärozonide werden durch Zugabe eines Überschußes an Natriumborhydrid reduziert.
MS (EI):
SF: $C_{21}H_{24}O_7$ (388)

Beispiel 103

3,9-Dimethyl-11-methoxy-7H-dibenzo[b,g][1,5]dioxocin-5-on

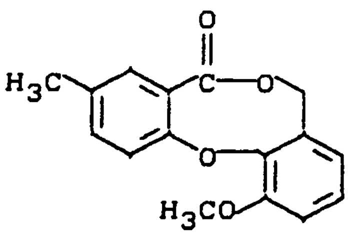

100 mg (0,32 mmol) 2-(2-methoxy-6-chlormethyl)-5-methylphenoxybenzoesäure in 2 m absolutem Dimethylformamid und 54 mg (0,48 mmol) 1,8-Diazabiyclo[5.4.0]undec-7-en werden 12 h bei 80 °C unter Argon gerührt. Nach Eindampfen im Hochvakuum wird mit Methylenchlorid aufgenommen, mit Wasser gewaschen, getrocknet und eingedampft. Chromatographie an Kieselgel Si60 in Methylenchlorid lieferte 17 mg (20% der Theorie) farblosen Feststoff.
MS (EI): 270, 255, 241, 225
SF: $C_{16}H_{14}O_4$ (270)

Beispiel 104

11-Carboxymethyloxy-3-(1-formyloxy-3-methylbutyl)-4-methoxy-9-methyl-7H-dibenzo[b,g][1,5]dioxocin-5-on

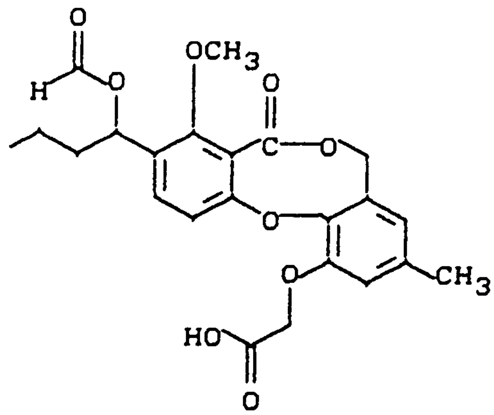

100 mg (0,2 mmol) der Verbindung aus Beispiel 11 werden in 2 ml Ameisensäure 2 h bei 25 °C gerührt. Nach Eindampfen im Hochvakuum fallen 90 mg (96% der Theorie) farbloser Feststoff aus.
SF: $C_{24}H_{26}O_9$ (458)
$^1$H-NMR (δ ppm): 8,08 (1H, s); 7,48 (1H, d); 7,05 (1H, d); 6,70 (1H, s); 6,55 (1H, s); 6,28 (1H, mc); 5,7 (1H, breit); 5,60 (2H, mc); 4,8 (2H, s); 4,02 (3H, s); 2,38 (3H, s); 1,81 (1H, mc); 1,65 (1H, mc); 1,51 (1H, mc); 0,95 (6H, d).

Beispiel 105

3-(1-Hydroxy-3-methylbutyl)-4-methoxy-11-(2-hydroxy-3-methoxypropyloxy)-9-methyl-7H-dibenzo[b,g][1,5]-dioxocin-5-on

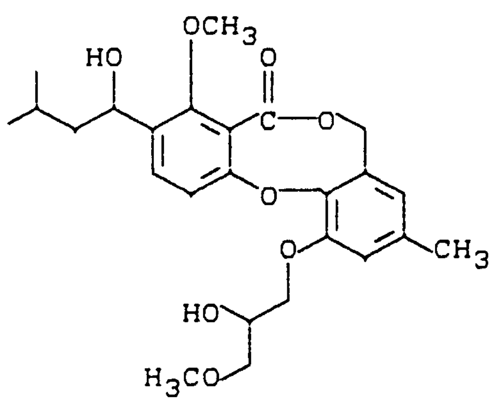

100 mg (0,23 mmol) der Verbindung aus Beispiel 24 werden 2,5 h bei 25 °C in 1%iger methanolischer Schwefelsäure gerührt. Nach Verdünnen mit Methylenchlorid erfolgt Waschen mit Wasser und wäßrigem Bicarbonat. Nach Trocknung, Eindampfen und Chromatographie an Kieselgel Si60 in Diethylether fallen 22 mg (20% der Theorie) farbloser Farbstoff an.
MS (EI): 460, 389
SF: $C_{25}H_{32}O_8$ (460)

Beispiel 106

11-(2,3-Dihydroxy-propyloxy)-3-(1-hydroxy-3-methylbutyl)-4-methoxy-9-methyl-7H-dibenzo[b,g][1,5]dioxocin-5-on

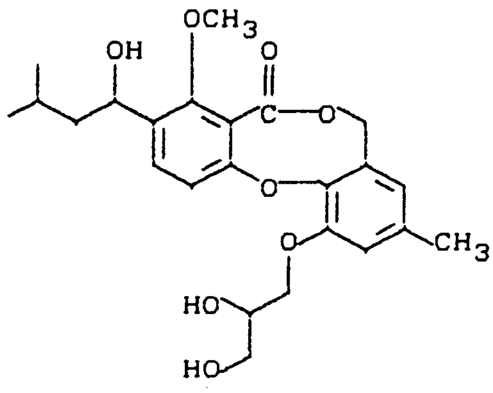

100 mg (0,23 mmol) der Verbindung aus Beispiel 24 werden 12 h bei 25 °C in 2 ml Tetrahydrofuran und 0,5 ml wäßriger 1%iger Perchlorsäure gerührt. Nach Zugabe von Methylenchlorid wird mit Wasser und wäßrigem Bicarbonat gewaschen, getrocknet und eingedampft. Nach Chromatographie an Kieselgel Si60 in Methylenchlorid/Methanol 95:5 fallen 12 mg (11% der Theorie) farbloser Feststoff an.
MS (EI): 446, 389, 359, 353, 269, 179
SF: $C_{24}H_{30}O_8$ (446)

Beispiel 107 und Beispiel 108

10-Bromo-11-hydroxy-3-(1-hydroxy-3-methylbutyl)-4-methoxy-9-methyl-7H-dibenzo[b,g][1,5]dioxocin-5-on (Beispiel 108) und
8-Bromo-11-hydroxy-3-(1-hydroxy-3-methylbutyl)-4-methoxy-9-methyl-7H-dibenzo[b,g][1,5]dioxocin-5-on (Beispiel 107)

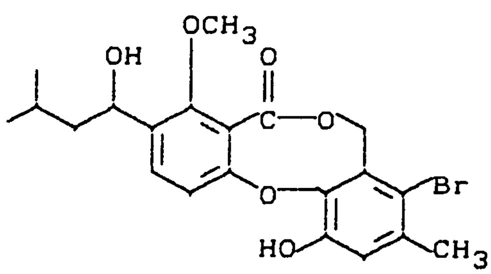

(Beispiel 107)

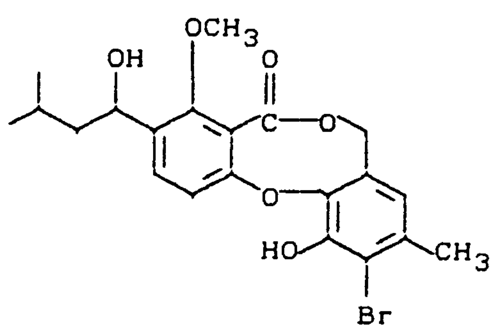

(Beispiel 108)

1 g (2,7 mmol) Penicillide (Ib) werden mit 0,8 g (5,7 mmol) Kaliumcarbonat und 2 ml (25 mmol) Bromnitromethan in 6 ml Dimethylformamid 23 h bei 90 °C unter Argon gerührt. Nach Eindampfen im Hochvakuum wird in 1 N wäßriger Salzsäure aufgenommen und erschöpfend mit Diethylether extrahiert. Nach Trocknen und Eindampfen wird an Kieselgel Si60 in Methylenchlorid/Ethylacetat 10:1 in 2 Fraktionen getrennt, die nach Eindampfen im Vakuum jeweils an Kieselgel RP8 in Acetonitril/Wasser 68:32 nachgetrennt werden und neben Ausgangsmaterial 3 chromatographisch einheitliche Produkte ergeben.

Beispiel 107

Ausbeute: 457 mg (37% der Theorie) farbloser Feststoff
MS (EI): 452, 450, 395, 393, 349, 347, 179
SF: $C_{21}H_{23}O_6Br$ (451)

Beispiel 108

Ausbeute: 36 mg (2,9% der Theorie) farbloser Feststoff
MS (EI): 452, 450, 395, 393, 349, 347, 179
SF: $C_{21}H_{23}O_6Br$ (451)
Außerdem entstehen bei der Darstellung der Beispiele 107 und 108 288 mg (20% der Theorie) der Verbindung (Beispiel 107a) 8,10-Dibromo-11-hydroxy-3-(1-hydroxy-3-methylbutyl)-4-methoxy-9-methyl-7H-dibenzo[b,g][1,5]dioxocin-5-on [Tetrahedron Letters 45, 3941, (1974)].

Beispiel 109

4-Hydroxy-11-methoxy-9-methyl-3-(3-methyl-1-butanonyl)-7H-dibenzo[b,g][1,5]dioxocin-5-on

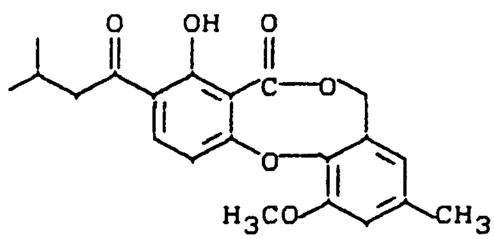

Zu einer Lösung von 6,2 g (16,1 mmol) 3-(3-Methyl-1-butanonyl)-4,11-dimethoxy-9-methyl-7H-dibenzo-[b,g][1,5]dioxocin-5-on in 160 ml Dichlormethan werden unter Argon bei -70 °C 10,8 ml einer 1 M Bortribromid-Lösung in Dichlormethan zugetropft. Nach 1,5 h werden 6 ml Methanol zugegeben und nach weiteren 40 Minuten wird die Lösung auf Raumtemperatur gebracht. Es wird mit Natriumhydrogencarbonatlösung und Wasser gewaschen, über Magnesiumsulfat getrocknet und im Vakuum eingeengt. Der Rückstand wird auf einer Kieselgelsäule mit Petrolether/Essigester 6:1 gereinigt.
Ausbeute: 4,0 g (67% der Theorie)
MS (EI): $M^+ = 370$
SF: $C_{21}H_{22}O_6$
Analog der Vorschrift für Beispiel 109 werden die in Tabelle 11 aufgeführten Beispiele hergestellt:

Tabelle 11

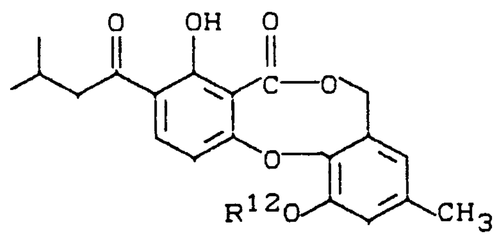

| Bsp. Nr. | $R^{12}$ | MS: (EI) | SF |
|---|---|---|---|
| 110 | H | 356, 299, 221, 137 | $C_{20}H_{20}O_6$ (356) |
| 111 | $-CH_2-CH_3$ | 384, 165, 164, 149, 136 | $C_{22}H_{24}O_6$ (384) |
| 112 | $-CH_2-CH(CH_3)_2$ | 412, 221, 150, 136 | $C_{24}H_{28}O_6$ (412) |

Beispiel 113

4-Hydroxy-3-(1-hydroxy-3-methylbutyl)-11-methoxy-9-methyl-7H-dibenzo[b,g][1,5]dioxocin-5-on

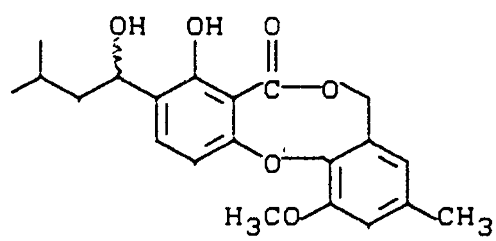

2,2 g (5,9 mmol) der Verbindung aus Beispiel 109 werden in 40 ml Tetrahydrofuran gelöst, mit 7 ml Eisessig und 0,96 g (15 mmol) Natriumcyanoborhydrid versetzt. Nach 14 h bei Raumtemperatur wird der Ansatz im Vakuum eingeengt, der Rückstand in Dichlormethan aufgenommen, mit Natriumhydrogencarbonatlösung und Wasser gewaschen, über Magnesiumsulfat getrocknet und im Vakuum eingeengt. Die Reinigung erfolgt säulenchromatographisch an Kieselgel mit Dichlormethan/Methanol 150/1 als Laufmittel.
Ausbeute: 1,3 g (39% der Theorie)
MS (EI): $M^+$ = 372
SF: $C_{21}H_{24}O_6$
Analog der Vorschrift für Beispiel 113 wurden die in den folgenden Tabellen 12 und 13 aufgeführten Verbindungen hergestellt:

Tabelle 12

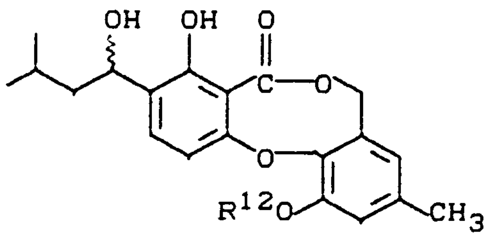

| Bsp. Nr. | $R^{12}$ | MS: (EI) | SF |
|---|---|---|---|
| 114 | H | 358, 301, 283, 165, 137 | $C_{20}H_{22}O_6$ (358) |
| 115 | $-CH_2-CH_3$ | 386, 368, 311, 165 | $C_{22}H_{26}O_6$ (386) |
| 116 | $-CH_2-CH(CH_3)_2$ | 414, 357, 339, 193 | $C_{24}H_{30}O_6$ (414) |

Tabelle 13

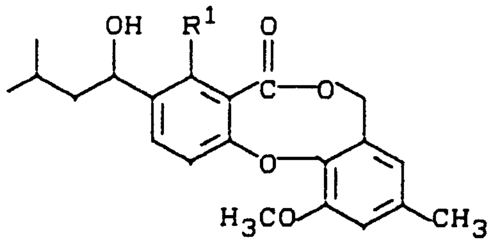

| Bsp. Nr. | $R^1$ | MS: (EI) | SF |
|---|---|---|---|
| 117 | 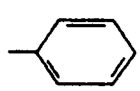 | 432, 345, 329, 303, 288 | $C_{27}H_{28}O_5$ (432) |

Fortsetzung Tabelle 13

| Bsp. Nr. | $R^1$ | MS: (EI) | SF |
|---|---|---|---|
| 118 | $-CH_2-$ 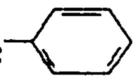 | 446, 311, 239 | $C_{28}H_{30}O_5$ (446) |
| 119 | $-CH=CH_2$ | 382, 281, 175 | $C_{23}H_{26}O_5$ (382) |
| 120 | $-CH_2-CH_3$ | 384, 327 177, 151 | |

Beispiel 121

3-[1-(R,S)-Hydroxy-3-methylbutyl]-4,11-dimethoxy-9-methyl-7H-dibenzo[b,g][1,5]dioxocin-5-on

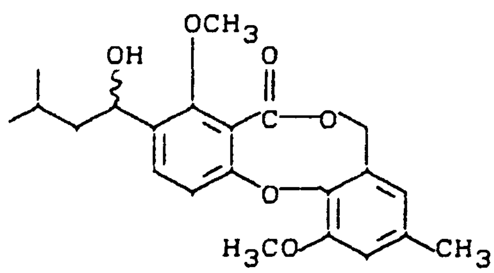

158 mg (0,41 mmol) 3-(3-Methyl-1-butanonyl)-4,11-dimethoxy-9-methyl-7H-dibenzo[b,g][1,5]dioxocin-5-on werden in 2 ml Tetrahydrofuran und 0,1 ml Wasser gelöst. Die Lösung wird mit 23 mg (0,61 mmol) Natriumborhydrid versetzt und über Nacht bei Raumtemperatur gerührt. Überschüssiges Natriumborhydrid wird durch Zugabe von einigen Tropfen Essigsäure zerstört. Es wird im Vakuum eingeengt, der Rückstand in Dichlormethan aufgenommen und mit Wasser gewaschen. Die organische Phase wird über Magnesiumsulfat getrocknet, eingeengt und säulenchromatographisch an Kieselgel mit Petrolether/Diethylether 1:1 gereinigt.
Ausbeute: 73% der Theorie
MS (EI): $M^+$ = 386
SF: $C_{22}H_{26}O_6$

Beispiel 122

4,11-Dimethoxy-3-hydroxymethyl-9-methyl-7H-dibenzo[b,g][1,5]dioxocin-5-on

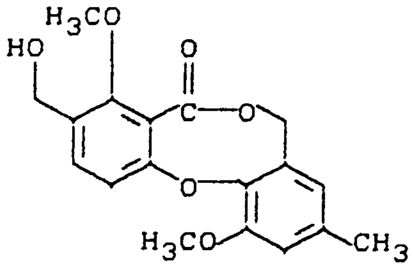

Die Titelverbindung wurde analog der Vorschrift für Beispiel 121 hergestellt.
Ausbeute:
MS (EI): 330, 180, 150
SF: $C_{18}H_{18}O_6$ (330)
Analog der Vorschrift von Beispiel 121 werden die in der folgenden Tabelle 14 aufgeführten Beispiele hergestellt:

Tabelle 14

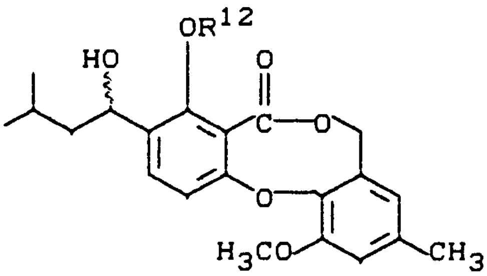

| Bsp. Nr. | $R^{12}$ | MS: (EI) | SF |
|---|---|---|---|
| 123 | 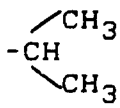 | 414, 315, 297, 151 | $C_{24}H_{30}O_6$ (414) |
| 124 | $-CH_2-CH=CH_2$ | 412, 355, 297, 151 | $C_{24}H_{28}O_6$ (412) |
| 125 | 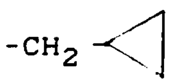 | 426, 369, 297, 285, 269 | $C_{25}H_{30}O_6$ (426) |
| 126 | 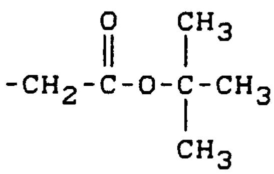 | DCI: 504 ($M^+NH_4^+$) 486, 469, 430, 413 | $C_{27}H_{34}O_8$ (486) |
| 127 | 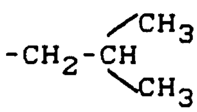 | 428, 371, 315, 297, 151 | $C_{25}H_{32}O_6$ (428) |
| 128 | $-CH_2-CH_3$ | 400, 343, 297, 269 | $C_{23}H_{28}O_6$ (400) |
| 129 | 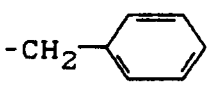 | 462, 405, 297, 269 | $C_{28}H_{30}O_6$ (462) |

Fortsetzung Tabelle 14

| Bsp. Nr. | $R^{12}$ | MS: (EI) | SF |
|---|---|---|---|
| 130 | $-CH_2-CH_2-CH_3$ | 414, 357, 297, 151 | $C_{24}H_{30}O_6$ (414) |
| 131 | $-CH_2-CH_2-$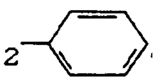 | DCI: 494($M^+NH_4^+$) 476, 459, 419, 355 | $C_{29}H_{32}O_6$ (476) |
| 132 | $-CH(C_6H_5)_2$ | FAB: 521, 355, 167 | $C_{34}H_{34}O_6$ (538) |
| 133 | $-(CH_2)_4-Br$ | 508, 506, 451, 449, 297 | $C_{25}H_{21}O_6Br$ (507) |
| 134 | 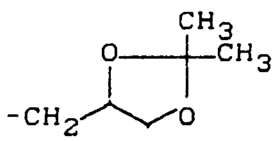 | 486, 429, 371, 297, 269 | $C_{28}H_{36}O_8$ (486) |
| 135 | $-CH_2-$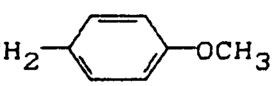 | FAB: 515(M+Na$^+$), 492, 475, 355, 154 | $C_{29}H_{32}O_7$ (492) |
| 136 | 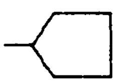 | 440, 315, 297, 151 | $C_{26}H_{32}O_6$ (440) |
| 137 | $-CH_2-C(=O)-NH_2$ | 429, 412, 372, 353, 297 | $C_{23}H_{27}NO_7$ (429) |
| 138 | $-CH_2-CH_2-Br$ | 480, 478, 462, 460, 343, 297 | $C_{23}H_{27}BrO_6$ (479) |

Beispiel 139

3-(1-Hydroxy-3-methylbutyl)-4-(2,3-dihydroxypropoxy)-11-methoxy-9-methyl-7H-dibenzo[b,g][1,5]dioxocin-5-on

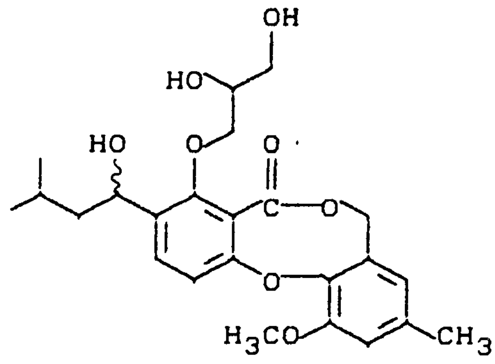

100 mg (0,21 mmol) der Verbindung aus Beispiel 134 werden in 5 ml 80%iger Essigsäure gelöst. Nach einer Reaktionszeit von 5 h bei Raumtemperatur wird die Lösung im Vakuum eingeengt, zweimal mit Toluol codestilliert und säulenchromatographisch an Kieselgel mit Dichlormethan/Methanol 25:1 gereinigt.
Ausbeute: 53 mg (58% der Theorie)
MS (EI): 446, 239, 151
SF: $C_{24}H_{30}O_8$ (446)

Beispiel 140

3-(1-Hydroxy-1,3-dimethylbutyl)-4,11-dimethoxy-9-methyl-7H-dibenzo[b,g][1,5]dioxocin-5-on

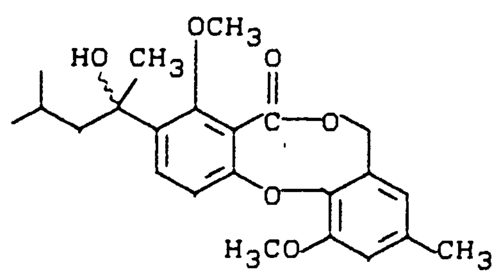

100 mg (0,26 mmol) 4,11-Dimethoxy-9-methyl-3-(3-methylbutan-1-onyl)-7H-dibenzo[b,g][1,5]dioxocin-5-on werden unter Argon in 5 ml absolutem Tetrahydrofuran gelöst und bei Raumtemperatur mit 0,2 ml (0,6 mmol) einer 3 M Methylmagnesiumbromidlösung in Diethylether versetzt. Nach 4 h wird die Reaktion durch Zugabe von 0,2 ml Ammoniumchloridlösung abgebrochen. Es wird im Vakuum eingeengt, in Dichlormethan aufgenommen und mit Wasser gewaschen. Nach dem Trocknen der organischen Phase über Magnesiumsulfat wird eingeengt und der Rückstand säulenchromatographisch an Kieselgel mit Petrolether/Essigester 10:1 gereinigt.
Ausbeute: 52 mg (50% der Theorie)
MS (EI): 400, 343, 283
SF: $C_{23}H_{28}O_6$ (400)

Beispiel 141

4-p-Chlorphenyl-3-(1-p-chlorphenyl-1-hydroxy-3-methylbutyl)-11-methoxy-9-methyl-7H-dibenzo[b,g][1,5]-dioxocin-5-on

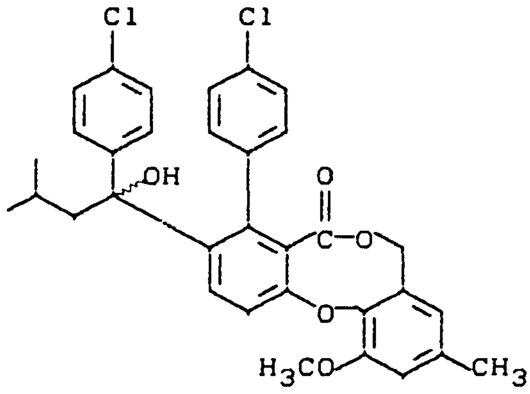

Die Titelverbindung wurde analog der Vorschrift zu Beispiel 140 hergestellt.
Ausbeute: 46% der Theorie
MS (EI): 578, 576, 519, 478, 363
SF: $C_{33}H_{30}Cl_2O_5$
Analog der Vorschrift für Beispiel 140 wurden die in den folgenden Tabellen 16 und 17 aufgeführten Verbindungen hergestellt.

Tabelle 16

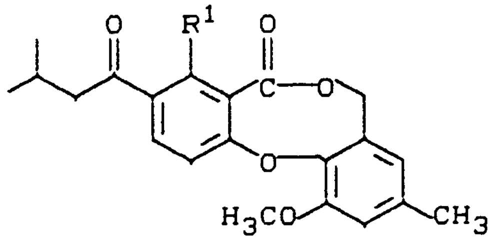

| Bsp. Nr. | $R^1$ | MS: (EI) | SF |
|---|---|---|---|
| 142 | 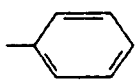 | 430, 373, 329, 223 | $C_{27}H_{26}O_5$ (430) |
| 143 | $-CH_2$ 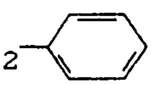 | 444, 401 | $C_{28}H_{28}O_5$ (444) |
| 144 | $-CH=CH_2$ | 380, 365, 323, 274, 173 | $C_{23}H_{24}O_5$ (380) |
| 145 | $-CH_2-CH_3$ | 382, 339, 325, 201, 175, | $C_{23}H_{26}O_5$ (382) |

Tabelle 17

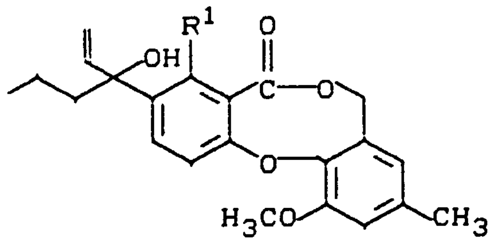

| Bsp. Nr. | $R^1$ | MS: (EI) | SF |
|---|---|---|---|
| 146 | $OCH_3$ | 412, 355, 357, 311, 283, | $C_{24}H_{28}O_6$ (412) |
| 147 | 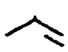 | 408, 351, 323, 307, 278, | $C_{25}H_{28}O_5$ (408) |

Beispiel 148

11-Ethoxy-4-methoxy-9-methyl-3-(3-methyl-1-methylenbutyl)-7H-dibenzo[b,g][1,5]dioxocin-5-on

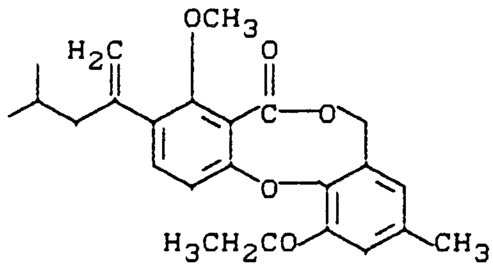

Zu einer Suspension von 150 mg (0,36 mmol) Methyltriphenylphosphoniumbromid und Natriumamid (Instant Ylid) in 5 ml absolutem Tetrahydrofuran werden unter Argon 100 mg (0,25 mmol) der Verbindung aus Beispiel 66 gelöst in 4 ml absolutem Tetrahydrofuran zugetropft. Nach einer Reaktionszeit von 14 h bei Raumtemperatur wird etwas Aceton zugegeben, von den festen Bestandteilen abgetrennt und im Vakuum eingeengt. Der Rückstand wird säulenchromatographisch an Kieselgel mit Petrolether/Essigester 6:1 gereinigt.

Ausbeute: 28 mg (28% der Theorie)
MS (EI): 396, 232, 190
SF: $C_{24}H_{28}O_5$ (396)

Beispiel 149

11-Ethoxy-3-(1-hydroxymethyl-3-methylbutyl)-4-methoxy-9-methyl-7H-dibenzo[b,g][1,5]dioxocin-5-on

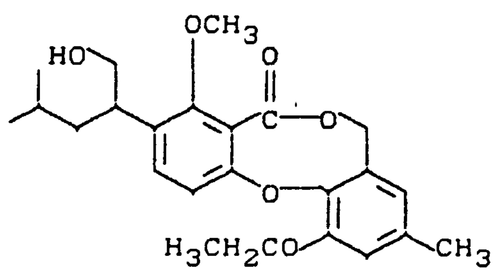

Zu einer Lösung von 130 mg (0,33 mmol) der Verbindung aus Beispiel 148 in 4 ml absolutem Tetrahydrofuran werden unter Argon 2 ml (1 mmol) einer 0,5 M 9-Borabicyclo-3.3.1-nonan-Lösung in Hexan zugetropft. Nach 14 h bei Raumtemperatur werden einige Tropfen Ethanol, 2 ml 2 N Natronlauge und 0,5 ml 30%ige Wasserstoffperoxid-Lösung zugegeben. Es wird 3 h kräftig gerührt, anschließend schonend eingeengt und der Rückstand in Dichlormethan aufgenommen. Es wird mit Wasser gewaschen, die organische Phase über Magnesiumsulfat getrocknet, eingeengt und säulenchromatographisch an Kieselgel mit Petrolether/Essigester 6:1 gereinigt.

Ausbeute: 29 mg (20% der Theorie)
MS (EI): 414, 327, 163
SF: $C_{24}H_{30}O_6$ (414)

Beispiel 150

11-Hydroxy-3-(1-hydroxyl-3-methylbutyl)-4-methoxy-9-methyl-10-prop-2-enyl-7H-dibenzo[b,g][1,5]dioxocin-5-on

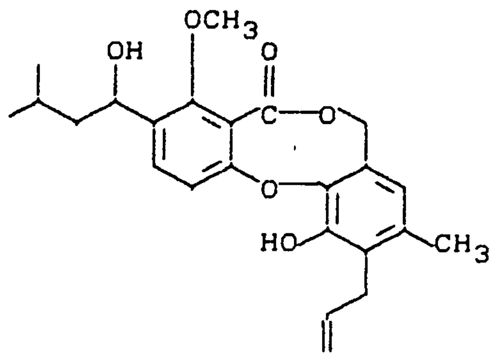

100 mg (0,25 mmol) der Verbindung aus Beispiel 20 gelöst in 2 ml Dimethylformamid werden unter Argon 14 h auf 160°C erhitzt. Anschließend wird die Lösung eingeengt, in Dichlormethan aufgenommen und mit Wasser gewaschen. Nach dem Einengen der Dichlormethanphase wird der Rückstand säulenchromatographisch an Kieselgel mit Petrolether/Essigester 6:1 gereinigt.
Ausbeute: 14 mg (14% der Theorie)
MS (EI): 412, 355, 325, 309
SF: $C_{24}H_{28}O_6$ (412)

Beispiel 151

11-Hydroxy-3-(1-hydroxyl-3-methylbutyl)-4-methoxy-9-methyl-10-(1-phenyl-prop-1-enyl)-7H-dibenzo[b,g]-[1,5]dioxocin-5-on

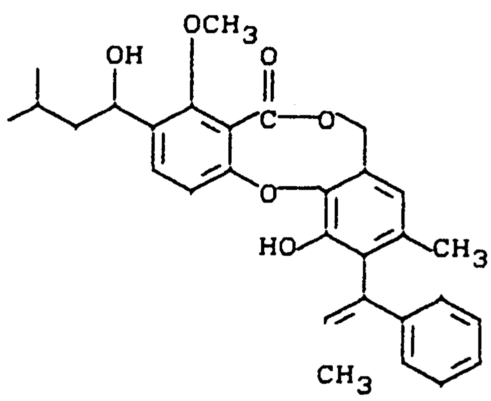

Die Titelverbindung wurde analog der Vorschrift für Beispiel 150 aus Beispiel 25 hergestellt.
MS (EI): 488, 431, 385, 150
SF: $C_{30}H_{32}O_6$ (448)

Beispiel 152

4-p-Chlorphenyl-3-(3-methyl-1-butanonyl)-11-methoxy-9-methyl-7H-dibenzo[b,g][1,5]dioxocin-5-on

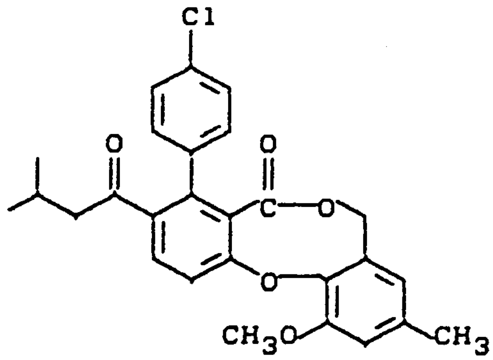

Die Titelverbindung wurde analog der Vorschrift von Beispiel 140 aus 4,11-Dimethoxy-9-methyl-3-(3-methylbutan-1-onyl)-7H-dibenzo[b,g][1,5]dioxocin-5-on hergestellt.
MS (EI): 464, 407
SF: $C_{27}H_{25}ClO_5$ (464)

Beispiel 153

4-p-Chlorphenyl-3-(1-hydroxy-3-methylbutyl)-11-methoxy-9-methyl-7H-dibenzo[b,g][1,5]dioxocin-5-on

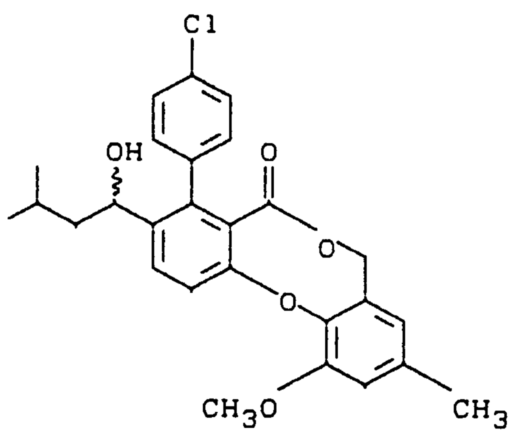

Die Titelverbindung wurde analog der Vorschrift von Beispiel 121 aus Beispiel 152 hergestellt.
MS (EI): 466, 409, 279, 363, 322
SF: $C_{27}H_{27}ClO_5$ (466)
Die in Tabelle 18 aufgeführten Verbindungen wurden in Analogie zur Vorschrift des Beispiels 1 hergestellt:

Tabelle 18:

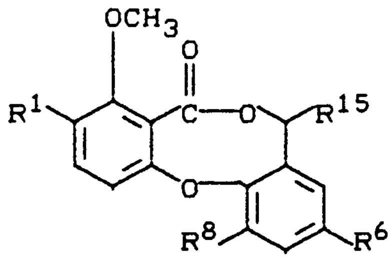

| Bsp. Nr. | $R^1$ | $R^{15}$ | $R^6$ | $R^8$ | MS (EI) | SF |
|---|---|---|---|---|---|---|
| 154 | 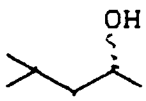 | H | H | $OCH_3$ | 372, 315 269, 243, 228 | $C_{21}H_{24}O_6$ |
| 155 | 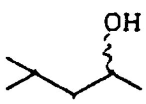 | H | $CH_3$ | H | 356, 299 269, 253 227 | $C_{21}H_{24}O_5$ (356) |
| 156 | 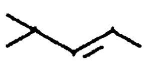 | H | Br | $OCH_3$ | 434, 432 389, 218 203 | $C_{21}H_{21}BrO_5$ (433) |
| 157 | 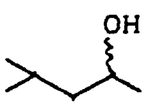 | $CH_3$ | $CH_3$ | $OCH_3$ | 400, 343 325, 297 149 | $C_{23}H_{28}O_6$ (418) |

Beispiel 158

4,11-Dimethoxy-8,9-dimethyl-3-(1-hydroxy-3-methylbutyl)-7H-dibenzo[b,g][1,5]dioxocin-5-on

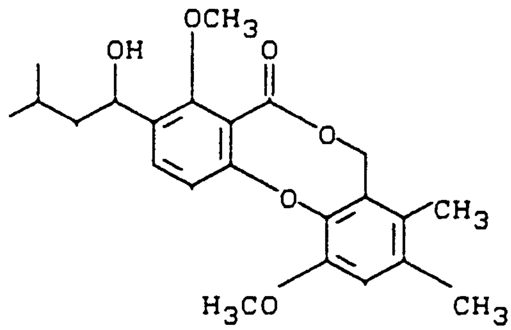

Unter Inertgas gibt man zu 3 mmol Methylmagnesiumbromid (3M in Diethylether) 3,3 mmol Zinkchlorid (1M in Diethylether) rührt 10 min bei 25 ° C, addiert eine Lösung von 0,15 mmol der Verbindung des Beispiels 39 in 3 ml wasserfreiem Tetrahydrofuran/6 ml Dimethylsulfoxid, gibt 5 mg Bis-(triphenylphosphin)-palladium(II)chlorid hinzu und rührt über Nacht bei 50 ° C. Es wird mit 0,05 ml 5N Salzsäure hydrolysiert, in Wasser/Ether aufgenommen, 3 x mit Ether gewaschen, die organische Phase mit Wasser gewaschen, getrocknet ($Na_2SO_4$) und an einer Kieselgelsäule (Methylenchlorid/Methanol 100:1) und anschließend an einer RP-8-Säule (Acetonitril/Wasser 5:6) chromatographiert. Ausbeute: 10,1 mg Öl (16,3 % der Theorie)
MS (EI): 400, 343, 297, 256
SF : $C_{23}H_{28}O_6$ (400)

Beispiel 159

4,11-Dimethoxy-3-(1-hydroxy-3-methylbutyl)-8,9,10-trimethyl-7H-dibenzo[b,g][1,5]dioxocin-5-on

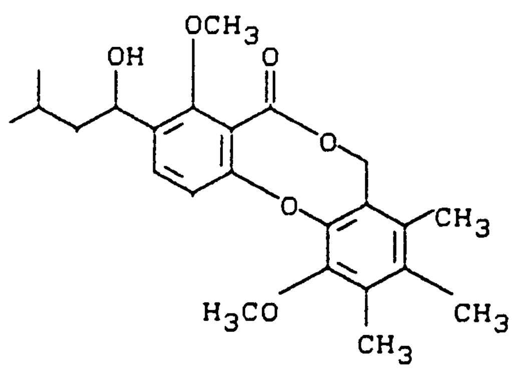

Analog zu Beispiel 158 erhält man ausgehend von der Verbindung des Beispiels 41 die Titelverbindung.
Ausbeute: 65 % der Theorie
MS (EI) : 414, 357, 339, 311, 270, 179
SF : $C_{24}H_{30}O_6$ (414)

Beispiel 160

10-Bromo-11-methoxy-3-(1-hydroxy-3-methylbutyl)-4-ethenyl-9-methyl-7H-dibenzo[b,g][1,5]dioxocin-5-on

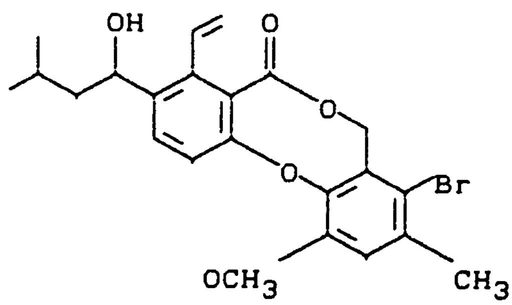

Die Titelverbindung wurde in Analogie zu den Vorschriften der Beispiele 65, 78, 121 aus Beispiel 39 dargestellt.
MS (EI): 462, 460, 405, 381, 359
SF : $C_{23}H_{25}BrO_6$ (461)

Beispiel 161

8-Bromo-4,11-dimethoxy-3-(1-hydroxy-1-ethenyl-3-methylbutyl)-9-methyl-7H-dibenzo[b,g][1,5]dioxocin-5-on

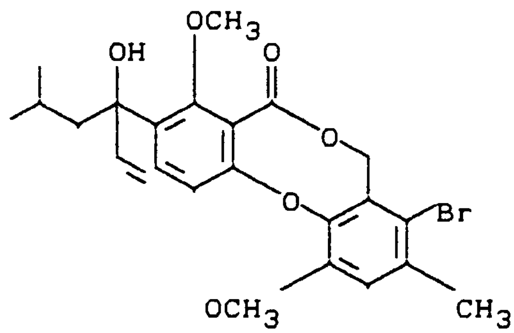

Die Titelverbindung wurde aus Beispiel 39 analog der Vorschrift des Beispiels 65 und nachfolgend des Beispiels 78 dargestellt.
MS (EI): 492, 490, 435, 433, 363, 361
SF : $C_{24}H_{27}BrO_6$ (491)

Beispiel 162

8-Ethenyl-11-hydroxy-3-(1-hydroxy-3-methylbutyl)-4-methoxy-9-methyl-7H-dibenzo[b,g][1,5]dioxocin-5-on

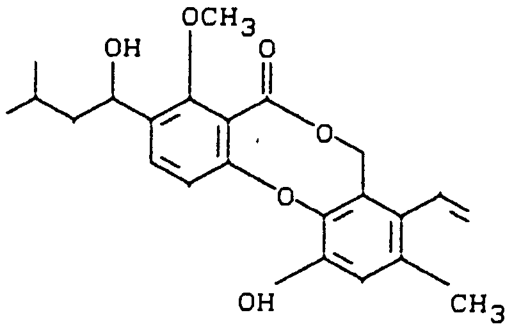

170 mg (0,38 mmol) der Verbindung aus Beispiel 108 in 7 ml Toluol werden unter Inertgas mit 18 mg (0,015 mmol) Tetrakis-(triphenylphosphin)-palladium und 0,2 ml (0,75 mmol) Tributylvinylzinn 22 h unter Rückfluß gekocht. Die Suspension wird an Kieselgel adsorbiert und mit Petrolether:Ether = 2:1 fraktioniert eluiert. Es fallen 116 mg (77 % d.Th.) farbloser Feststoff an.
MS (EI): 398, 341, 323
SF : $C_{23}H_{26}O_6$ (398)
Analog der Vorschrift Beispiel 162 wurden die in Tabelle 19 aufgeführten Verbindungen dargestellt:

Tabelle 19

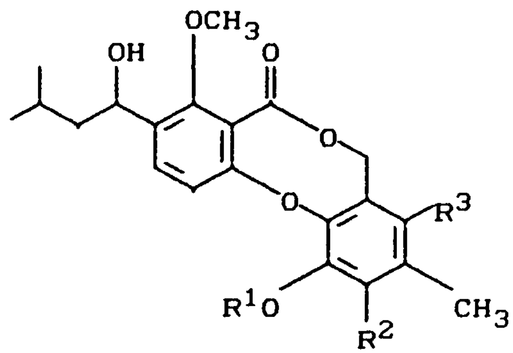

| Bsp. Nr. | $R^1$ | $R^2$ | $R^3$ | MS(EI) | SF |
|---|---|---|---|---|---|
| 163 | $CH_3$ | H | $-CH=CH_2$ | 412,383,355,337 | $C_{24}H_{28}O_6$ (412) |
| 164 | $CH_3$ | H | 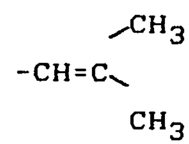 | 440,411,383,365 | $C_{26}H_{32}O_6$ (440) |

<u>Tabelle 19</u> (Fortsetzung)

| Bsp. Nr. | $R^1$ | $R^2$ | $R^3$ | MS(EI) | SF |
|---|---|---|---|---|---|
| 165 | $CH_3$ | H | 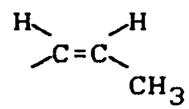 | 426,397,369,351 | $C_{25}H_{30}O_6$ (426) |
| 166 | $CH_3$ | H | 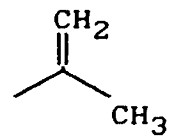 | 426,369,351,313 | $C_{25}H_{30}O_6$ (426) |
| 167 | $CH_3$ | $-CH=CH_2$ | $-CH_2=CH_2$ | 438,381,363,332 | $C_{26}H_{30}O_6$ (438) |
| 168 | $CH_3$ | H | 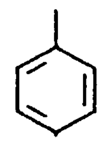 | 462,405,388,359,210 | $C_{28}H_{30}O_6$ (462) |
| 169 | $CH_3$ | H | 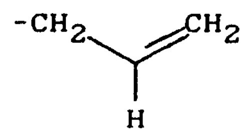 | 426,369,351,323 277 | $C_{25}H_{30}O_6$ (426) |
| 170 | H | $-CH=CH_2$ | $-CH=CH_2$ | 424,395,367,349 | $C_{25}H_{28}O_6$ (424) |

Die in Tabelle 20 aufgeführten Beispiele wurden in Analogie zur Vorschrift des Beispiels 70 hergestellt:

Tabelle 20

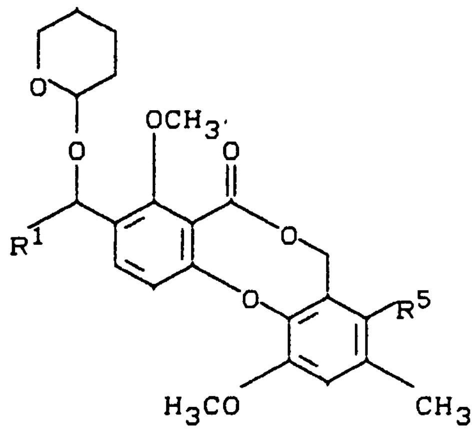

| Bsp. Nr. | $R^1$ | $R^5$ | MS (EI) | SF |
|---|---|---|---|---|
| 171 | 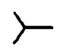 | 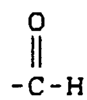 | 414,396, 367, 357, 311 | $C_{28}H_{34}O_8$ (484) |
| 172 | 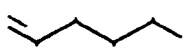 | H | 496, 412, 329, 299, 283 | $C_{29}H_{36}O_7$ (496) |

Beispiel 173

4,11-Dimethoxy-3-(1-hydroxy-3-methylbutyl)-8-oxomethylen-9-methyl-7H-dibenzo[b,g][1,5]dioxocin-5-on

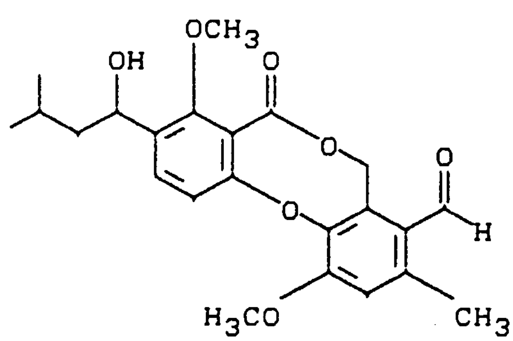

Die Titelverbindung wurde analog der Vorschrift des Beispiels 101 aus Beispiel 162 dargestellt.
MS (EI): 414, 385, 357, 327, 311
SF : $C_{23}H_{26}O_7$ (414)

Beispiel 174

8-Azidomethylen-4-11,dimethoxy-3-(1-hydroxy-3-methylbutyl)-9-methyl-7H-dibenzo[b,g][1,5]dioxocin-5-on

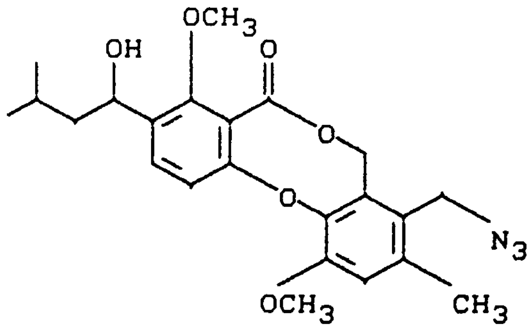

Die Titelverbindung wurde analog der Vorschrift des Beispiels 72 aus der Verbindung des Beispiels 173 nach Ankopplung und nachträglicher Abspaltung einer Silylschutzgruppe gemäß Standardverfahren (s.J. Amer. Chem. Soc. 94, 6190 (1972)) dargestellt.
MS (EI): 441, 384, 341, 161, 28
SF : $C_{23}H_{27}N_3O_6$ (441)

Beispiel 175

8-Aminomethylen-4,11-dimethoxy-3-(1-hydroxy-3-methylbutyl)-9-methyl-7H-dibenzo[b,g][1,5]dioxocin-5-on

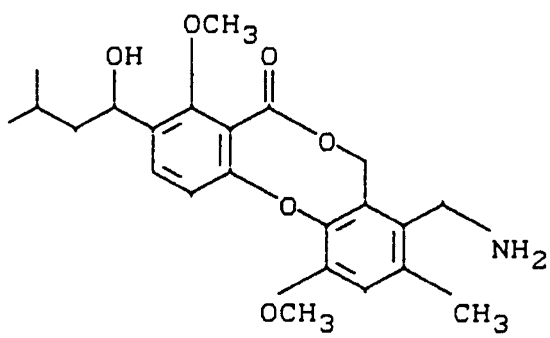

Die Verbindung wurde analog der Vorschrift des Beispiels 74 aus der Verbindung des Beispiels 174 nach Ankopplung und Abspaltung einer Silylschutzgruppe gemäß Standardverfahren (s. J. Amer. J. Chem. 94, 6190 (1972)) dargestellt.
MS (DCI): 416, 398, 384, 176, 162
SF : $C_{23}H_{29}NO_6$ (415)
Die in Tabelle 21 aufgeführten Beispiele wurden in Analogie zur Vorschrift des Beispiels 78 aus der Verbindung des Beispiels 173 hergestellt.

Tabelle 21

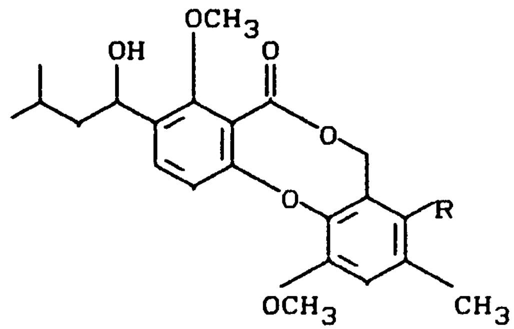

| Bsp. | R | MS (EI) | SF |
| --- | --- | --- | --- |
| 176 | 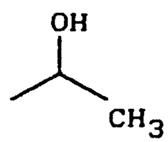 | 430,373,355, 337,325 | $C_{27}H_{30}O_7$ (430) |
| 177 | 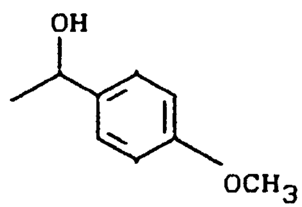 | 522,504,486, 455,269 | $C_{30}H_{34}O_8$ (522) |
| 178 | 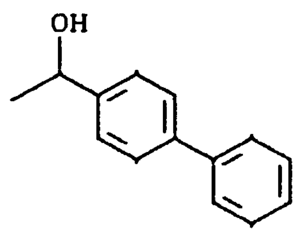 | 568,550,506, 315<br>Diastereomer I<br>Diastereomer II | $C_{35}H_{36}O_7$ (568) |

**Tabelle 21** (Fortsetzung)

| Bsp. | R | MS (EI) | SF |
|---|---|---|---|
| 179 | 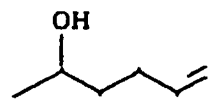 | 470,452,415<br>395,377 | $C_{27}H_{34}O_7$<br>(470) |
| 180 | 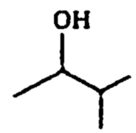 | 458<br>Diastereomer I<br>Diastereomer II | $C_{26}H_{24}O_7$<br>(458) |
| 181 | 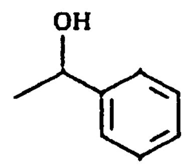 | 492,474,435<br>417,389 | $C_{29}H_{32}O_7$<br>(492) |
| 182 | 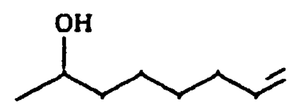 | 498,480,441,<br>415,397 | $C_{29}H_{38}O_7$<br>(498) |
| 183 | 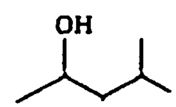 | 472,454,415,397,<br>379<br>Diastereomer I<br>472,454,415,379<br>Diastereomer II | $C_{27}H_{36}O_7$<br>(472) |

Beispiel 184

4,11-Dimethoxy-3-(1-hydroxy-3-methylbutyl)-8-hydroxymethylen-9-methyl-7H-dibenzo[b,g][1,5]dioxocin-5-on

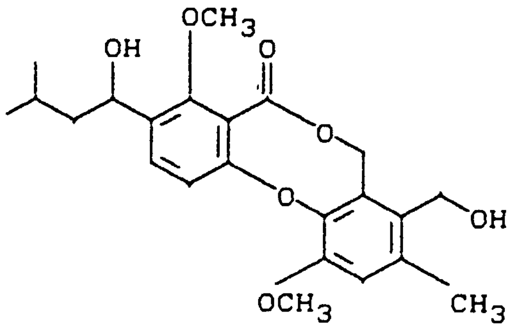

Die Titelverbindung wurde analog der Vorschrift des Beispiels 121 aus der Verbindung des Beispiels 173 dargestellt
MS (EI): 416, 359, 341, 311, 297
SF : $C_{23}H_{28}O_7$ (416)

Beispiel 185

4,11-Dimethyl-3-(1-hydroxy-3-methylbutyl)-8-(3-ethylpropenat)-9-methyl-7H-dibenzo[b,g][1,5]-dioxocin-5-on

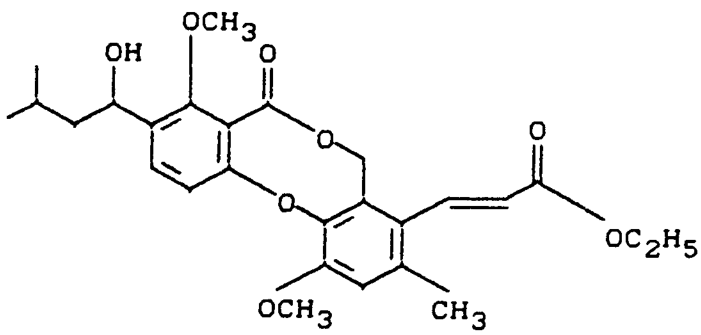

100 mg (0,24 mmol) der Verbindung aus Beispiel 173 werden mit 168 mg (0,48 mmol) Ethoxycarbonylmethylentriphenylphosphoran in 3 ml Toluol bei 80 °C 14 h unter Inertgas gerührt.

Nach Zusatz von Methylenchlorid wird mit 0,1 N aq. Salzsäure gewaschen, getrocknet und eingedampft. Nach Chromatographie an Kieselgel in Methylenchlorid 5 % Methanol fallen 103 mg (88 % der Th.) farbloser Feststoff an.
MS (DCI): 485 (M + H), 467, 427
SF : $C_{27}H_{32}O_8$ (484)

Beispiel 186

4,11-Dimethoxy-3-(3-ethylpropenat)-9-methyl-7H-dibenzo[b,g][1,5]dioxocin-5-on

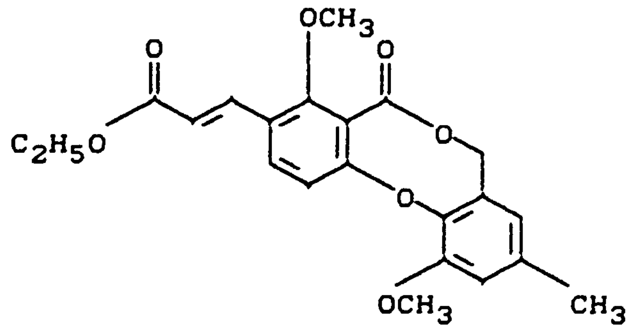

Die Titelverbindung wurde analog der Vorschrift des Beispiels 185 aus der Verbindung des Beispiels 101 dargestellt
MS (EI): 398, 353, 248,
SF : $C_{22}H_{22}O_7$ (398)

Beispiel 187

11-Hydroxy-3-(1-hydroxy-3-methylbutyl)-4-methoxy-9-methyl-8-nitro-7H-dibenzo[b,g][1,5]dioxocin-5-on

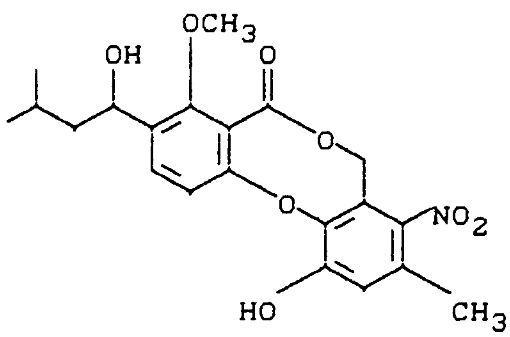

150 mg (0,4 mmol) Penicillide werden in 15 ml Methylenchlorid gelöst und unter Inertgas bei -68 °C Badtempertur nach Zugabe von 0,8 ml einer 0,5 molaren Lösung von Nitroniumtetrafluoroborat 1 h gerührt. Die Reaktionsmischung wird auf Eis gegeben. Nach Extraktion mit Methylenchlorid, waschen mit Wasser und Bicarbonat, Trocknen und Eindampfen wird an Kieselgel in Petrolether:Ether = 1:1 chromatographiert. Nach Eindampfen fallen 86 mg (51 % d.Th.) gelblicher Feststoff an.
MS (EI): 417, 399, 360, 342
SF : $C_{21}H_{23}NO_8$ (417)

Beispiel 188

10-Bromo-11-hydroxy-3-(1-hydroxy-3-methylbutyl)-4-methoxy-9-methyl-8-nitro-7H-dibenzo[b,g][1,5]dioxocin-5-on

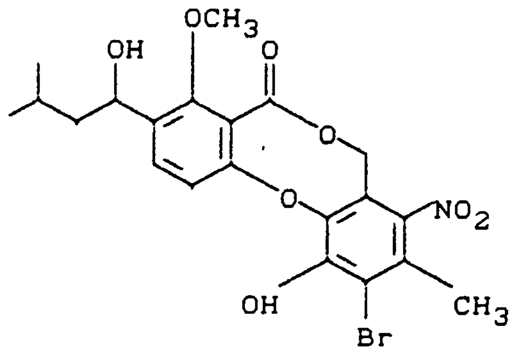

Die Titelverbindung wurde analog Verfahren des Beispiel 187 aus der Verbindung des Beispiel 107 dargestellt.
MS (EI): 497, 495, 479, 477, 440, 438
SF : $C_{21}H_{22}NO_8$ (496)

Beispiele 189/190

8-Chloro-11-hydroxy-3-(1-hydroxy-3-methylbutyl)-4-methoxy-9-methyl-7H-dibenzo[b,g][1,5]dioxocin-5-on (189)

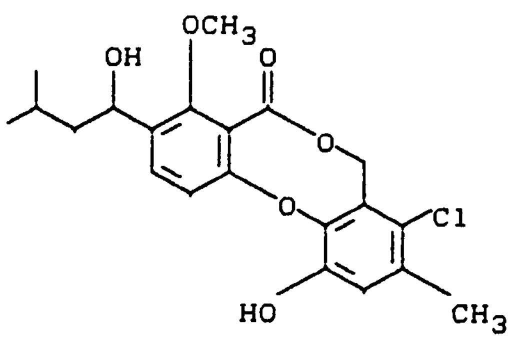

8,10-Dichloro-11-hydroxy-3-(1-hydroxy-3-methylbutyl)-4-methoxy-9-methyl-7H-dibenzo[b,g][1,5]dioxocin-5-on (190)

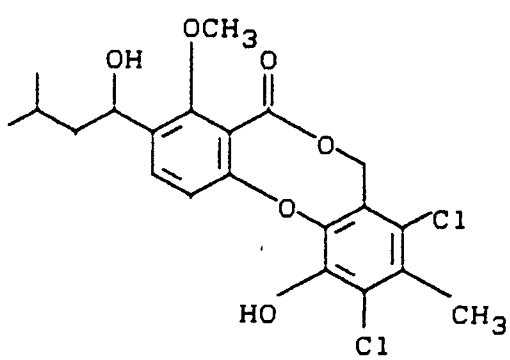

100 mg (0,27 mmol) Penicillide wurden in 2 ml Ethanol:Wasser = 1:1 nach Zugabe von 36 mg (0,27 mmol) N-Chlorosuccinimid und 70 mg (0,26 mmol) Eisen(III)-chlorid-hexahydrat 12 h bei 20°C gerührt. Nach Extraktion mit Methylenclorid, waschen mit Wasser und Trocknen wird an Kieselgel in Petrolether:Ether = 1:2 fraktioniert, chromatografiert. Es fallen 2 chromatografisch einheitliche Produkte an.

Beispiel 189: Ausbeute 39 mg (= 35 % d.Th.) farbloser Feststoff
MS (EI): 406, 349, 303
SF : $C_{21}H_{23}ClO_6$ (406)

Beispiel 190: Ausbeute 50 mg (42 % d. Th.) farbloser Feststoff
MS (EI): 442, 440, 424, 422, 385, 383
SF : $C_{21}H_{22}Cl_2O_6$ (442)

Beispiel 191

10-Jodo-11-hydroxy-3-(1-hydroxy-3-methylbutyl)-4-methoxy-9-methyl-7H-dibenzo[b,g][1,5]dioxocin-5-on

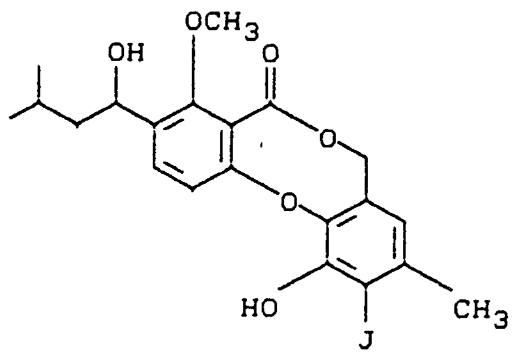

Die Titelverbindung wurde analog der Vorschrift der Beispiele 189 und 190 mit Jodoniumchlorid als Halogenierungsreagenz dargestellt.
MS (EI): 498, 441, 395
SF : $C_{21}H_{23}JO_6$ (498)

Beispiel 192

4,11-Dimethoxy-3-(1-hydroxy-3-methylbutyl)-8-iodo-9-methyl-7H-dibenzo[b,g][,5]dioxocin-5-on

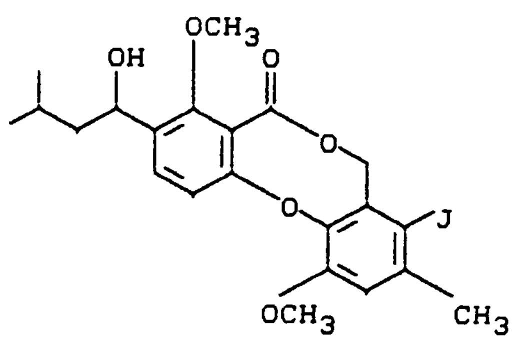

Zur Darstellung der Titelverbindung wird Penicillide analog zur Vorschrift des Beispiels 9 zum Monomethylether und nachfolgend analog der Vorschrift des Beispiels 191 umgesetzt.
MS (EI): 512, 455, 427, 409, 368
SF : $C_{22}H_{25}JO_6$ (512)
Analog der Vorschrift für das Beispiel 9 wurden die in der Tabelle 22 aufgeführten Verbindungen hergestellt

Tabelle 22

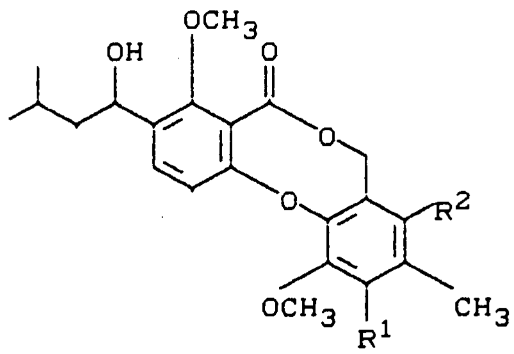

| Bsp. Nr. | $R^1$ | $R^2$ | MS(EI) | SF |
|---|---|---|---|---|
| 192 | H | Cl | 420,363,345 | $C_{22}H_{25}ClO_6$ (420) |
| 194 | Cl | Cl | 456,454,399,397 | $C_{22}H_{24}Cl_2O_6$ (456) |
| 195 | H | $NO_2$ | 431,374 | $C_{22}H_{25}NO_8$ (431) |
| 196 | J | H | 512,455,409 | $C_{22}H_{25}JO_6$ (512) |

Die in Tabelle 23 aufgeführten Beispiele wurden in Analogie zur Vorschrift des Beispiels 78 hergestellt:

Tabelle 23

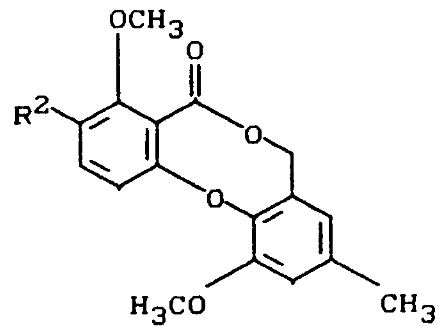

| Bsp. Nr. | $R^2$ | MS (EI) | SF |
|---|---|---|---|
| 197 | 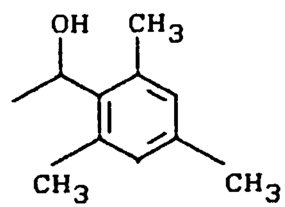 | 448,399,301 271,242 | $C_{27}H_{28}O_6$ (448) |
| 198 | 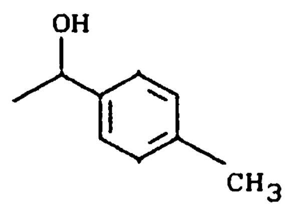 | 420,402,387, 361,328 | $C_{25}H_{24}O_6$ (420) |
| 199 | 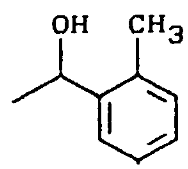 | 458,420,402, 387,371 | $C_{25}H_{24}O_6$ (420) |

Tabelle 23 (Fortsetzung)

| Bsp. Nr. | $R^2$ | MS (EI) | SF |
|---|---|---|---|
| 200 | 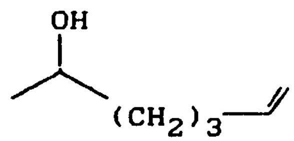 | 398,329,311, 299,283 | $C_{23}H_{26}O_6$ (398) |
| 201 | 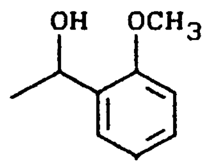 | 436,418,403, 285,135 | $C_{25}H_{24}O_7$ (436) |
| 202 | 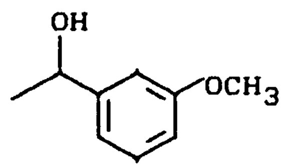 | 436,418,403, 285,135 | $C_{25}H_{24}O_7$ (436) |
| 203 | 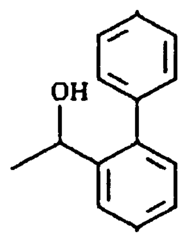 | 482, 464, 449, 433, 419 | $C_{30}H_{26}O_6$ (482) |

Tabelle 23 (Fortsetzung)

| Bsp. Nr. | $R^2$ | MS (EI) | SF |
|---|---|---|---|
| 204 | 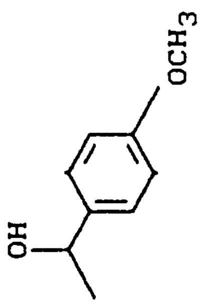 | 436,418,403, 327,297 | $C_{25}H_{34}O_7$ (436) |
| 205 | 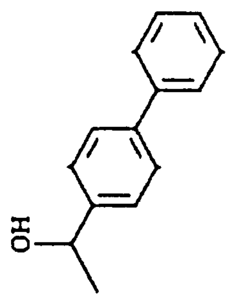 | 482,464,449, 359,331 | $C_{30}H_{26}O_6$ (482) |
| 206 | 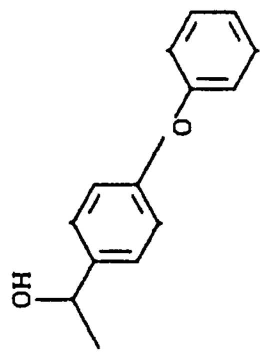 | 498,480,465, 347,283 | $C_{30}H_{28}O_7$ (500) |
| 207 | 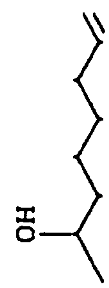 | 412,339,31, 299,283 | $C_{24}H_{28}O_6$ (412) |

## Beispiel 208

4,11-Dimethoxy-1-hydroxy-9-methyl-8(1-tetrahydropyranyl-2-oxy)oxymethylen)-7H-dibenzo[b,g][1,5]dioxocin-5-on

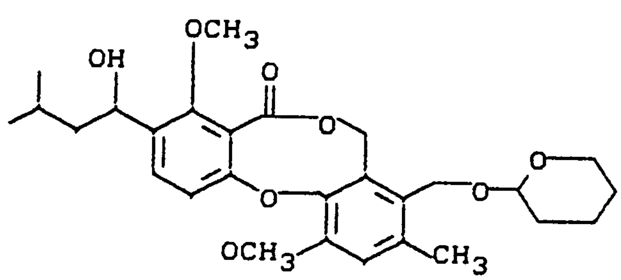

Die Titelverbindung wurde aus der Verbindung des Beispiels 184 durch Einfürhung und Entfernung der Sily-Schutzgruppe nach Standardmethoden (vgl. J. Am. Chem. Soc. 94, 6190 (1972) in Analogie zum Beispiel 70 hergestellt.

MS (EI): 500, 399, 341,
SF: $C_{28}H_{36}O_8$ (500)

Beispiel 209

3-(1-amino-3-methylbutyl)-8-bromo-4,11-dimethoxy-9-methyl-7H-dibenzo[b,g][1,5]dioxocin-5-on

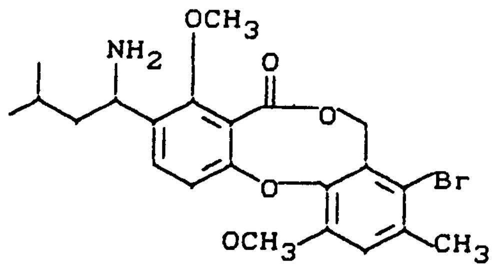

Die Titelverbindung wurde in Analogie zu den Vorschriften der Beispiele 72 und 74 hergestellt.
MS (DCI): 464, 462 (M + H), 449, 447, 408, 406
SF: $C_{22}H_{26}BrNO_5$ (464)

Beispiel 210

4,11-Dimethoxy-1-hydroxy-9-methyl-8-(1-pyrrolomethylen)-7H-debenzo[b,g][1,5]dioxocin-5-one

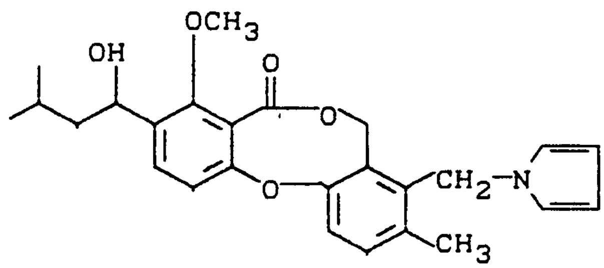

Die Titelverbindung wurde aus der Verbindung des Beispiels 174 durch Behandeln mit äquimolaren Mengen von 2,5-Diethoxy-tetrahydrofuran und Natriumacetat in Essigsäure bei 100 °C (15 Min), anschließender Extraktion und Chromatographie hergestellt.
MS (FAB): 472 ($M^+$ + Li)
SF: $C_{27}H_{31}NO_6$ (465)

Beispiel 211

3-(Methylsulfonylamido-3-methylbutyl)-8-bromo-4,11-dimethoxy-9-methyl-7H-dibenzo[b,g][1,5]dioxocin-5-one

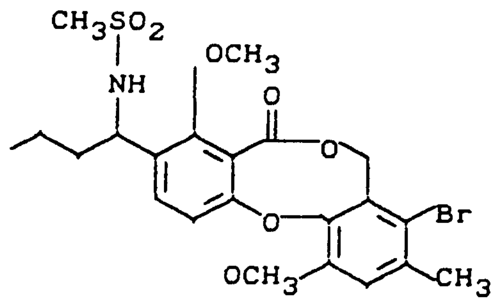

Die Titelverbindung wurde in Analogie zur Vorschrift des Beispiels 77 aus der Verbindung des Beispiels 209 hergestellt.
MS (DCl): 561, 559 ($M^{+} + NH_4^{+}$), 484, 449, 447
SF: $CH_{23}H_{28}BrNSO_7$ (542)

Die in der Tabelle 24 aufgeführten Beispiele werden in Analogie zur Vorschrift des Beispiels 101 hergestellt:

Tabelle 24

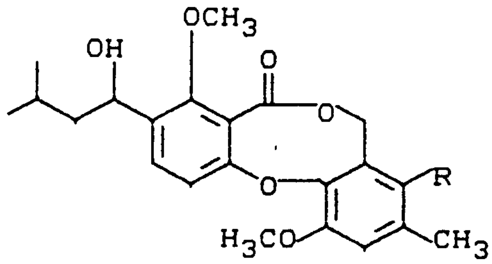

| Beispiel Nr. | R | MS(EI) | SF |
|---|---|---|---|
| 212 | $-CO-OC_2H_5$ | 458,401,355 | $C_{25}H_{30}O_8$(458) |
| 213 | 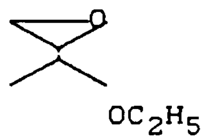 | 472,458,443 429,415,401 | $C_{26}H_{32}O_8$(472) |
| 214 | 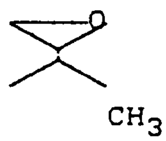 | 42,385,467 | $C_{25}H_{30}O_7$(442) |
| 215 | 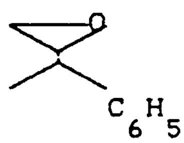 | 504,429,251 | $C_{30}H_{32}O_7$(507) |

Beispiel 216

11-Hydroxy-3(1-hydroxy-3-methylbutyl)-4-methoxy-9-methyl-8(3-pyridyl)-7H-dibenzo[b,g][1,5]dioxocin-5-on-methojodid

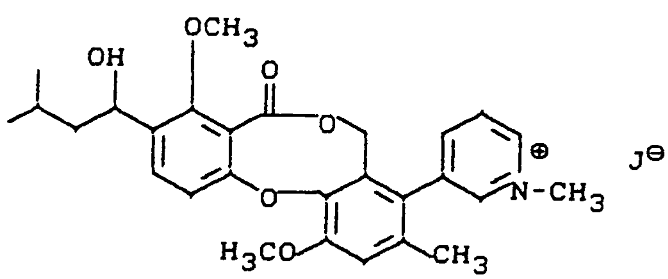

32,6 mg (0.07 mmol) der Verbindung aus Beispiel 224 wurden mit 3 ml Jodmethan solange gerührt, bis die Ausgangsverbindung die dünnschichtchromatischer Kontrolle nicht mehr nachweisbar war.

Nach Entfernung des überschüssigen Jodmethans erhielt man 41 mg (96,5 %) der Titelverbindung.

SF: $C_{28}H_{32}JNO_6$(605) MS(FAB) 478, 307, 242, 154

Die in Tabelle 25 aufgeführten Verbindungen wurden in Analogie zur Vorschrift des Beispiels 162

hergestellt.

**Tabelle 25:**

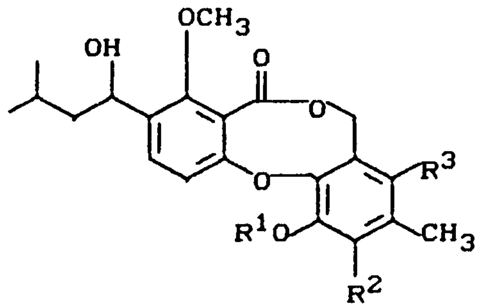

| Bsp.-Nr. | $R^1$ | $R^2$ | $R^3$ | MS(EI) | SF |
|---|---|---|---|---|---|
| 217 | $CH_3$ | H | -C≡C- 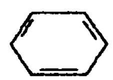 | 486,383,342, | $C_{30}H_{30}O_6$ (486) |
| 218 | $CH_3$ | H | 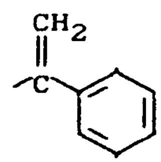 | 488,431,413 | $C_{30}H_{32}O_6$ (488) |
| 219 | $CH_3$ | H | 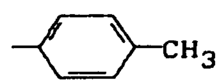 | 476,419,402 | $C_{29}H_{32}O_6$ (476) |
| 220 | $CH_3$ | H | 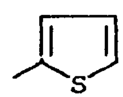 | 468,411,394 | $C_{26}H_{28}O_6S$ |

| Bsp.-Nr. | $R^1$ | $R^2$ | $R^3$ | MS(EI) | SF |
|---|---|---|---|---|---|
| 221 | $CH_3$ | H | 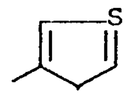 | 468,411,394, | $C_{26}H_{28}O_6S$ (468) |
| 222 | $CH_3$ | H | 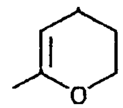 | 468,439,437,411 | $C_{27}H_{32}O_7$ (468) |
| 223 | $CH_3$ | H | 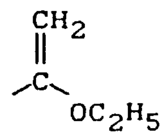 | 456,427,425,399 | $C_{26}H_{32}O_7$ (456) |
| 224 | $CH_3$ | H | 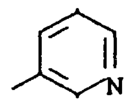 | 463,406,388 | $C_{27}H_{29}NO_6$ (463) |
| 225 | $CH_3$ | H | $-C\equiv C-CH_2-$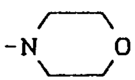 | 509,478,464 | $C_{29}H_{35}NO_7$ (509) |
| 226 | $CH_3$ | H | $-C\equiv C-CH_2-$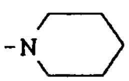 | 507,277,189 | $C_{30}H_{37}NO_6$ (507) |
| 227 | $CH_3$ | H | 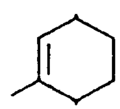 | 466,409,391 | $C_{28}H_{34}O_6$ (466) |

| Ex. No. | $R^1$ | $R^2$ | $R^3$ | MS(EI) | SF |
|---|---|---|---|---|---|
| 228 | $CH_3$ | H | 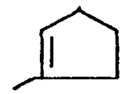 | 452,423,395,377 | $C_{27}H_{32}O_6$ (452) |
| 229 | $CH_3$ | H | 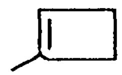 | 438,409,381,363,353 | $C_{26}H_{30}O_6$ (438) |
| 230 | $CH_3$ | H | 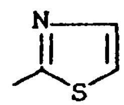 | 469,412,368 | $C_{25}H_{27}NO_6S$ (469) |
| 231 | $CH_3$ | H | 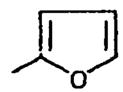 | 452,395,377 | $C_{26}H_{28}O_7$ (452) |
| 232 | $CH_3$ | H | 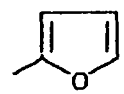 | 454,425,397 | $C_{26}H_{30}O_7$ (454) |
| 233 | $CH_3$ | H | 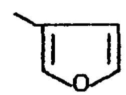 | 452,395,377 | $C_{26}H_{28}O_7$ (452) |
| 234 | $CH_3$ | H | $-S-CH_3$ | 432,375,357 | $C_{23}H_{28}O_6S$ (432) |

Die in Tabelle 26 aufgeführten Beispiele wurden durch Alkylierung des Beispiels 184 nach bekannten Methoden unter Einführung und Entfernung des Silylschutzgruppen hergestellt (vgl. A. F. Kluge et al, J. Am. Chem, Soc. 94, 7827 (1972); E. J. Casey et al., J. Am. Chem. Soc. 94, 6190 (1972).

Tabelle 26

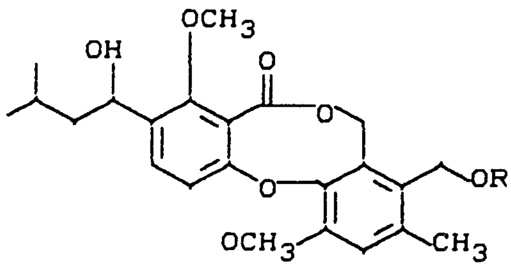

| Bsp. Nr. | R | SF | MS(EI) |
|---|---|---|---|
| 235 | $-(CH_2)_2O(CH_2)_2OCH_3$ | $C_{28}H_{38}O_9$ (518) | 518,500,398, 369,341,311 |
| 236 | $-CH_3$ | $C_{24}H_{30}O_8$ (430) | 430,373,341,311 |
| 237 | 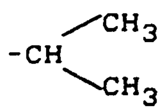 | $C_{26}H_{34}O_7$ (458) | 458,341,311 |
| 238 | 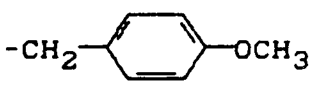 | $C_{31}H_{26}O_8$ (536) | 536,415,163 |
| 239 | 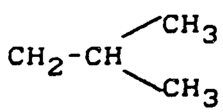 | $C_{27}H_{36}O_7$ (472) | 472,415,341,311 |

Die in der Tabelle 27 aufgeführten Beispiele wurden in Analogie zur Vorschrift des Beispiela 162 hergestellt.

Tabelle 27

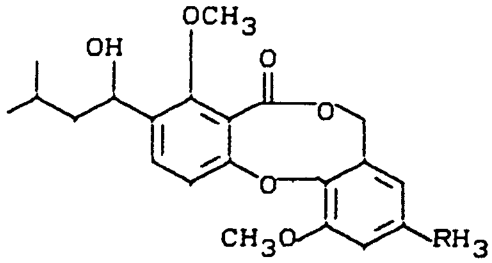

| Bsp. Nr. | R | MS(EI) | SF |
|---|---|---|---|
| 240 | 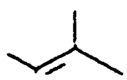 | 426,369,323, 282 | $C_{25}H_{30}O_6$ (426) |
| 241 | 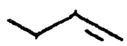 | 412,355,309, 268 | $C_{24}H_{28}O_6$ (412) |
| 242 | 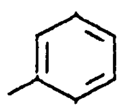 | 448,391,345, 329,320 | $C_{27}H_{28}O_6$ (448) |
| 243 | 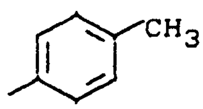 | 462,405,359, 318 | $C_{28}H_{30}O_6$ (462) |
| 244 | 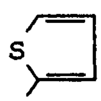 | 454,351,310 | $C_{25}H_{26}O_6S$ (454) |
| 245 | 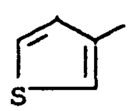 | 454,351,315, 269 | $C_{25}H_{26}O_6S$ (454) |
| 246 | 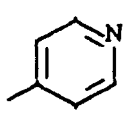 | | $C_{26}H_{27}NO_6$ (449) |
| 247 | 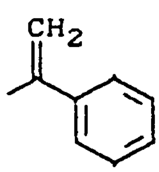 | 474,377,365 | $C_{29}H_{30}O_6$ (474) |

| Bsp. Nr. | R | MS(EI) | SF |
|---|---|---|---|
| 248 | 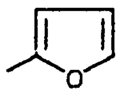 | 438,355,315, 269 | $C_{25}H_{26}O_7$ (438) |
| 249 | 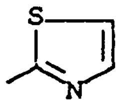 | 455,315,269, | $C_{24}H_{25}NO_6S$ (455) |
| 250 | S-$CH_3$ | | $C_{22}H_{26}O_6S$ |
| 251 | 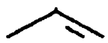 | 398,341,295, 254 | $C_{23}H_{26}O_6$ (398) |

Die in der Tabelle 28 aufgeführten Beispiele wurden in Analogie zur Vorschrift des Beispiels 78 hergestellt.

Tabelle 28

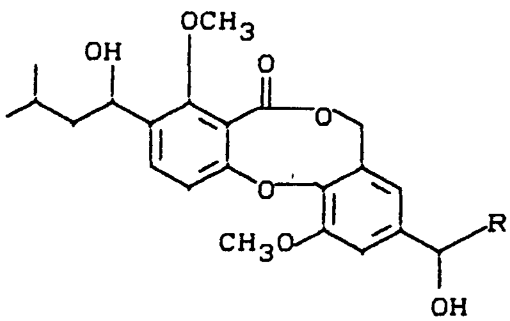

| Bsp. Nr. | R | MS(EI) | SF |
|---|---|---|---|
| 252 | -$CH_3$ | 416,359,313, 272 | $C_{23}H_{28}O_7$ (416) |
| 253 | 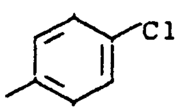 | 512,455,409 | $C_{28}H_{29}ClO_7$ (512,5) |
| 254 | 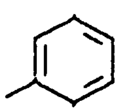 | 478,421,375 | $C_{28}H_{30}O_7$ |

Beispiel 255

9-Formyl-3-(1-hydroxy-3-methylbutyl)-4,11-dimethoxy-7H-dibenzo[b,g][1,5]dioxocin-5-on

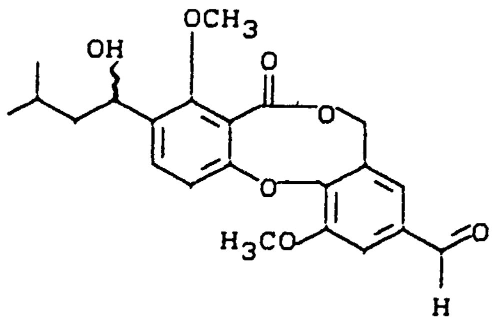

Die Titelverbindung wurde in Analogie zur Vorschrift des Beispiels 101 aus der Verbindung des Beispiels 251 hergestellt:
MS (EI): 400, 382, 343, 297
SF: $C_{22}H_{24}O_7$ (400)

Beispiel 256

9-(2-Carboxyethylester-ethenyl)-3-(1-hydroxy-3-methylbutyl)-4,11-dimethoxy-7H-dibenzo[b,g][1,5]dioxocin-5-on

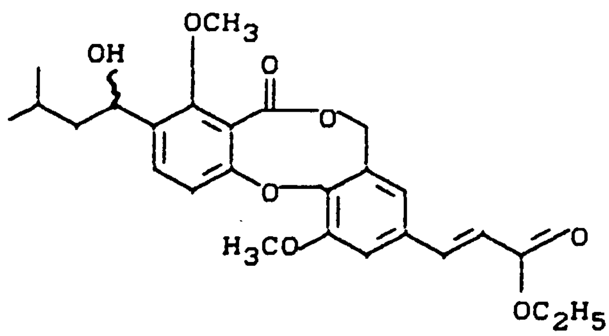

Dit Titelverbindung wurde in Analogie zur Vorschrift des Beispiels 185 aus der Verbindung des Beispiels 255 hergestellt.
MS (EI): 470, 452, 413, 383, 367
SF: $C_{26}H_{30}O_8$ (470)

Beispiel 257:

9-Hydroxymethyl-3-(1-hydroxy-3-methylbutyl)-4,11-dimethoxy-7H-dibenzo[b,g][1,5]dioxocin-5-on

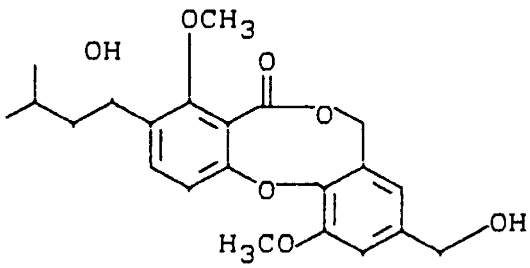

Die Titelverbindung wurde in Analogie zur Vor-schrift des Beispiels 121 aus der Verbindung des Beispiels 251 hergestellt.
MS(EI): 402, 345, 299, 258
SF: $C_{22}H_{26}O_7$ (402)

Beispiel 258

9-Bromo-3-(1-hydroxy-3-methylbutyl)-4,11-dimethoxy-7H-dibenzo[b,g][1,5]dioxocin-5-on

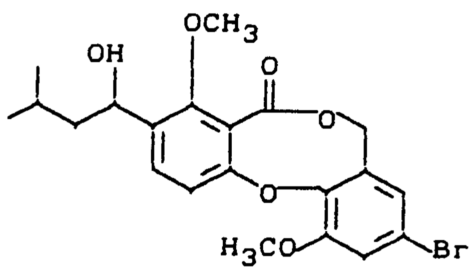

Die Titelverbindung wurde in Analogie zur Vorschrift des Beispiels 1 hergestellt.
MS(EI): 452 ($M^+$), 450, 395, 393, 349, 347
SF: $C_{21}H_{23}BrO_6$ (451)

**Patentansprüche**
**Patentansprüche für folgende Vertragsstaaten : AT, BE, CH, DE, DK, ES, FR, GB, IT, LI, LU, NL, SE**

**1.** Verbindungen der allgemeinen Formel (I)

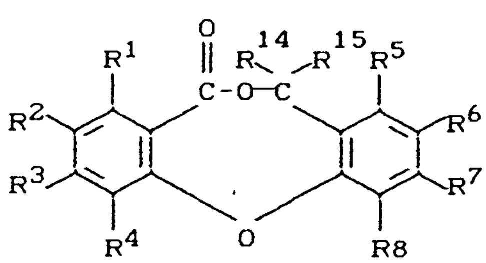

(I)

in welcher
$R^1$, $R^2$, $R^3$, $R^4$, $R^5$, $R^6$, $R^7$ und $R^8$ gleich oder verschieden sind und jeweils
- für Wasserstoff oder

- für geradkettiges oder verzweigtes Alkylthio, Alkyl, Alkenyl oder Alkinyl mit jeweils bis zu 12 Kohlenstoffatomen stehen, die gegebenenfalls durch Halogen, Azido, Imino, hydroxysubstituiertes Imino, Hydroxy, Cyano, Cycloalkyl mit 3 bis 8 Kohlenstoffatomen, oder durch einen 3- bis 7-gliedrigen, gesättigten oder ungesättigten Heterocyclus mit bis zu 4 Heteroatomen aus der Reihe Stickstoff, Schwefel oder Sauerstoff, oder durch Aryl oder Aryloxy mit 6 bis 10 Kohlenstoffatomen substituiert sind, die ihrerseits bis zu 3-fach gleich oder verschieden durch Halogen, Nitro, Phenyl, Phenoxy, Cyano, geradkettiges oder verzweigtes Alkyl, Alkoxy oder Alkoxycarbonyl mit jeweils bis zu 8 Kohlenstoffatomen substituiert sein können, oder durch einen Rest der Formel

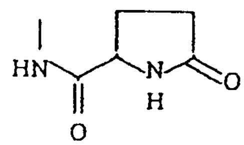

oder durch eine Gruppe der Formel $-NR^9R^{10}$, $-COR^{11}$ oder $-OR^{12}$ substituiert sind,

worin

$R^9$ und $R^{10}$ gleich oder verschieden sind und jeweils Wasserstoff, geradkettiges oder verzweigtes Alkyl oder Alkoxy mit bis zu 10 Kohlenstoffatomen, Aryl mit 6 bis 10 Kohlenstoffatomen oder eine Gruppe der Formel $-S(O)_pR^{13}$ bedeuten,

$R^{11}$ Wasserstoff, Hydroxy, geradkettiges oder verzweigtes Alkyl mit bis zu 10 Kohlenstoffatomen, Phenoxy, Aryl mit 6 bis 10 Kohlenstoffatomen oder die Gruppe $-NR^9R^{10}$ bedeutet,

worin

$R^9$ und $R^{10}$ die oben angegebene Bedeutung haben,

$R^{12}$ Wasserstoff, Cycloalkyl mit 3 bis 8 Kohlenstoffatomen, Formyl, Acyl mit bis zu 8 Kohlenstoffatomen, Tetrahyropyranyl, Trifluormethoxy oder eine Gruppe der Formel $-S(O)_pR^{13}$ bedeutet,

worin

p eine Zahl 1 oder 2 bedeutet

$R^{13}$ - geradkettiges oder verzweigtes Alkyl mit bis zu 8 Kohlenstoffatomen oder die Gruppe $-NR^9R^{10}$ bedeutet,

worin

$R^9$ und $R^{10}$ die oben angegebene Bedeutung haben,

oder

$R^{12}$ geradkettiges oder verzweigtes Alkyl oder Alkenyl mit bis zu 10 Kohlenstoffatomen bedeutet, das gegebenenfalls durch Hydroxy, Halogen, Cycloalkyl mit 3 bis 8 Kohlenstoffatomen, Alkoxy oder Alkoxycarbonyl mit bis zu 8 Kohlenstoffatomen, oder durch Aryl mit 6 bis 10 Kohlenstoffatomen substituiert ist, das seinerseits durch Halogen, Hydroxy, Alkyl, Alkoxy oder Alkoxycarbonyl mit jeweils bis zu 8 Kohlenstoffatomen, Nitro oder Cyano substituiert sein kann,

oder durch einen 3- bis 7-gliedrigen, gesättigten oder ungesättigten Heterocyclus mit bis zu 4 Heteroatomen aus der Reihe Schwefel, Sauerstoff oder Stickstoff oder durch eine Gruppe der Formel $-NR^9R^{10}$, $-COR^{11}$ oder $-OR^{12}$ substituiert ist,

worin

$R^9$, $R^{10}$, $R^{11}$ und $R^{12}$ die oben angegebene Bedeutung haben,

oder durch einen Rest der Formel

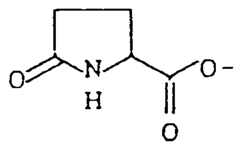, 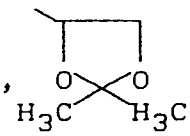 oder 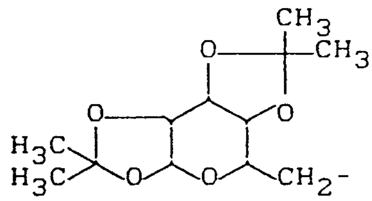

substituiert ist,

oder Aryl mit 6 bis 10 Kohlenstoffatomen bedeutet, das gegebenenfalls durch Halogen, Hydroxy, Nitro oder Cyano substituiert ist,

oder

$R^1$, $R^2$, $R^3$, $R^4$, $R^5$, $R^6$, $R^7$ und $R^8$ gleich oder verschieden sind und jeweils

- für Halogen, Cyano, Hydroxy, Nitro,
- für einen 3- bis 7-gliedrigen Heterocyclus mit bis zu 4 Heteroatomen aus der Reihe Schwefel, Sauerstoff oder Stickstoff, Pyrid-3-yl-methojodid oder
- für Aryl mit 6 bis 10 Kohlenstoffatomen stehen, die ihrerseits durch Halogen, Cyano, Phenyl, Nitro, geradkettiges oder verzweigtes Alkyl oder Alkoxy mit jeweils bis zu 8 Kohlenstoffatomen oder Hydroxy substituiert sein können, oder
- für Cycloalkenyl mit 3 bis 8 Kohlenstoffatomen stehen,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,

oder $R^1$ und $R^2$, $R^2$ und $R^3$, $R^3$ und $R^4$, $R^5$ und $R^6$, $R^6$ und $R^7$ oder $R^7$ und $R^8$ jeweils gemeinsam einen gesättigten oder ungesättigten 5- bis 7-gliedrigen Carbocyclus bilden, der gegebenenfalls durch Nitro, Cyano, Hydroxy, geradkettiges oder verzweigtes Alkyl mit bis zu 8 Kohlenstoffatomen, oder durch eine Gruppe der Formel $-NR^9R^{10}$, $-COR^{11}$ oder $-OR^{12}$ substituiert ist,

$R^{14}$ und $R^{15}$ gleich oder verschieden sind und jeweils

- Wasserstoff, geradkettiges oder verzweigtes Alkyl mit bis zu 10 Kohlenstoffatomen bedeuten, das gegebenenfalls durch Halogen, Nitro, Cyano, Hydroxy oder eine Gruppe der Formel
  $-NR^9R^{10}$, $-COR^{11}$ oder $-OR^{12}$ substituiert ist
  worin
  $R^9$, $R^{10}$, $R^{11}$ und $R^{12}$ die oben angegebene Bedeutung haben,
- Aryl mit 6 bis 10 Kohlenstoffatomen bedeuten, das gegebenenfalls durch Nitro, Cyano, Halogen, Alkyl, Alkoxy oder Alkoxycarbonyl mit bis zu 8 Kohlenstoffatomen oder durch eine Gruppe der Formel $-NR^9R^{10}$ substituiert ist,
  worin
  $R^9$ und $R^{10}$ die oben angegebene Bedeutung haben

oder $R^{14}$ und $R^{15}$ gemeinsam einen 5- bis 7-gliedrigen, gesättigten oder ungesättigten Carbocyclus bilden

und deren physiologisch unbedenklichen Salze, zur Anwendung bei der Bekämpfung von Erkrankungen.

2. Verbindungen der allgemeinen Formel (I) gemäß Anspruch 1, in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 10 Kohlenstoffatomen stehen, die gegebenenfalls bis zu 4fach gleich oder verschieden durch folgende Reste wie Fluor, Chlor, Brom, Azido, Imino, hydroxysubstituiertes Imino, Hydroxy, Cyano, Cyclopropyl, Cyclopentyl, Cyclohexyl, Pyrryl, Morpholino, Piperidyl oder durch Oxiranyl oder durch Phenyl substituiert sind, die ihrerseits bis zu 3-fach gleich oder verschieden durch Fluor, Chlor, Brom, Jod, Nitro, Phenyl, Phenoxy, Cyano, geradkettiges oder verzweigtes Alkyl, Alkoxy oder Alkoxycarbonyl mit jeweils bis zu 6 Kohlenstoffatomen substituiert sein können, oder durch einen Rest der Formel

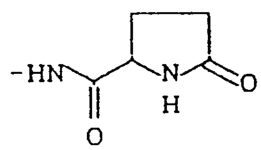

oder durch eine Gruppe der Formel $-NR^9R^{10}$, $-COR^{11}$ oder $-OR^{12}$ substituiert sind,

worin

$R^9$ und $R^{10}$ gleich oder verschieden sind und jeweils Wasserstoff, geradkettiges oder verzweigtes Alkyl mit bis zu 8 Kohlenstoffatomen, Phenyl oder eine Gruppe der Formel $-S(O)_pR^{13}$ bedeuten,

$R^{11}$ Wasserstoff, Hydroxy, geradkettiges oder verzweigtes Alkyl oder Alkoxy mit bis zu 8 Kohlenstoffatomen, Phenoxy, Phenyl oder die Gruppe $-NR^9R^{10}$ bedeutet,

worin

$R^9$ und $R^{10}$ die oben angegebene Bedeutung haben,

$R^{12}$ Wasserstoff, Cyclopropyl, Cyclobutyl, Cyclopentyl, Cyclohexyl, Cycloheptyl, Formyl, Acyl mit bis zu 6 Kohlenstoffatomen, Tetrahydropyranyl, Trifluormethoxy oder eine Gruppe der Formel $-S(O)_p-R^{13}$ bedeutet,

worin

p eine Zahl 1 oder 2 bedeutet,

$R^{13}$ - geradkettiges oder verzweigtes Alkyl mit bis zu 6 Kohlenstoffatomen oder die Gruppe $-NR^9R^{10}$ bedeutet,

worin

$R^9$ und $R^{10}$ die oben angegebene Bedeutung haben

oder

$R^{12}$ geradkettiges oder verzweigtes Alkyl oder Alkenyl mit bis zu 8 Kohlenstoffatomen bedeutet, das gegebenenfalls bis zu 4-fach gleich oder verschieden durch folgende Reste wie Hydroxy, Fluor, Chlor, Brom, Cyclopropyl, Cyclopentyl, Cyclohexyl, Alkoxy oder Alkoxycarbonyl mit bis zu 6 Kohlenstoffatomen, oder durch Phenyl substituiert ist, das seinerseits durch Fluor, Chlor, Brom, Hydroxy, Alkyl, Alkoxy oder Alkoxycarbonyl mit jeweils bis zu 6 Kohlenstoffatomen, Nitro oder Cyano substituiert sein kann, oder durch Oxiranyl, Pyrimidyl, Pyridyl oder durch eine Gruppe der Formel $-NR^9R^{10}$, $-COR^{11}$ oder $-OR^{12}$ substituiert ist,

worin

$R^9$, $R^{10}$, $R^{11}$ und $R^{12}$ die oben angegebene Bedeutung haben,

oder durch einen Rest der Formel

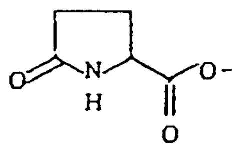, 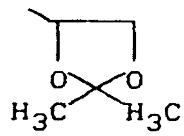 oder 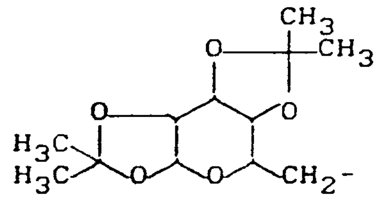

substituiert ist,
oder

$R^1$, $R^2$, $R^3$, $R^4$, $R^5$, $R^6$, $R^7$ und $R^8$ gleich oder verschieden sind und jeweils

- für Fluor, Chlor, Brom, Jod, Cyano, Hydroxy, Nitro, Pyrid-3-yl-methojodid, 2,3-Dihydrofuryl, Furyl, Pyridyl, Thienyl, Dihydropyranyl, Thiazolyl, Oxiranyl oder für Phenyl stehen, die gegebenenfalls durch Fluor, Chlor, Brom, Phenyl, geradkettiges oder verzweigtes Alkyl oder Alkoxy mit bis zu 6 Kohlenstoffatomen oder Hydroxy substiutiert sind, oder
- für Cyclobutenyl, Cyclopentenyl oder Cyclohexenyl stehen,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,
  oder $R^1$ und $R^2$, $R^2$ und $R^3$, $R^3$ und $R^4$, $R^5$ und $R^6$, $R^6$ und $R^7$ oder $R^7$ und $R^8$ gemeinsam für Phenyl, Cyclopentyl oder Cyclohexyl stehen,

$R^{14}$ und $R^{15}$ gleich oder verschieden sind und jeweils

- Wasserstoff, geradkettiges oder verzweigtes Alkyl mit bis zu 8 Kohlenstoffatomen bedeuten, das gegebenenfalls durch Fluor, Chlor, Brom, Cyano, Hydroxy oder durch eine Gruppe der Formel $-NR^9R^{10}$, $-COR^{11}$ oder $-OR^{12}$ substituiert ist,
  worin
  $R^9$, $R^{10}$, $R^{11}$ und $R^{12}$ die oben angegebene Bedeutung haben,
  oder
- Phenyl bedeuten, das gegebenenfalls durch Nitro, Cyano, Fluor, Chlor, Brom, Alkyl, Alkoxy oder Alkoxycarbonyl mit bis zu 6 Kohlenstoffatomen oder durch die Gruppe der Formel $-NR^9R^{10}$ substituiert ist,
  worin
  $R^9$ und $R^{10}$ die oben angegebene Bedeutung haben

und deren physiologisch unbedenklichen Salze, zur Anwendung bei der Bekämpfung von Erkrankungen.

**3.** Verbindungen der allgemeinen Formel (I) gemäß Anspruch 1,
in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 8 Kohlenstoffatomen stehen, die gegebenenfalls bis zu 4-fach gleich oder verschieden durch folgende Reste wie Fluor, Chlor, Brom, Jod, Azido, Imino, hydroxysubstituiertes Imino, Hydroxy, Cyclopropyl, Cyclopentyl, Cyclohexyl, Morpholino,

Piperidyl, Pyrryl oder durch Oxiranyl oder Phenylsubstituiert sind, die ihrerseits bis zu 3-fach gleich oder verschieden durch Fluor, Chlor, Phenyl, Phenoxy oder durch geradkettiges oder verzweigtes Alkyl oder Alkoxy mit bis zu 4 Kohlenstoffatomen substituiert sein können, oder durch einen Rest der Formel

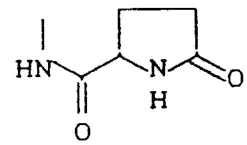

oder durch eine Gruppe der Formel $-NR^9R^{10}$, $-COR^{11}$ oder $-OR^{12}$ substituiert sind,
worin

$R^9$ und $R^{10}$ gleich oder verschieden sind und jeweils
- Wasserstoff, geradkettiges oder verzweigtes Alkyl mit bis zu 6 Kohlenstoffatomen, Phenyl oder eine Gruppe der Formel $-S(O)_pR^{13}$ bedeuten,

$R^{11}$ Wasserstoff, Hydroxy, Trifluormethyl, geradkettiges oder verzweigtes Alkyl oder Alkoxy mit bis zu 6 Kohlenstoffatomen, Phenoxy, Phenyl oder die Gruppe $-NR^9R^{10}$ bedeutet,
worin
$R^9$ und $R^{10}$ die oben angegebene Bedeutung haben,

$R^{12}$ Wasserstoff, Cyclopropyl, Cyclopentyl, Cyclohexyl, Cycloheptyl, Formyl, Acyl mit bis zu 4 Kohlenstoffatomen, Tetrahydropyranyl, Trifluormethoxy oder eine Gruppe der Formel $-S(O)_p-R^{13}$ bedeutet,
worin

p eine Zahl 1 oder 2 bedeutet,

$R^{13}$ - geradkettiges oder verzweigtes Alkyl mit bis zu 4 Kohlenstoffatomen bedeutet,

oder geradkettiges oder verzweigtes Alkyl oder Alkenyl mit bis zu 6 Kohlenstoffatomen bedeutet, das gegebenenfalls bis zu 3fach gleich oder verschieden durch folgende Reste wie Hydroxy, Fluor, Chlor, Brom, Cyclopropyl, Cyclopentyl, Cyclohexyl, Alkoxy oder Alkoxycarbonyl mit bis zu 4 Kohlenstoffatomen oder durch Phenyl substituiert ist, das seinerseits durch Fluor, Chlor, Hydroxy oder Alkoxy mit bis zu 4 Kohlenstoffatomen substituiert sein kann, oder durch Oxiranyl oder durch eine Gruppe der Formel $-NR^9R^{10}$, $-COR^{11}$ oder $-OR^{12}$ substituiert ist,
worin
$R^9$, $R^{10}$, $R^{11}$ und $R^{12}$ die oben angegebene Bedeutung haben,
oder durch einen Rest der Formel

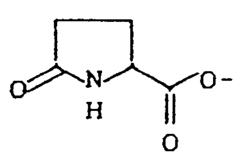, 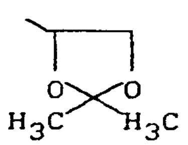 oder 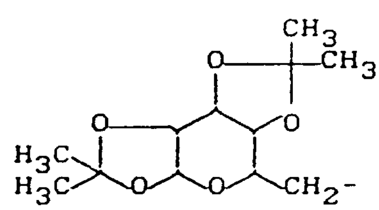

substituiert ist,

oder

$R^1$, $R^2$, $R^3$, $R^4$, $R^5$, $R^6$, $R^7$ und $R^8$ gleich oder verschieden sind und jeweils

- für Fluor, Chlor, Brom, Jod, Hydroxy oder Nitro stehen oder für Phenyl, Pyrid-3-yl-methojodid, 2,3-Dihydrofuryl, Pyrryl, Pyridyl, Thienyl, Dihydropyranyl, Thiazolyl oder Oxiranyl stehen, die ihrerseits durch Fluor, Chlor, Methyl, Isopropyl, Phenyl oder Ethoxy substituiert sein können,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,

$R^{14}$ und $R^{15}$ gleich oder verschieden sind und Wasserstoff, geradkettiges oder verzweigtes Alkyl mit bis zu 6 Kohlenstoffatomen oder Phenyl bedeuten,

und deren physiologisch unbedenklichen Salze, zur Anwendung bei der Bekämpfung von Erkrankungen.

**4.** Verbindungen der allgemeinen Formel (I) gemäß Anspruch 1, in welcher $R^1$-$R^8$, $R^{14}$ und $R^{15}$ die im Anspruch 1 angegebene Bedeutung haben, mit der Maßgabe, daß

a) $R^8$ nicht Hydroxy, Methoxy oder Acetyl bedeuten darf, wenn $R^1$ für Methoxy, $R^3$, $R^4$, $R^5$, $R^7$, $R^{14}$ und $R^{15}$ für Wasserstoff, $R^6$ für Methyl und $R^2$ für die Gruppe

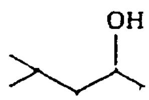

steht,

b) $R^5$ und $R^7$ nicht Brom und $R^8$ nicht Hydroxy bedeuten dürfen, wenn $R^1$ für Methoxy, $R^3$, $R^4$, $R^{14}$ und $R^{15}$ für Wasserstoff, $R^6$ für Methyl und $R^2$ für die Gruppe

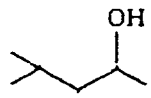

steht,

oder

c) $R^8$ nicht Methoxy bedeuten darf, wenn $R^1$ für Methoxy, $R^3$, $R^4$, $R^5$, $R^7$, $R^{14}$ und $R^{15}$ für Wasserstoff, $R^6$ für Methyl und $R^7$ für die Gruppe

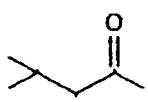

steht,

oder

d) $R^1$ nicht Methoxy bedeuten darf, wenn $R^3$, $R^4$, $R^5$, $R^6$, $R^{14}$ und $R^{15}$ für Wasserstoff und $R^2$ für die Gruppe der Formel

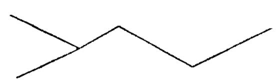

steht.

5. Verwendung von Verbindungen der allgemeinen Formel (I) gemäß Anspruch 1 bei der Herstellung von Kreislaufmitteln.

6. Verfahren zur Herstellung von Verbindungen der allgemeinen Formel (I)

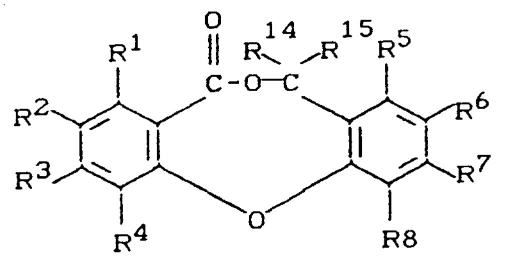

(I)

in welcher
$R^1$ bis $R^{15}$ die in Anspruch 1 angegebene Bedeutung haben, dadurch gekennzeichnet, daß man
[A] Verbindungen der allgemeinen Formel (II)

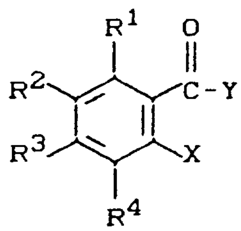

(II)

in welcher
$R^1$, $R^2$, $R^3$ und $R^4$ die oben angegebene Bedeutung haben,
X - für Fluor, Chlor, Brom oder Jod steht
und
Y - für $(C_1$-$C_6)$-Alkoxy oder Aryloxy mit 6 bis 10 Kohlenstoffatomen steht,
mit Verbindungen der allgemeinen Formel (III)

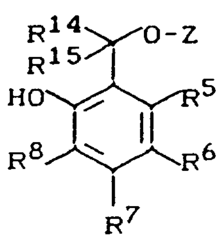

(III)

in welcher
$R^5$, $R^6$, $R^7$, $R^8$, $R^{14}$ und $R^{15}$ die oben angegebene Bedeutung haben
und
Z - für eine typische Hydroxylschutzgruppe, wie beispielsweise Tetrahydropyranyl steht,
oder Verbindungen der allgemeinen Formel (IIa)

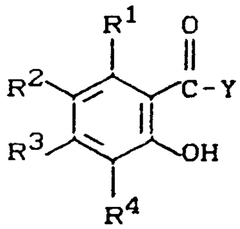

(IIa)

in welcher
$R^1$, $R^2$, $R^3$, $R^4$ und Y die oben angegebene Bedeutung haben,
mit Verbindungen der allgemeinen Formel (IIIa)

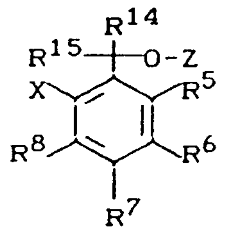

(IIIa)

in welcher
$R^5$, $R^6$, $R^7$, $R^8$, $R^{14}$, $R^{15}$, X und Z die oben angegebene Bedeutung haben,
unter Abspaltung von Halogenwasserstoffsäuren, wie beispielsweise Bromwasserstoff, zu Verbindungen der allgemeinen Formel (IV)

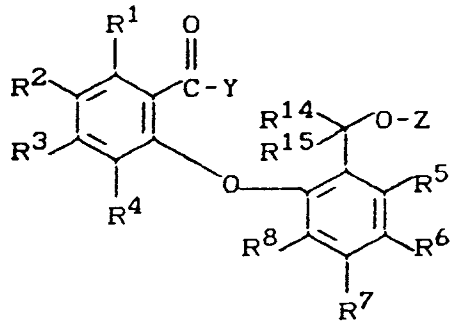

(IV)

in welcher
$R^1$, $R^2$, $R^3$, $R^4$, $R^5$, $R^6$, $R^7$, $R^8$, $R^{14}$, $R^{15}$, Y und Z die oben angegebene Bedeutung haben,
in inerten Lösemitteln kondensiert, anschließend die Hydroxylgruppe nach üblicher Methode deblokkiert und unter Wasserabspaltung cyclisiert, wobei die Substituenten $R^1$, $R^2$, $R^3$, $R^4$, $R^5$, $R^6$, $R^7$, $R^8$, $R^{14}$ und $R^{15}$ sowohl vor der Kondensation in die Verbindungen der allgemeinen Formeln (II), (IIa), (III) und (IIIa) als auch nach der Cyclisierung in die Verbindungen der allgemeinen Formel (IV) nach bekannten Methoden, wie beispielsweise durch Alkylierung, Acylierung, Substitution, Addition, Eliminierung, Umlagerung, Oxidation, Radikalreaktion oder Reduktion eingeführt und gegebenenfalls anschließend in andere funktionelle Gruppen überführt werden können,
oder indem man

[B] ausgehend von dem Naturstoff der Formel (Ib)

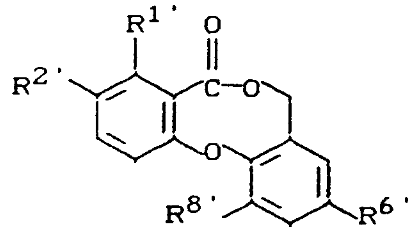

(Ib)

in welcher

$R^{1'}$ für Methoxy steht,

$R^{2'}$ für die Gruppe

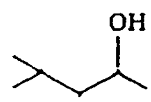

steht,

$R^{6'}$ für Methyl steht, und

$R^{8'}$ für Hydroxy steht

nach den unter Verfahren [A] erwähnten üblichen Methoden, wie beispielsweise Umlagerung, Alkylierung, Acylierung, Addition, Eliminierung, Oxidation, Radikalreaktion oder Reduktion, in inerten Lösemitteln, gegebenenfalls in Anwesenheit von Hilfsstoffen, wie beispielsweise Basen, Säuren, Katalysatoren oder akitvierenden Reagenzien, die Substituenten $R^1$, $R^2$, $R^3$, $R^4$, $R^5$, $R^6$, $R^7$, $R^8$, $R^{14}$ und $R^{15}$ einführt und diese wie auch die Substituenten $R^{1'}$, $R^{2'}$, $R^{6'}$ und $R^{8'}$ in andere funktionelle Gruppen überführt.

**7.** Arzneimittel enthaltend mindestens eine Verbindung der allgemeinen Formel (I) gemäß Anspruch 1.

**8.** Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

**9.** Pilz vom Stamm Penicillium funiculosum Thom mit der DSM-Hinterlegungsnummer 5249.

**10.** Verwendung des Pilzes gemäß Anspruch 9, zur Herstellung von Verbindungen der allgemeinen Formel (I) gemäß Anspruch 1.

**Patentansprüche für folgenden Vertragsstaat : GR**

**1.** Verfahren zur Herstellung von Verbindungen der allgemeinen Formel (I)

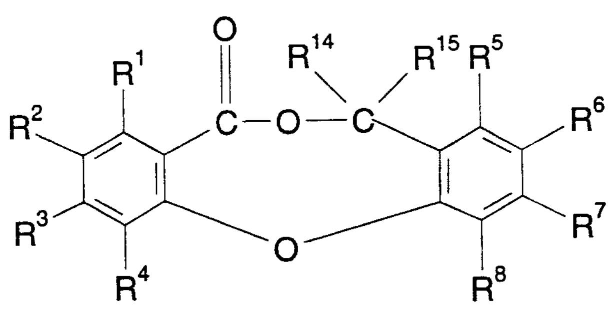

(I)

in welcher

$R^1$, $R^2$, $R^3$, $R^4$, $R^5$, $R^6$, $R^7$ und $R^8$ gleich oder verschieden sind und jeweils

- für Wasserstoff oder
- für geradkettiges oder verzweigtes Alkylthio, Alkyl, Alkenyl oder Alkinyl mit jeweils bis zu 12 Kohlenstoffatomen stehen, die gegebenenfalls durch Halogen, Azido, Imino, hydroxysubstituiertes Imino, Hydroxy, Cyano, Cycloalkyl mit 3 bis 8 Kohlenstoffatomen, oder durch einen 3- bis 7-gliedrigen, gesättigten oder ungesättigten Heterocyclus mit bis zu 4 Heteroatomen aus der Reihe Stickstoff, Schwefel oder Sauerstoff, oder durch Aryl oder Aryloxy mit 6 bis 10 Kohlenstoffatomen substituiert sind, die ihrerseits bis zu 3-fach gleich oder verschieden durch Halogen, Nitro, Phenyl, Phenoxy, Cyano, geradkettiges oder verzweigtes Alkyl, Alkoxy oder Alkoxycarbonyl mit jeweils bis zu 8 Kohlenstoffatomen substituiert sein können, oder durch einen Rest der Formel

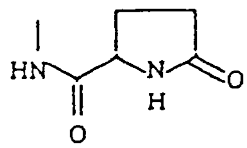

oder durch eine Gruppe der Formel $-NR^9R^{10}$, $-COR^{11}$ oder $-OR^{12}$ substituiert sind,
worin

$R^9$ und $R^{10}$ gleich oder verschieden sind und jeweils Wasserstoff, geradkettiges oder verzweigtes Alkyl oder Alkoxy mit bis zu 10 Kohlenstoffatomen, Aryl mit 6 bis 10 Kohlenstoffatomen oder eine Gruppe der Formel $-S(O)_pR^{13}$ bedeuten,

$R^{11}$ Wasserstoff, Hydroxy, geradkettiges oder verzweigtes Alkyl mit bis zu 10 Kohlenstoffatomen, Phenoxy, Aryl mit 6 bis 10 Kohlenstoffatomen oder die Gruppe $-NR^9R^{10}$ bedeutet,
worin
$R^9$ und $R^{10}$ die oben angegebene Bedeutung haben,

$R^{12}$ Wasserstoff, Cycloalkyl mit 3 bis 8 Kohlenstoffatomen, Formyl, Acyl mit bis zu 8 Kohlenstoffatomen, Tetrahyropyranyl, Trifluormethoxy oder eine Gruppe der Formel $-S(O)_pR^{13}$ bedeutet,
worin

p eine Zahl 1 oder 2 bedeutet

$R^{13}$ - geradkettiges oder verzweigtes Alkyl mit bis zu 8 Kohlenstoffatomen oder die Gruppe $-NR^9R^{10}$ bedeutet,
worin
$R^9$ und $R^{10}$ die oben angegebene Bedeutung haben,

oder

$R^{12}$ geradkettiges oder verzweigtes Alkyl oder Alkenyl mit bis zu 10 Kohlenstoffatomen bedeutet, das gegebenenfalls durch Hydroxy, Halogen, Cycloalkyl mit 3 bis 8 Kohlenstoffatomen, Alkoxy oder Alkoxycarbonyl mit bis zu 8 Kohlenstoffatomen, oder durch Aryl mit 6 bis 10 Kohlenstoffatomen substituiert ist, das seinerseits durch Halogen, Hydroxy, Alkyl, Alkoxy oder Alkoxycarbonyl mit jeweils bis zu 8 Kohlenstoffatomen, Nitro oder Cyano substituiert sein kann,
oder durch einen 3- bis 7-gliedrigen, gesättigten oder ungesättigten Heterocyclus mit bis zu 4 Heteroatomen aus der Reihe Schwefel, Sauerstoff oder Stickstoff oder durch eine Gruppe der Formel $-NR^9R^{10}$, $-COR^{11}$ oder $-OR^{12}$ substituiert ist,
worin
$R^9$, $R^{10}$, $R^{11}$ und $R^{12}$ die oben angegebene Bedeutung haben,
oder durch einen Rest der Formel

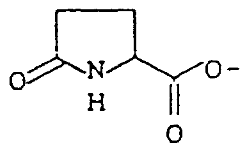 , 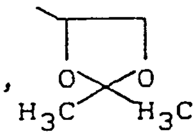 oder 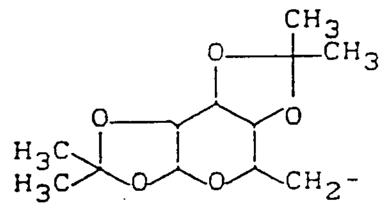

substituiert ist,
oder Aryl mit 6 bis 10 Kohlenstoffatomen bedeutet, das gegebenenfalls durch Halogen, Hydroxy, Nitro oder Cyano substituiert ist,

oder

$R^1$, $R^2$, $R^3$, $R^4$, $R^5$, $R^6$, $R^7$ und $R^8$ gleich oder verschieden sind und jeweils

- für Halogen, Cyano, Hydroxy, Nitro,
- für einen 3- bis 7-gliedrigen Heterocyclus mit bis zu 4 Heteroatomen aus der Reihe Schwefel, Sauerstoff oder Stickstoff, Pyrid-3-yl-methojodid oder
- für Aryl mit 6 bis 10 Kohlenstoffatomen stehen, die ihrerseits durch Halogen, Cyano, Phenyl, Nitro, geradkettiges oder verzweigtes Alkyl oder Alkoxy mit jeweils bis zu 8 Kohlenstoffatomen oder Hydroxy substituiert sein können, oder
- für Cycloalkenyl mit 3 bis 8 Kohlenstoffatomen stehen,
- für eine Gruppe der Formel $-NR^9R^{10}$, $-COR^{11}$ oder $-OR^{12}$ stehen,
  worin
  $R^9$, $R^{10}$, $R^{11}$ und $R^{12}$ die oben angegebene Bedeutung haben,

oder $R^1$ und $R^2$, $R^2$ und $R^3$, $R^3$ und $R^4$, $R^5$ und $R^6$, $R^6$ und $R^7$ oder $R^7$ und $R^8$ jeweils gemeinsam einen gesättigten oder ungesättigten 5- bis 7-gliedrigen Carbocyclus bilden, der gegebenenfalls durch Nitro, Cyano, Hydroxy, geradkettiges oder verzweigtes Alkyl mit bis zu 8 Kohlenstoffatomen, oder durch eine Gruppe der Formel $-NR^9R^{10}$, $-COR^{11}$ oder $-OR^{12}$ substituiert ist,

$R^{14}$ und $R^{15}$ gleich oder verschieden sind und jeweils

- Wasserstoff, geradkettiges oder verzweigtes Alkyl mit bis zu 10 Kohlenstoffatomen bedeuten, das gegebenenfalls durch Halogen, Nitro, Cyano, Hydroxy oder eine Gruppe der Formel
  $-NR^9R^{10}$, $-COR^{11}$ oder $-OR^{12}$ substituiert ist
  worin
  $R^9$, $R^{10}$, $R^{11}$ und $R^{12}$ die oben angegebene Bedeutung haben,
- Aryl mit 6 bis 10 Kohlenstoffatomen bedeuten, das gegebenenfalls durch Nitro, Cyano, Halogen, Alkyl, Alkoxy oder Alkoxycarbonyl mit bis zu 8 Kohlenstoffatomen oder durch eine Gruppe der Formel $-NR^9R^{10}$ substituiert ist,
  worin
  $R^9$ und $R^{10}$ die oben angegebene Bedeutung haben

oder $R^{14}$ und $R^{15}$ gemeinsam einen 5- bis 7-gliedrigen, gesättigten oder ungesättigten Carbocyclus bilden

und deren physiologisch unbedenklichen Salze, mit der Maßgabe, daß

a) $R^8$ nicht Hydroxy, Methoxy oder Acetyl bedeuten darf, wenn
$R^1$ für Methoxy, $R^3$, $R^4$, $R^5$, $R^7$, $R^{14}$ und $R^{15}$ für Wasserstoff, $R^6$ für Methyl und $R^2$ für die Gruppe

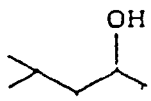

steht,

b) $R^5$ und $R^7$ nicht Brom und $R^8$ nicht Hydroxy bedeuten dürfen, wenn $R^1$ für Methoxy, $R^3$, $R^4$, $R^{14}$ und $R^{15}$ für Wasserstoff, $R^6$ für Methyl und $R^2$ für die Gruppe

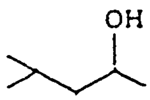

steht,

oder

c) $R^8$ nicht Methoxy bedeuten darf, wenn $R^1$ für Methoxy, $R^3$, $R^4$, $R^5$, $R^7$, $R^{14}$ und $R^{15}$ für Wasserstoff, $R^6$ für Methyl und $R^7$ für die Gruppe

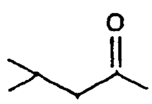

steht,

oder

d) $R^1$ nicht Methoxy bedeuten darf, wenn $R^3$, $R^4$, $R^5$, $R^6$, $R^{14}$ und $R^{15}$ für Wasserstoff und $R^2$ für die Gruppe der Formel

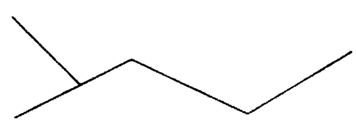

steht,

dadurch gekennzeichnet, daß man

[A] Verbindungen der allgemeinen Formel (II)

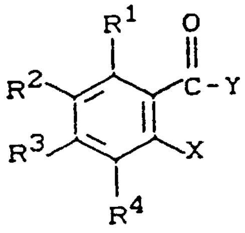

(II)

in welcher

$R^1$, $R^2$, $R^3$ und $R^4$ die oben angegebene Bedeutung haben,

X - für Fluor, Chlor, Brom oder Jod steht

und

Y - für ($C_1$-$C_6$)-Alkoxy oder Aryloxy mit 6 bis 10 Kohlenstoffatomen steht,

mit Verbindungen der allgemeinen Formel (III)

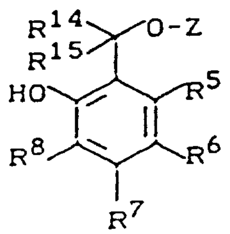

(III)

in welcher
$R^5$, $R^6$, $R^7$, $R^8$, $R^{14}$ und $R^{15}$ die oben angegebene Bedeutung haben
und
Z - für eine typische Hydroxylschutzgruppe, wie beispielsweise Tetrahydropyranyl steht,
oder Verbindungen der allgemeinen Formel (IIa)

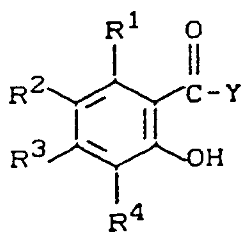

(IIa)

in welcher
$R^1$, $R^2$, $R^3$, $R^4$ und Y die oben angegebene Bedeutung haben,
mit Verbindungen der allgemeinen Formel (IIIa)

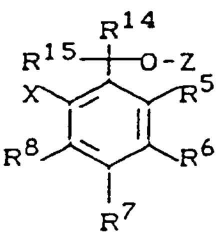

(IIIa)

in welcher
$R^5$, $R^6$, $R^7$, $R^8$, $R^{14}$, $R^{15}$, X und Z die oben angegebene Bedeutung haben,
unter Abspaltung von Halogenwasserstoffsäuren, wie beispielsweise Bromwasserstoff, zu Verbindungen der allgemeinen Formel (IV)

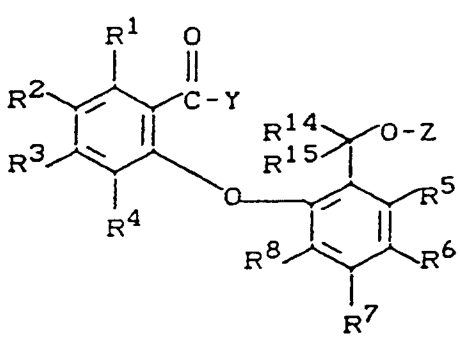

(IV)

in welcher
$R^1$, $R^2$, $R^3$, $R^4$, $R^5$, $R^6$, $R^7$, $R^8$, $R^{14}$, $R^{15}$, Y und Z die oben angegebene Bedeutung haben,
in inerten Lösemitteln kondensiert, anschließend die Hydroxylgruppe nach üblicher Methode

deblockiert und unter Wasserabspaltung cyclisiert, wobei die Substituenten $R^1$, $R^2$, $R^3$, $R^4$, $R^5$, $R^6$, $R^7$, $R^8$, $R^{14}$ und $R^{15}$ sowohl vor der Kondensation in die Verbindungen der allgemeinen Formeln (II), (IIa), (III) und (IIIa) als auch nach der Cyclisierung in die Verbindungen der allgemeinen Formel (IV) nach bekannten Methoden, wie beispielsweise durch Alkylierung, Acylierung, Substitution, Addition, Eliminierung, Umlagerung, Oxidation, Radikalreaktion oder Reduktion eingeführt und gegebenenfalls anschließend in andere funktionelle Gruppen überführt werden können,
oder indem man
[B] ausgehend von dem Naturstoff der Formel (Ib)

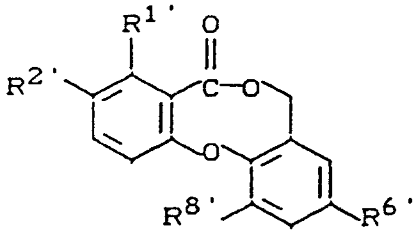

(Ib)

in welcher
$R^{1'}$ für Methoxy steht,
$R^{2'}$ für die Gruppe

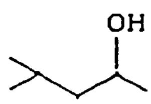

steht,
$R^{6'}$ für Methyl steht, und
$R^{8'}$ für Hydroxy steht
nach den unter Verfahren [A] erwähnten üblichen Methoden, wie beispielsweise Umlagerung, Alkylierung, Acylierung, Addition, Eliminierung, Oxidation, Radikalreaktion oder Reduktion, in inerten Lösemitteln, gegebenenfalls in Anwesenheit von Hilfsstoffen, wie beispielsweise Basen, Säuren, Katalysatoren oder akitvierenden Reagenzien, die Substituenten $R^1$, $R^2$, $R^3$, $R^4$, $R^5$, $R^6$, $R^7$, $R^8$, $R^{14}$ und $R^{15}$ einführt und diese wie auch die Substituenten $R^{1'}$, $R^{2'}$, $R^{6'}$ und $R^{8'}$ in andere funktionelle Gruppen überführt.

2. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

**Claims**
**Claims for the following Contracting States : AT, BE, CH, DE, DK, ES, FR, GB, IT, LI, LU, NL, SE**

1. Compounds of the general formula (I)

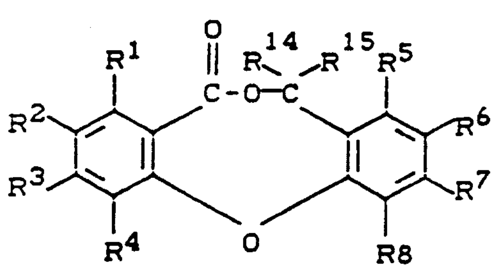

(I)

in which

$R^1$, $R^2$, $R^3$, $R^4$, $R^5$, $R^6$, $R^7$ and $R^8$ are identical or different and in each case

- represent hydrogen or
- represent straight-chain or branched alkylthio, alkyl, alkenyl or alkynyl in each case having up to 12 carbon atoms, which are optionally substituted by halogen, azido, imino, hydroxyl-substituted imino, hydroxyl, cyano, cycloalkyl having 3 to 8 carbon atoms, or by a 3- to 7-membered, saturated or unsaturated heterocycle having up to 4 heteroatoms from the series comprising nitrogen, sulphur or oxygen, or by aryl or aryloxy having 6 to 10 carbon atoms, which in turn may be monosubstituted to trisubstituted by identical or different substituents from the series comprising halogen, nitro, phenyl, phenoxy, cyano, straight-chain or branched alkyl, alkoxy or alkoxycarbonyl in each case having up to 8 carbon atoms, or by a radical of the formula

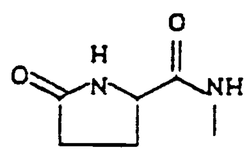

or by a group of the formula $-NR^9R^{10}$, $-COR^{11}$ or $-OR^{12}$, in which

$R^9$ and $R^{10}$ are identical or different and in each case denote hydrogen, straight-chain or branched alkyl or alkoxy having up to 10 carbon atoms, aryl having 6 to 10 carbon atoms or a group of the formula $-S(O)_pR^{13}$,

$R^{11}$ denotes hydrogen, hydroxyl, straight-chain or branched alkyl having up to 10 carbon atoms, phenoxy, aryl having 6 to 10 carbon atoms or the group $-NR^9R^{10}$,

in which

$R^9$ and $R^{10}$ have the abovementioned meaning,

$R^{12}$ denotes hydrogen, cycloalkyl having 3 to 8 carbon atoms, formyl, acyl having up to 8 carbon atoms, tetrahydropyranyl, trifluoromethoxy or a group of the formula $-S(O)_pR^{13}$, in which

p denotes a number 1 or 2,

$R^{13}$ - denotes straight-chain or branched alkyl having up to 8 carbon atoms or the group $-NR^9R^{10}$, in which $R^9$ and $R^{10}$ have the abovementioned meaning,

or

$R^{12}$ denotes straight-chain or branched alkyl or alkenyl having up to 10 carbon atoms, which is optionally substituted by hydroxyl, halogen, cycloalkyl having 3 to 8 carbon atoms, alkoxy or alkoxycarbonyl having up to 8 carbon atoms, or by aryl having 6 to 10 carbon atoms, which in turn may be substituted by halogen, hydroxyl, alkyl, alkoxy or alkoxycarbonyl in each case having up to 8 carbon atoms, or nitro or cyano, or by a 3- to 7-membered, saturated or unsaturated heterocycle having up to 4 heteroatoms from the series comprising sulphur, oxygen or nitrogen or by a group of the formula $-NR^9R^{10}$, $-COR^{11}$ or $-OR^{12}$, in which $R^9$, $R^{10}$, $R^{11}$ and $R^{12}$ have the abovementioned meaning, or is substituted by a radical of the formula

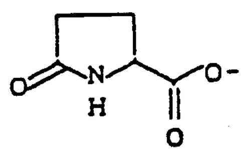 , 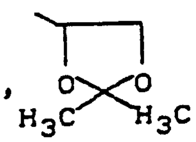 or 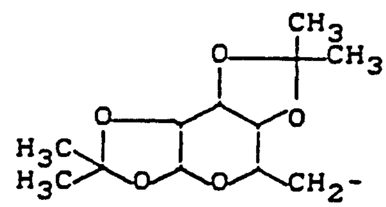

or denotes aryl having 6 to 10 carbon atoms, which is optionally substituted by halogen, hydroxyl, nitro or cyano,

or

$R^1$, $R^2$, $R^3$, $R^4$, $R^5$, $R^6$, $R^7$ and $R^8$ are identical or different and in each case

- represent halogen, cyano, hydroxyl or nitro,
- represent a 3- to 7-membered heterocycle having up to 4 heteroatoms from the series comprising sulphur, oxygen or nitrogen, pyrid-3-yl-methoiodide or
- represent aryl having 6 to 10 carbon atoms, which in turn may be substituted by halogen, cyano, phenyl, nitro, straight-chain or branched alkyl or alkoxy in each case having up to 8 carbon atoms or hydroxyl, or
- represent cycloalkenyl having 3 to 8 carbon atoms,
- represent a group of the formula $-NR^9R^{10}$, $-COR^{11}$ or $-OR^{12}$, in which $R^9$, $R^{10}$, $R^{11}$ and $R^{12}$ have the abovementioned meaning,

or $R^1$ and $R^2$, $R^2$ and $R^3$, $R^3$ and $R^4$, $R^5$ and $R^6$, $R^6$ and $R^7$, or $R^7$ and $R^8$ in each case together form a saturated or unsaturated 5- to 7-membered carbocycle which is optionally substituted by nitro, cyano, hydroxyl, straight-chain or branched alkyl having up to 8 carbon atoms, or by a group of the formula $-NR^9R^{10}$, $-COR^{11}$ or $-OR^{12}$,

$R^{14}$ and $R^{15}$ are identical or different and in each case

- denote hydrogen, or straight-chain or branched alkyl having up to 10 carbon atoms, which is optionally substituted by halogen, nitro, cyano, hydroxyl or a group of the formula $-NR^9R^{10}$, $-COR^{11}$ or $-OR^{12}$, in which $R^9$, $R^{10}$, $R^{11}$ and $R^{12}$ have the abovementioned meaning,
- denote aryl having 6 to 10 carbon atoms, which is optionally substituted by nitro, cyano, halogen, alkyl, alkoxy or alkoxycarbonyl having up to 8 carbon atoms or by a group of the formula $-NR^9R^{10}$, in which $R^9$ and $R^{10}$ have the abovementioned meaning,

or $R^{14}$ and $R^{15}$ together form a 5- to 7-membered, saturated or unsaturated carbocycle

and their physiologically acceptable salts, for use in the control of disorders.

**2.** Compounds of the general formula (I) according to Claim 1, in which

$R^1$, $R^2$, $R^3$, $R^4$, $R^5$, $R^6$, $R^7$ and $R^8$ are identical or different and in each case

- represent hydrogen or
- represent straight-chain or branched alkylthio, alkyl, alkenyl or alkynyl in each case having up to 10 carbon atoms, which are optionally monosubstituted to tetrasubstituted by identical or different radicals from the following such as fluorine, chlorine, bromine, azido, imino, hydroxyl-substituted imino, hydroxyl, cyano, cyclopropyl, cyclopentyl, cyclohexyl, pyrryl, morpholino, piperidyl or by oxiranyl or phenyl, which in turn may be monosubstituted to trisubstituted by identical or different substituents from the series comprising fluorine, chlorine, bromine, iodine, nitro, phenyl, phenoxy, cyano, straight-chain or branched alkyl, alkoxy or alkoxycarbonyl in each case having up to 6 carbon atoms, or by a radical of the formula

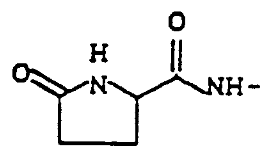

or are substituted by a group of the formula $-NR^9R^{10}$, $-COR^{11}$ or $-OR^{12}$,

in which

$R^9$ and $R^{10}$ are identical or different and in each case denote hydrogen, straight-chain or branched alkyl having up to 8 carbon atoms, phenyl or a group of the formula $-S(O)_pR^{13}$,

$R^{11}$ denotes hydrogen, hydroxyl, straight-chain or branched alkyl or alkoxy having up to 8 carbon atoms, phenoxy, phenyl or the group $-NR^9R^{10}$,

in which

$R^9$ and $R^{10}$ have the abovementioned meaning,

$R^{12}$ denotes hydrogen, cyclopropyl, cyclobutyl, cyclopentyl, cyclohexyl, cycloheptyl, formyl, acyl having up to 6 carbon atoms, tetrahydropyranyl, trifluoromethoxy or a group of the formula $-S(O)_p-R^{13}$,

in which

p denotes a number 1 or 2,

$R^{13}$ - denotes straight-chain or branched alkyl having up to 6 carbon atoms or the group $-NR^9R^{10}$,

in which

$R^9$ and $R^{10}$ have the abovementioned meaning

or

$R^{12}$ denotes straight-chain or branched alkyl or alkenyl having up to 8 carbon atoms, which is optionally monosubstituted to tetrasubstituted by identical or different radicals from the following such as hydroxyl, fluorine, chlorine, bromine, cyclopropyl, cyclopentyl, cyclohexyl, alkoxy or alkoxycarbonyl having up to 6 carbon atoms, or by phenyl which may in turn be substituted by fluorine, chlorine, bromine, hydroxyl, alkyl, alkoxy or alkoxycarbonyl in each case having up to 6 carbon atoms, nitro or cyano, or is substituted by oxiranyl, pyrimidyl, pyridyl or by a group of the formula $-NR^9R^{10}$, $-COR^{11}$ or $-OR^{12}$,

in which

$R^9$, $R^{10}$, $R^{11}$ and $R^{12}$ have the abovementioned meaning,

or is substituted by a radical of the formula

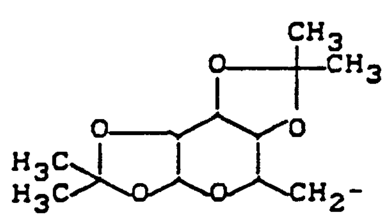

or

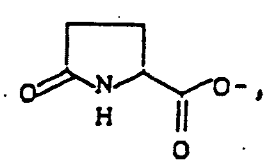

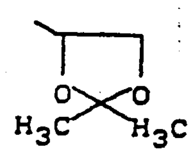

or

$R^1$, $R^2$, $R^3$, $R^4$, $R^5$, $R^6$, $R^7$ and $R^8$ are identical or different and in each case

- represent fluorine, chlorine, bromine, iodine, cyano, hydroxyl, nitro, pyrid-3-yl-methoiodide, 2,3-dihydrofuryl, furyl, pyridyl, thienyl, dihydropyranyl, thiazolyl, oxiranyl or phenyl, which are optionally substituted by fluorine, chlorine, bromine, phenyl, straight-chain or branched alkyl or alkoxy having up to 6 carbon atoms or hydroxyl, or
- represent cyclobutenyl, cyclopentenyl or cyclohexenyl,
- represent a group of the formula $-NR^9R^{10}$, $-COR^{11}$ or $-OR^{12}$,
  in which
  $R^9$, $R^{10}$, $R^{11}$ and $R^{12}$ have the abovementioned meaning,
  or $R^1$ and $R^2$, $R^2$ and $R^3$, $R^3$ and $R^4$, $R^5$ and $R^6$, $R^6$ and $R^7$, or $R^7$ and $R^8$ together represent phenyl, cyclopentyl or cyclohexyl,

$R^{14}$ and $R^{15}$ are identical or different and in each case

- denote hydrogen, or straight-chain or branched alkyl having up to 8 carbon atoms, which is optionally substituted by fluorine, chlorine, bromine, cyano, hydroxyl or by a group of the formula $-NR^9R^{10}$, $-COR^{11}$ or $-OR^{12}$,
  in which
  $R^9$, $R^{10}$, $R^{11}$ and $R^{12}$ have the abovementioned meaning,
  or
- denote phenyl which is optionally substituted by nitro, cyano, fluorine, chlorine, bromine, alkyl, alkoxy or alkoxycarbonyl having up to 6 carbon atoms or by the group of the formula $-NR^9R^{10}$,
  in which
  $R^9$ and $R^{10}$ have the abovementioned meaning

and their physiologically acceptable salts, for use in the control of disorders.

**3.** Compounds of the general formula (I) according to Claim 1,
in which

$R^1$, $R^2$, $R^3$, $R^4$, $R^5$, $R^6$, $R^7$ and $R^8$ are identical or different and in each case

- represent hydrogen or
- represent straight-chain or branched alkylthio, alkyl, alkenyl or alkynyl in each case having up to 8 carbon atoms, which are optionally monosubstituted to tetrasubstituted by identical or different radicals from the following such as fluorine, chlorine, bromine, iodine, azido, imino, hydroxyl-substituted imino, hydroxyl, cyclopropyl, cyclopentyl, cyclohexyl, morpholino, piperidyl, pyrryl or are substituted by oxiranyl or phenyl, which in turn may be monosubstituted to trisubstituted by identical or different substituents from the series comprising fluorine, chlorine, phenyl, phenoxy or by straight-chain or branched alkyl or alkoxy having up to 4 carbon atoms,
  or
  are substituted by a radical of the formula

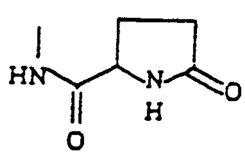

or by a group of the formula $-NR^9R^{10}$, $-COR^{11}$ or $-OR^{12}$,
in which

$R^9$ and $R^{10}$ are identical or different and in each case

- denote hydrogen, straight-chain or branched alkyl having up to 6 carbon atom, phenyl or a group of the formula $-S(O)_pR^{13}$,

$R^{11}$ denotes hydrogen, hydroxyl, trifluoromethyl, straight-chain or branched alkyl or alkoxy having up to 6 carbon atoms, phenoxy, phenyl or the group $-NR^9R^{10}$,
in which
$R^9$ and $R^{10}$ have the abovementioned meaning,

$R^{12}$ denotes hydrogen, cyclopropyl, cyclopentyl, cyclohexyl, cycloheptyl, formyl, acyl having up to 4 carbon atoms, tetrahydropyranyl, trifluoromethoxy or a group of the formula $-S(O)_p-R^{13}$,
in which

p denotes a number 1 or 2,

$R^{13}$ - denotes straight-chain or branched alkyl having up to 4 carbon atoms, or denotes straight-chain or branched alkyl or alkenyl having up to 6 carbon atoms, which is optionally monosubstituted to trisubstituted by identical or different radicals from the following such as hydroxyl, fluorine, chlorine, bromine, cyclopropyl, cyclopentyl, cyclohexyl, alkoxy or alkoxycarbonyl having up to 4 carbon atoms or by phenyl which may in turn be substituted by fluorine, chlorine, hydroxyl or alkoxy having up to 4 carbon atoms, or is substituted by oxiranyl or by a group of the formula $-NR^9R^{10}$, $-COR^{11}$ or $-OR^{12}$,
in which
$R^9$, $R^{10}$, $R^{11}$ and $R^{12}$ have the abovementioned meaning,
or is substituted by a radical of the formula

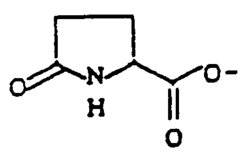 , 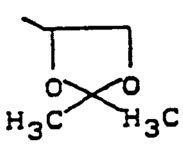 or 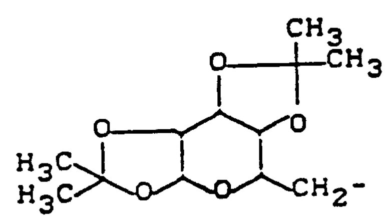

or

$R^1$, $R^2$, $R^3$, $R^4$, $R^5$, $R^6$, $R^7$ and $R^8$ are identical or different and in each case

- represent fluorine, chlorine, bromine, iodine, hydroxyl or nitro, or represent phenyl, pyrid-3-yl-methoiodide, 2,3-dihydrofuryl, pyrryl, pyridyl, thienyl, dihydropyranyl, thiazolyl or oxiranyl which may in turn be substituted by fluorine, chlorine, methyl, isopropyl, phenyl or ethoxy,
- represent a group of the formula $-NR^9R^{10}$, $-COR^{11}$ or $-OR^{12}$,
  in which
  $R^9$, $R^{10}$, $R^{11}$ and $R^{12}$ have the abovementioned meaning,

$R^{14}$ and $R^{15}$ are identical or different and denote hydrogen, straight-chain or branched alkyl having up to 6 carbon atoms or phenyl,

and their physiologically acceptable salts, for use in the control of disorders.

**4.** Compounds of the general formula (1) according to Claim 1, in which
$R^1$-$R^8$, $R^{14}$ and $R^{15}$ have the meaning indicated in Claim 1, with the proviso that

a) $R^8$ may not denote hydroxyl, methoxy or acetyl if $R^1$ represents methoxy, $R^3$, $R^4$, $R^5$, $R^7$, $R^{14}$ and $R^{15}$ represent hydrogen, $R^6$ represents methyl and $R^2$ represents the group

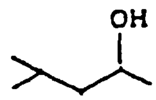

b) $R^5$ and $R^7$ may not denote bromine and $R^8$ may not denote hydroxyl if $R^1$ represents methoxy, $R^3$, $R^4$, $R^{14}$ and $R^{15}$ represent hydrogen, $R^6$ represents methyl and $R^2$ represents the group

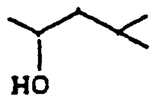

or

c) $R^8$ may not denote methoxy if, $R^1$ represents methoxy, $R^3$, $R^4$, $R^5$, $R^7$, $R^{14}$ and $R^{15}$ represent hydrogen, $R^6$ represents methyl and $R^7$ represents the group

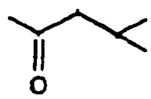

or

d) $R^1$ may not denote methoxy if $R^3$, $R^4$, $R^5$, $R^6$, $R^{14}$ and $R^{15}$ represent hydrogen and $R^2$ represents the group of the formula

**5.** Use of compounds of the general formula (I) according to Claim 1 in the production of circulatory agents.

**6.** Process for the preparation of compounds of the general formula (I)

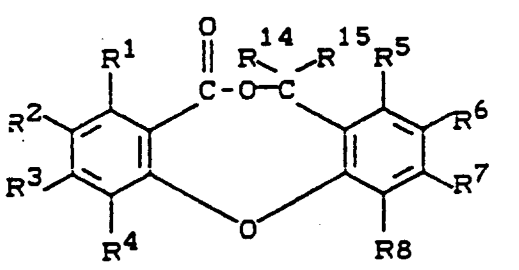

(I)

in which $R^1$ to $R^{15}$ have the meaning given in Claim 1, characterized in that

[A] compounds of the general formula (II)

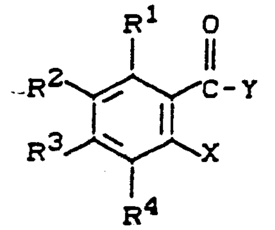

(II)

in which
$R^1$, $R^2$, $R^3$ and $R^4$ have the abovementioned meaning,
X - represents fluorine, chlorine, bromine or iodine
and
Y - represents ($C_1$-$C_6$)-alkoxy or aryloxy having 6 to 10 carbon atoms,
are condensed with compounds of the general formula (III)

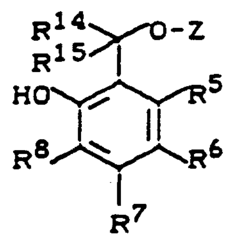

(III)

in which
$R^5$, $R^6$, $R^7$, $R^8$, $R^{14}$ and $R^{15}$ have the abovementioned meaning and
Z - represents a typical hydroxyl protecting group, such as, for example, tetrahydropyranyl,
or compounds of the general formula (IIa)

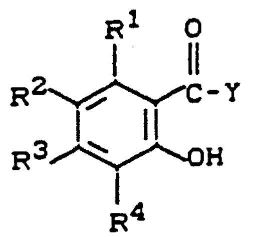

(IIa)

in which
$R^1$, $R^2$, $R^3$, $R^4$ and Y have the abovementioned meaning,
are condensed with compounds of the general formula (IIIa)

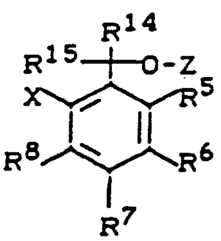

(IIIa)

in which

$R^5$, $R^6$, $R^7$, $R^8$, $R^{14}$, $R^{15}$, X and Z have the above-mentioned meaning,
in inert solvents with elimination of hydrohalic acids, such as, for example, hydrogen bromide, to give compounds of the general formula (IV)

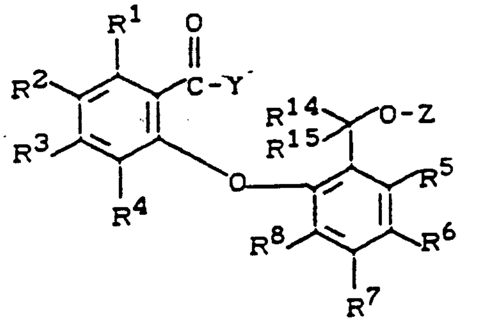

(IV)

in which
$R^1$, $R^2$, $R^3$, $R^4$, $R^5$, $R^6$, $R^7$, $R^8$, $R^{14}$, $R^{15}$, Y and Z have the abovementioned meaning,
then the hydroxyl group is deblocked by the customary method and the compound is cyclized with elimination of water,
it being possible to introduce the substituents $R^1$, $R^2$, $R^3$, $R^4$, $R^5$, $R^6$, $R^7$, $R^8$, $R^{14}$, and $R^{15}$ into the compounds of the general formulae (II), (IIa), (III) and (IIIa) both before the condensation and after the cyclization to the compounds of the general formula (IV) by known methods, such as, for example, by alkylation, acylation, substitution, addition, elimination, rearrangement, oxidation, radical reaction or reduction and, if appropriate, subsequently to convert them into other functional groups,
or in that
[B] starting from the natural substance of the formula (Ib)

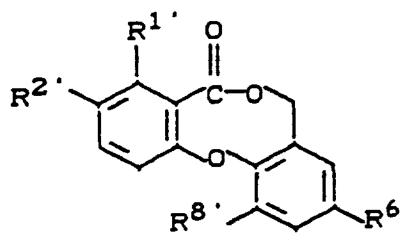

(Ib)

in which
$R^{1'}$ represents methoxy,
$R^{2'}$ represents the group

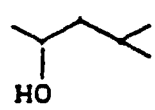
,

$R^{6'}$ represents methyl
and
$R^{8'}$ represents hydroxyl,
the substituents $R^1$, $R^2$, $R^3$, $R^4$, $R^5$, $R^6$, $R^7$, $R^8$, $R^{14}$, and $R^{15}$ are introduced by the customary methods mentioned in process [A] such as, for example, rearrangement, alkylation, acylation, addition, elimination, oxidation, radical reaction or reduction, in inert solvents, if appropriate in the presence of auxiliaries, such as, for example, bases, acids, catalysts or activating reagents, and these, as also the substituents $R^{1'}$, $R^{2'}$, $R^{6'}$ and $R^{8'}$, are converted into other functional groups.

**7.** Medicaments containing at least one compound of the general formula (I) according to Claim 1.

**8.** Process for the production of medicaments, characterized in that compounds of the general formula (I) according to Claim 1 are converted into a suitable form for administration, if appropriate using customary auxiliaries and excipients.

**9.** Fungus of the strain Penicillium funiculosum Thom with the DSM deposit number 5249.

**10.** Use of the fungus according to Claim 9 for the production of compounds of the general formula (I) according to Claim 1.

**Claims for the following Contracting State : GR**

**1.** Process for the preparation of compounds of the general formula (I)

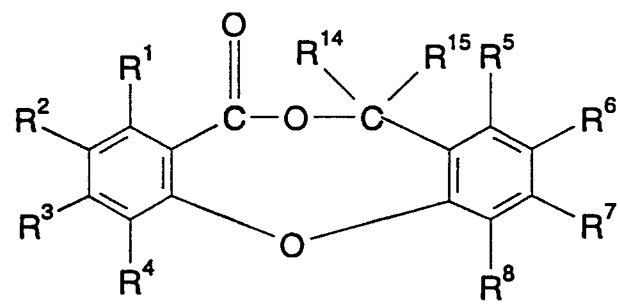

(I)

in which

| | |
|---|---|
| $R^1$, $R^2$, $R^3$, $R^4$, $R^5$, $R^6$, $R^7$ and $R^8$ | are identical or different and in each case<br>- represent hydrogen or<br>- represent straight-chain or branched alkylthio, alkyl, alkenyl or alkynyl in each case having up to 12 carbon atoms, which are optionally substituted by halogen, azido, imino, hydroxyl-substituted imino, hydroxyl, cyano, cycloalkyl having 3 to 8 carbon atoms, or by a 3- to 7-membered, saturated or unsaturated heterocycle having up to 4 heteroatoms from the series comprising nitrogen, sulphur or oxygen, or by aryl or aryloxy having 6 to 10 carbon atoms, which in turn may be monosubstituted to trisubstituted by identical or different substituents from the series comprising halogen, nitro, phenyl, phenoxy, cyano, straight-chain or branched alkyl, alkoxy or alkoxycarbonyl in each case having up to 8 carbon atoms, or by a radical of the formula |

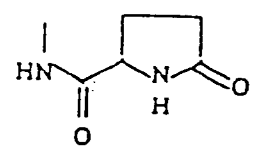

or by a group of the formula $-NR^9R^{10}$, $-COR^{11}$ or $-OR^{12}$,

in which

| | |
|---|---|
| $R^9$ and $R^{10}$ | are identical or different and in each case denote hydrogen, straight-chain or branched alkyl or alkoxy having up to 10 carbon atoms, aryl having 6 to 10 carbon atoms or a group of the formula $-S(O)_pR^{13}$, |
| $R^{11}$ | denotes hydrogen, hydroxyl, straight-chain or branched alkyl having up to 10 carbon atoms, phenoxy, aryl having 6 to 10 carbon atoms or the group $-NR^9R^{10}$, |

in which

$R^9$ and $R^{10}$ have the abovementioned meaning,

$R^{12}$ denotes hydrogen, cycloalkyl having 3 to 8 carbon atoms, formyl, acyl having up to 8 carbon atoms, tetrahydropyranyl, trifluoromethoxy or a group of the formula $-S(O)_pR^{13}$,

in which

p denotes a number 1 or 2,

$R^{13}$ - denotes straight-chain or branched alkyl having up to 8 carbon atoms or the group $-NR^9R^{10}$,

in which

$R^9$ and $R^{10}$ have the abovementioned meaning,

or

$R^{12}$ denotes straight-chain or branched alkyl or alkenyl having up to 10 carbon atoms, which is optionally substituted by hydroxyl, halogen, cycloalkyl having 3 to 8 carbon atoms, alkoxy or alkoxycarbonyl having up to 8 carbon atoms, or by aryl having 6 to 10 carbon atoms, which in turn may be substituted by halogen, hydroxyl, alkyl, alkoxy or alkoxycarbonyl in each case having up to 8 carbon atoms, or nitro or cyano,

or by a 3- to 7-membered, saturated or unsaturated heterocycle having up to 4 heteroatoms from the series comprising sulphur, oxygen or nitrogen or by a group of the formula $-NR^9R^{10}$, $-COR^{11}$ or $-OR^{12}$,

in which

$R^9$, $R^{10}$, $R^{11}$ and $R^{12}$ have the abovementioned meaning,

or is substituted by a radical of the formula

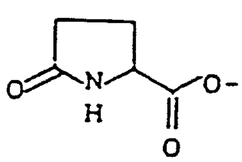 , 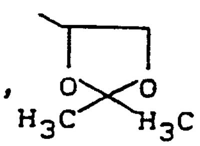 or 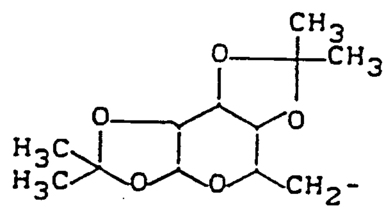

or denotes aryl having 6 to 10 carbon atoms, which is optionally substituted by halogen, hydroxyl, nitro or cyano,

or

$R^1$, $R^2$, $R^3$, $R^4$, $R^5$, $R^6$, $R^7$ and $R^8$ are identical or different and in each case

- represent halogen, cyano, hydroxyl or nitro,
- represent a 3- to 7-membered heterocycle having up to 4 heteroatoms from the series comprising sulphur, oxygen or nitrogen, pyrid-3-yl-methoiodide or
- represent aryl having 6 to 10 carbon atoms, which in turn may be substituted by halogen, cyano, phenyl, nitro, straight-chain or branched alkyl or alkoxy in each case having up to 8 carbon atoms or hydroxyl, or
- represent cycloalkenyl having 3 to 8 carbon atoms,
- represent a group of the formula $-NR^9R^{10}$, $-COR^{11}$ or $-OR^{12}$,

in which

$R^9$, $R^{10}$, $R^{11}$ and $R^{12}$ have the abovementioned meaning,

or $R^1$ and $R^2$, $R^2$ and $R^3$, $R^3$ and $R^4$, $R^5$ and $R^6$, $R^6$ and $R^7$, or $R^7$ and $R^8$ in each case together form a saturated or unsaturated 5- to 7-membered carbocycle which is optionally substituted by nitro, cyano, hydroxyl, straight-chain or branched alkyl having up to 8 carbon atoms, or by a group of the formula $-NR^9R^{10}$, $-COR^{11}$ or $-OR^{12}$,

$R^{14}$ and $R^{15}$ are identical or different and in each case

- denote hydrogen, or straight-chain or branched alkyl having up to 10 carbon atoms, which is optionally substituted by halogen, nitro, cyano, hydroxyl or a group of the

formula
$-NR^9R^{10}$, $-COR^{11}$ or $-OR^{12}$,
in which
$R^9$, $R^{10}$, $R^{11}$ and $R^{12}$ have the abovementioned meaning,

- denote aryl having 6 to 10 carbon atoms, which is optionally substituted by nitro, cyano, halogen, alkyl, alkoxy or alkoxycarbonyl having up to 8 carbon atoms or by a group of the formula $-NR^9R^{10}$,
in which
$R^9$ and $R^{10}$ have the abovementioned meaning,

or $R^{14}$ and $R^{15}$ together form a 5- to 7-membered, saturated or unsaturated carbocycle

and their physiologically acceptable salts, with the proviso that

a) $R^8$ may not denote hydroxyl, methoxy or acetyl if $R^1$ represents methoxy, $R^3$, $R^4$, $R^5$, $R^7$, $R^{14}$ and $R^{15}$ represent hydrogen, $R^6$ represents methyl and $R^2$ represents the group

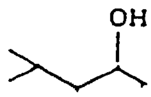

b) $R^5$ and $R^7$ may not denote bromine and $R^8$ may not denote hydroxyl if
$R^1$ represents methoxy, $R^3$, $R^4$, $R^{14}$ and $R^{15}$ represent hydrogen, $R^6$ represents methyl and $R^2$ represents the group

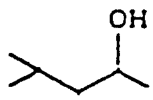

or

c) $R^8$ may not denote methoxy if,
$R^1$ represents methoxy, $R^3$, $R^4$, $R^5$, $R^7$, $R^{14}$ and $R^{15}$ represent hydrogen, $R^6$ represents methyl and $R^7$ represents the group

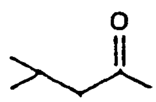

or

d) $R^1$ may not denote methoxy if
$R^3$, $R^4$, $R^5$, $R^6$, $R^{14}$ and $R^{15}$ represent hydrogen and $R^2$ represents the group of the formula

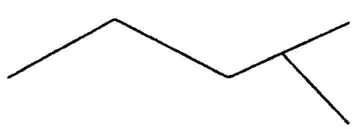

[A] compounds of the general formula (II)

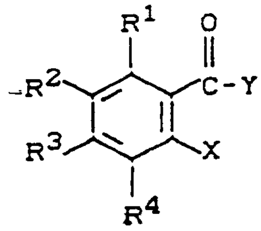

(II)

in which
$R^1$, $R^2$, $R^3$ and $R^4$ have the abovementioned meaning,
X - represents fluorine, chlorine, bromine or iodine
and
Y - represents ($C_1$-$C_6$)-alkoxy or aryloxy having 6 to 10 carbon atoms,
are condensed with compounds of the general formula (III)

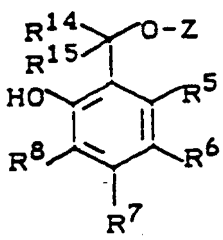

(III)

in which
$R^5$, $R^6$, $R^7$, $R^8$, $R^{14}$ and $R^{15}$ have the abovementioned meaning
and
Z - represents a typical hydroxyl protecting group, such as, for example, tetrahydropyranyl,
or compounds of the general formula (IIa)

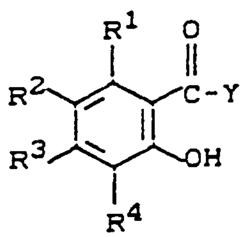

(IIa)

in which
$R^1$, $R^2$, $R^3$, $R^4$ and Y have the abovementioned meaning,
are condensed with compounds of the general formula (IIIa)

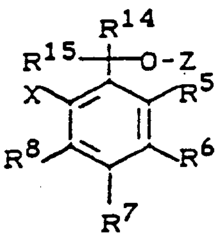

(IIIa)

in which
$R^5$, $R^6$, $R^7$, $R^8$, $R^{14}$, $R^{15}$, X and Z have the above-mentioned meaning,

in inert solvents with elimination of hydrohalic acids, such as, for example, hydrogen bromide, to give compounds of the general formula (IV)

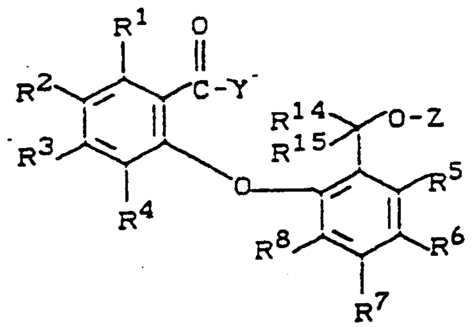

(IV)

in which
$R^1$, $R^2$, $R^3$, $R^4$, $R^5$, $R^6$, $R^7$, $R^8$, $R^{14}$, $R^{15}$, Y and Z have the abovementioned meaning,
then the hydroxyl group is deblocked by the customary method and the compound is cyclized with elimination of water,
it being possible to introduce the substituents $R^1$, $R^2$, $R^3$, $R^4$, $R^5$, $R^6$, $R^7$, $R^8$, $R^{14}$, and $R^{15}$ into the compounds of the general formulae (II), (IIa), (III) and (IIIa) both before the condensation and after the cyclization to the compounds of the general formula (IV) by known methods, such as, for example, by alkylation, acylation, substitution, addition, elimination, rearrangement, oxidation, radical reaction or reduction and, if appropriate, subsequently to convert them into other functional groups,
or in that
[B] starting from the natural substance of the formula (Ib)

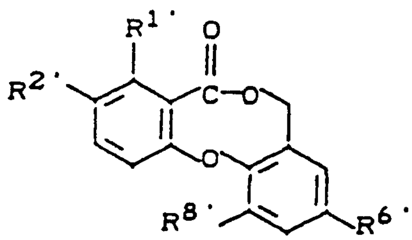

(Ib)

in which
$R^{1'}$ represents methoxy,
$R^{2'}$ represents the group

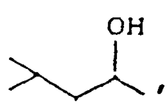

$R^{6'}$ represents methyl and
$R^{8'}$ represents hydroxyl,
the substituents $R^1$, $R^2$, $R^3$, $R^4$, $R^5$, $R^6$, $R^7$, $R^8$, $R^{14}$, and $R^{15}$ are introduced by the customary methods mentioned in process [A] such as, for example, rearrangement, alkylation, acylation, addition, elimination, oxidation, radical reaction or reduction, in inert solvents, if appropriate in the presence of auxiliaries, such as, for example, bases, acids, catalysts or activating reagents, and these, as also the substituents $R^{1'}$, $R^{2'}$, $R^{6'}$ and $R^{8'}$, are converted into other functional groups.

**2.** Process for the production of medicaments, characterized in that compounds of the general formula (I) according to Claim 1 are converted into a suitable form for administration, if appropriate using customary auxiliaries and excipients.

**Revendications**

**Revendications pour les Etats contactants suivants : AT, BE, CH, DE, DK, ES, FR, GB, IT, LI, LU, NL, SE**

1. Composés de formule générale (I)

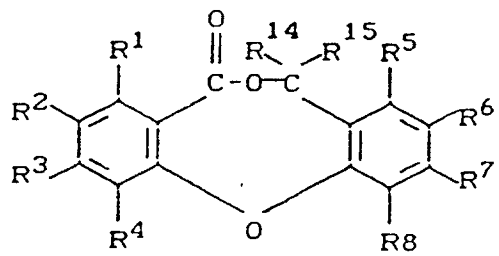

(I)

dans laquelle

$R^1$, $R^2$, $R^3$, $R^4$, $R^5$, $R^6$, $R^7$ et $R^8$ sont identiques ou différents et représentent chacun
- de l'hydrogène ou
- un groupe alkylthio, alkyle, alcényle ou alcynyle linéaire ou ramifié ayant chacun jusqu'à 12 atomes de carbone, qui sont substitués le cas échéant par un halogène, un groupe azido, imino, imino à substituant hydroxy, hydroxy, cyano, cycloalkyle ayant 3 à 8 atomes de carbone ou par un hétérocycle saturé ou non saturé de 3 à 7 chaînons ayant jusqu'à 4 hétéroatomes de la série azote, soufre ou oxygène, ou par un groupe aryle ou aryloxy ayant 6 à 10 atomes de carbone, qui peuvent être substitués de leur côté jusqu'à trois fois identiques ou différentes par un radical halogéno, nitro, phényle, phénoxy, cyano, alkyle, alkoxy ou alkoxycarbonyle linéaire ou ramifié ayant chacun jusqu'à 8 atomes de carbone, ou par un reste de formule

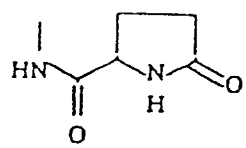

ou par un groupe de formule $-NR^9R^{10}$, $-COR^{11}$ ou $-OR^{12}$

où

$R^9$ et $R^{10}$ sont identiques ou différents et représentent chacun de l'hydrogène, un groupe alkyle ou alkoxy linéaire ou ramifié ayant jusqu'à 10 atomes de carbone, un groupe aryle ayant 6 à 10 atomes de carbone ou un groupe de formule $-S(O)_pR^{13}$,

$R^{11}$ représente l'hydrogène, un groupe hydroxy, alkyle linéaire ou ramifié ayant jusqu'à 10 atomes de carbone, phénoxy, aryle ayant 6 à 10 atomes de carbone ou le groupe $-NR^9R^{10}$,
dans lequel
$R^9$ et $R^{10}$ ont la définition indiquée ci-dessus,

$R^{12}$ est de l'hydrogène, un groupe cycloalkyle ayant 3 à 8 atomes de carbone, un groupe formyle, acyle ayant jusqu'à 8 atomes de carbone, tétrahydropyrannyle, trifluorométhoxy ou un groupe de formule $-S(O)_pR^{13}$,
dans laquelle

p est le nombre 1 ou 2,

$R^{13}$ - est un groupe alkyle linéaire ou ramifié ayant jusqu'à 8 atomes

de carbone ou le groupe $-NR^9R^{10}$,
dans lequel
$R^9$ et $R^{10}$ ont la définition indiquée ci-dessus,

ou bien

$R^{12}$ est un groupe alkyle ou alcényle linéaire ou ramifié ayant jusqu'à 10 atomes de carbone, qui est substitué le cas échéant par un radical hydroxy, halogéno, cycloalkyle de 3 à 8 atomes de carbone, alkoxy ou alkoxycarbonyle ayant jusqu'à 8 atomes de carbone ou par un radical aryle ayant 6 à 10 atomes de carbone, qui peut être substitué de son côté par un halogène, un radical hydroxy, alkyle, alkoxy ou alkoxycarbonyle ayant chacun jusqu'à 8 atomes de carbone, nitro ou cyano,
ou par un hétérocycle saturé ou non saturé de 3 à 7 chaînons ayant jusqu'à 4 hétéroatomes de la série soufre, oxygène ou azote ou par un groupe de formule $-NR^9R^{10}$, $-COR^{11}$ ou $-OR^{12}$,
où

$R^9$, $R^{10}$, $R^{11}$ et $R^{12}$ ont la définition indiquée ci-dessus,
ou par un reste de formule

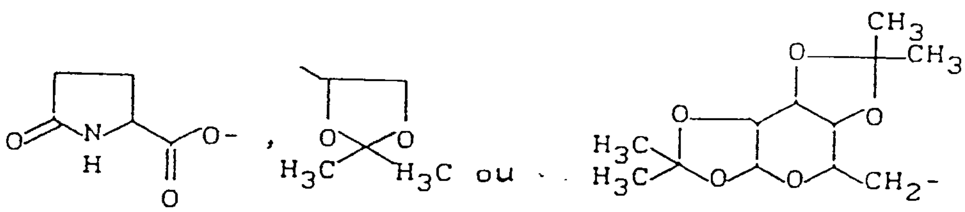

ou un reste aryle ayant 6 à 10 atomes de carbone, qui est substitué le cas échéant par un halogène, un radical hydroxy, nitro ou cyano,

ou bien

$R^1$, $R^2$, $R^3$, $R^4$, $R^5$, $R^6$, $R^7$ et $R^8$ sont identiques ou différents et représentent chacun

- un halogène, un groupe cyano, hydroxy, nitro,
- un hétérocycle de 3 à 7 chaînons ayant jusqu'à 4 hétéroatomes de la série soufre, oxygène ou azote, un groupe pyrid-3-yl-méthiodure ou
- un groupe aryle ayant 6 à 10 atomes de carbone, qui peut lui-même être substitué par un radical halogéno, cyano, phényle, nitro, alkyle ou alkoxy linéaire ou ramifié ayant chacun jusqu'à 8 atomes de carbone, ou hydroxy, ou bien
- un groupe cycloalcényle de 3 à 8 atomes de carbone,
- un groupe de formule $-NR^9R^{10}$, $-COR^{11}$ ou $-OR^{12}$,
  dans laquelle
  $R^9$, $R^{10}$, $R^{11}$ et $R^{12}$ ont la définition indiquée ci-dessus,

ou bien $R^1$ et $R^2$, $R^2$ et $R^3$, $R^3$ et $R^4$, $R^5$ et $R^6$, $R^6$ et $R^7$ ou $R^7$ et $R^8$ forment ensemble dans chaque cas un carbocycle pentagonal à heptagonal saturé ou non saturé qui est substitué le cas échéant par un radical nitro, cyano, hydroxy, alkyle linéaire ou ramifié ayant jusqu'à 8 atomes de carbone ou par un groupe de formule $-NR^9R^{10}$, $-COR^{11}$ ou $-OR^{12}$,

$R^{14}$ et $R^{15}$ sont identiques ou différents et représentent chacun

- de l'hydrogène, un groupe alkyle linéaire ou ramifié ayant jusqu'à 10 atomes de carbone, qui est substitué le cas échéant par un halogène, un radical nitro, cyano, hydroxy ou un groupe de formule
  $-NR^9R^{10}$, $-COR^{11}$ ou $-OR^{12}$
  où
  $R^9$, $R^{10}$, $R^{11}$ et $R^{12}$ ont la définition indiquée ci-dessus,
- un groupe aryle de 6 à 10 atomes de carbone, qui est substitué le cas échéant par un radical nitro, cyano, halogéno, alkyle, alkoxy ou alkoxycarbonyle ayant chacun jusqu'à

8 atomes de carbone ou par un groupe de formule $-NR^9R^{10}$,où

$R^9$ et $R^{10}$ ont la définition indiquée ci-dessus,

ou bien $R^{14}$ et $R^{15}$ forment ensemble un carbocycle pentagonal à heptagonal saturé ou non saturé et leurs sels acceptables du point de vue physiologique destinés à être utilisés pour combattre des maladies.

**2.** Composés de formule générale (I) suivant la revendication 1, dans laquelle

$R^1$, $R^2$, $R^3$, $R^4$, $R^5$, $R^6$, $R^7$ et $R^8$ sont identiques ou différents et représentent chacun

- de l'hydrogène ou
- un groupe alkylthio, alkyle, alcényle ou alcynyle linéaire ou ramifié ayant chacun jusqu'à 10 atomes de carbone, qui sont substitués le cas échéant jusqu'à 4 fois identiques ou différentes par les restes suivants, tels que fluoro, chloro, bromo, azido, imino, imino à substituant hydroxy, hydroxy, cyano, cyclopropyle, cyclopentyle, cyclohexyle, pyrryle, morpholino, pipéridyle ou par un reste oxiranyle ou par un reste phényle, qui peuvent être substitués quant à eux jusqu'à trois fois identiques ou différentes par un radical fluoro, chloro, bromo, iodo, nitro, phényle, phénoxy, cyano, alkyle, alkoxy ou alkoxycarbonyle linéaire ou ramifié ayant chacun jusqu'à 6 atomes de carbone, ou par un reste de formule

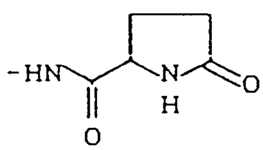

ou par un groupe de formule $-NR^9R^{10}$, $-COR^{11}$ ou $-OR^{12}$,

dans laquelle

$R^9$ et $R^{10}$ sont identiques ou différents et représentent chacun de l'hydrogène, un groupe alkyle linéaire ou ramifié ayant jusqu'à 8 atomes de carbone, phényle, ou un groupe de formule $-S(O)_pR^{13}$,

$R^{11}$ représente de l'hydrogène, un groupe hydroxy, alkyle ou alkoxy linéaire ou ramifié ayant jusqu'à 8 atomes de carbone, phénoxy, phényle ou le groupe $-NR^9R^{10}$,

dans lequel

$R^9$ et $R^{10}$ ont la définition indiquée ci-dessus,

$R^{12}$ représente de l'hydrogène, un groupe cyclopropyle, cyclobutyle, cyclopentyle, cyclohexyle, cycloheptyle, formyle, acyle ayant jusqu'à 6 atomes de carbone, tétrahydropyrannyle, trifluorométhoxy ou un groupe de formule $-S(O)_p-R^{13}$,

dans laquelle

p est le nombre 1 ou 2,

$R^{13}$ - est un groupe alkyle linéaire ou ramifié ayant jusqu'à 6 atomes de carbone ou le groupe $-NR^9R^{10}$,

dans lequel

$R^9$ et $R^{10}$ ont la définition indiquée ci-dessus,

ou bien

$R^{12}$ est un groupe alkyle ou alcényle linéaire ou ramifié ayant jusqu'à 8 atomes de carbone, qui est substitué le cas échéant jusqu'à 4 fois identiques ou différentes par les restes suivants, à savoir hydroxy, fluoro, chloro, bromo, cyclopropyle, cyclopentyle, cyclohexyle, alkoxy ou alkoxycarbonyle ayant jusqu'à 6 atomes de carbone ou par un reste phényle qui peut être substitué lui-même par un radical fluoro, chloro, bromo, hydroxy, alkyle, alkoxy ou alkoxycarbonyle ayant chacun jusqu'à 6 atomes de carbone, nitro ou cyano, ou substitué par un groupe oxiranyle, pyrimidyle, pyridyle ou par un groupe de formule $-NR^9R^{10}$, $-COR^{11}$ ou

$-OR^{12}$,
dans laquelle
$R^9$, $R^{10}$, $R^{11}$ et $R^{12}$ ont la définition indiquée ci-dessus,
ou substitué par un reste de formule

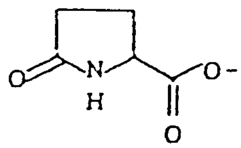
,
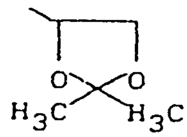
ou
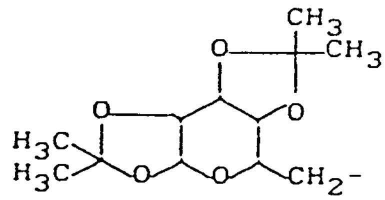

ou bien

$R^1$, $R^2$, $R^3$, $R^4$, $R^5$, $R^6$, $R^7$ et $R^8$ sont identiques ou différents et représentent chacun

- du fluor, du chlore, du brome, de l'iode, un groupe cyano, hydroxy, nitro, pyrid-3-yl-méthiodure, 2,3-dihydrofuryle, furyle, pyridyle, thiényle, dihydropyrannyle, thiazolyle, oxiranyle ou un groupe phényle, portant éventuellement des substituants fluoro, chloro, bromo, phényle, alkyle ou alkoxy linéaire ou ramifié ayant jusqu'à 6 atomes de carbone ou hydroxy, ou bien
- un groupe cyclobutényle, cyclopentényle ou cyclohexényle,
- un groupe de formule $-NR^9R^{10}$, $-COR^{11}$ ou $-OR^{12}$,
  dans laquelle
  $R^9$, $R^{10}$, $R^{11}$ et $R^{12}$ ont la définition indiquée ci-dessus,

ou bien $R^1$ et $R^2$, $R^2$ et $R^3$, $R^3$ et $R^4$, $R^5$ et $R^6$, $R^6$ et $R^7$ ou $R^7$ et $R^8$ forment ensemble un groupe phényle, cyclopentyle ou cyclohexyle,

$R^{14}$ et $R^{15}$ sont identiques ou différents et représentent chacun

- de l'hydrogène, un groupe alkyle linéaire ou ramifié ayant jusqu'à 8 atomes de carbone, qui est substitué le cas échéant par du fluor, du chlore, du brome, un radical cyano, hydroxy ou par un groupe de formule $-NR^9R^{10}$, $-COR^{11}$ ou $-OR^{12}$,
  dans laquelle
  $R^9$, $R^{10}$, $R^{11}$ et $R^{12}$ ont la définition indiquée ci-dessus,
  ou bien
- un groupe phényle qui est substitué le cas échéant par un radical nitro, cyano, fluoro, chloro, bromo, alkyle, alkoxy ou alkoxycarbonyle ayant chacun jusqu'à 6 atomes de carbone ou par le groupe de formule $-NR^9R^{10}$,
  dans laquelle

$R^9$ et $R^{10}$ ont la définition indiquée ci-dessus, et leurs sels acceptables du point de vue physiologique, destinés à être utilisés pour combattre des maladies.

**3.** Composés de formule générale (I) suivant la revendication 1,
dans laquelle

$R^1$, $R^2$, $R^3$, $R^4$, $R^5$, $R^6$, $R^7$ et $R^8$ sont identiques ou différents et représentent chacun

- de l'hydrogène ou
- un groupe alkylthio, alkyle, alcényle ou alcynyle linéaire ou ramifié ayant chacun jusqu'à 8 atomes de carbone, qui sont substitués le cas échéant jusqu'à 4 fois identiques ou différentes par les restes suivants, tels que fluoro, chloro, bromo, iodo, azido, imino, imino à substituant hydroxy, hydroxy, cyclopropyle, cyclopentyle, cyclohexyle, morpholino, pipéridyle, pyrryle ou par un reste oxiranyle ou par un reste phényle, qui peuvent être substitués quant à eux jusqu'à trois fois identiques ou différentes par un radical fluoro, chloro, phényle, phénoxy ou par un radical alkyle ou alkoxy linéaire ou ramifié ayant jusqu'à 4 atomes de carbone, ou substitués par un reste de formule

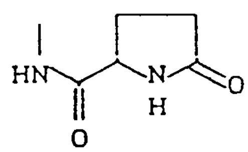

ou par un groupe de formule $-NR^9R^{10}$, $-COR^{11}$ ou $-OR^{12}$, où

$R^9$ et $R^{10}$ sont identiques ou différents et représentent chacun

- de l'hydrogène, un groupe alkyle linéaire ou ramifié ayant jusqu'à 6 atomes de carbone, un groupe phényle ou un groupe de formule $-S(O)_pR^{13}$,

$R^{11}$ est de l'hydrogène, un groupe hydroxy, trifluorométhyle, alkyle ou alkoxy linéaire ou ramifié ayant jusqu'à 6 atomes de carbone, phénoxy, phényle ou un groupe $-NR^9R^{10}$,
dans lequel
$R^9$ et $R^{10}$ ont la définition indiquée ci-dessus,

$R^{12}$ est de l'hydrogène, un groupe cyclopropyle, cyclopentyle, cyclohexyle, cycloheptyle, formyle, acyle ayant jusqu'à 4 atomes de carbone, tétrahydropyrannyle, trifluorométhoxy ou un groupe de formule $-S(O)_p-R^{13}$,
dans laquelle

p est le nombre 1 ou 2,

$R^{13}$ - est un groupe alkyle linéaire ou ramifié ayant jusqu'à 4 atomes de carbone,

ou un groupe alkyle ou alcényle linéaire ou ramifié ayant jusqu'à 6 atomes de carbone, qui est substitué le cas échéant jusqu'à 3 fois identiques ou différentes par les restes suivants, à savoir hydroxy, fluoro, chloro, bromo, cyclopropyle, cyclopentyle, cyclohexyle, alkoxy ou alkoxycarbonyle ayant jusqu'à 4 atomes de carbone ou par un groupe phényle qui peut être substitué quant à lui par un radical fluoro, chloro, hydroxy ou alkoxy ayant jusqu'à 4 atomes de carbone, ou substitué par un groupe oxiranyle ou par un groupe de formule $-NR^9R^{10}$, $-COR^{11}$ ou $-OR^{12}$,
dans laquelle
$R^9$, $R^{10}$, $R^{11}$ et $R^{12}$ ont la définition indiquée ci-dessus,
ou est substitué par un reste de formule

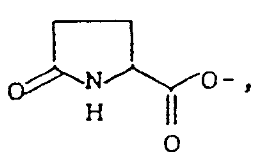 , 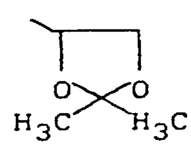 ou 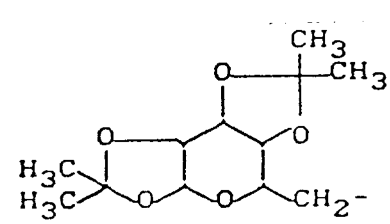

ou bien

$R^1$, $R^2$, $R^3$, $R^4$, $R^5$, $R^6$, $R^7$ et $R^8$ sont identiques ou différents et représentent chacun

- du fluor, du chlore, du brome, de l'iode, un radical hydroxy ou nitro ou un groupe phényle, pyrid-3-yl-méthiodure, 2,3-dihydrofuryle, pyrryle, pyridyle, thiényle, dihydropyrannyle, thiazolyle ou oxiranyle, qui peuvent être substitués quant à

eux par du fluor, du chlore, un radical méthyle, isopropyle, phényle ou éthoxy,

- un groupe de formule $-NR^9R^{10}$, $-COR^{11}$ ou $-OR^{12}$, dans laquelle $R^9$, $R^{10}$, $R^{11}$ et $R^{12}$ ont la définition indiquée ci-dessus,

$R^{14}$ et $R^{15}$ sont identiques ou différents et représentent de l'hydrogène, un groupe alkyle linéaire ou ramifié ayant jusqu'à 6 atomes de carbone ou un groupe phényle,

et leurs sels acceptables du point de vue physiologique, destinés à être utilisés pour combattre des maladies.

**4.** Composés de formule générale (I) suivant la revendication 1, dans laquelle $R^1$-$R^8$, $R^{14}$ et $R^{15}$ ont la définition indiquée dans la revendication 1, sous réserve que

a) $R^8$ ne puisse pas être un groupe hydroxy, méthoxy ou acétyle lorsque $R^1$ est un groupe méthoxy, $R^3$, $R^4$, $R^5$, $R^7$, $R^{14}$ et $R^{15}$ sont de l'hydrogène, $R^6$ est un groupe méthyle et $R^2$ est le groupe

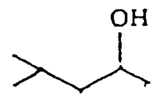

b) $R^5$ et $R^7$ ne puissent pas représenter du brome et $R^8$ ne puisse pas représenter un groupe hydroxy lorsque $R^1$ est un groupe méthoxy, $R^3$, $R^4$, $R^{14}$ et $R^{15}$ sont de l'hydrogène, $R^6$ est un groupe méthyle et $R^2$ est le groupe

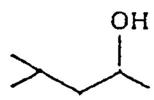

ou bien

c) $R^8$ ne puisse pas représenter un groupe méthoxy, lorsque $R^1$ est un groupe méthoxy, $R^3$, $R^4$, $R^5$, $R^7$, $R^{14}$ et $R^{15}$ représentent de l'hydrogène, $R^6$ est un groupe méthyle et $R^7$ représente le groupe

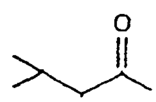

ou bien

d) $R^1$ ne puisse pas être un groupe méthoxy lorsque $R^3$, $R^4$, $R^5$, $R^6$, $R^{14}$ et $R^{15}$ représentent de l'hydrogène et $R^2$ est le groupe de formule

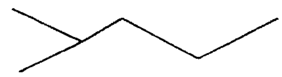

**5.** Utilisation de composés de formule générale (I) suivant la revendication 1 dans la préparation de compositions agissant sur la circulation.

**6.** Procédé de production de composés de formule générale (I)

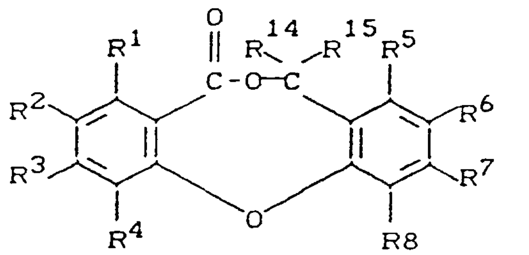

(I)

dans laquelle

$R^1$ à $R^{15}$ ont la définition donnée dans la revendication 1, caractérisé en ce que

[A] on condense des composés de formule générale (II)

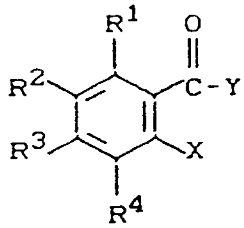

(II)

dans laquelle

$R^1$, $R^2$, $R^3$ et $R^4$ ont la définition indiquée ci-dessus,

X est du fluor, du chlore, du brome ou de l'iode

et

Y est un groupe alkoxy en $C_1$ à $C_6$ ou aryloxy ayant 6 à 10 atomes de carbone,

avec des composés de formule générale (III)

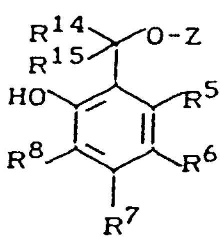

(III)

dans laquelle

$R^5$, $R^6$, $R^7$, $R^8$, $R^{14}$ et $R^{15}$ ont la définition indiquée ci-dessus

et

Z est un groupe typique protégeant la fonction hydroxyle tel que, par exemple, un groupe tétrahydropyrannyle,

ou des composés de formule générale (IIa)

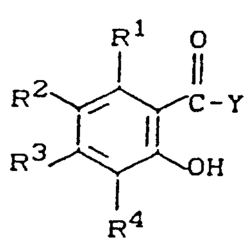

(IIa)

dans laquelle
$R^1$, $R^2$, $R^3$, $R^4$ et Y ont la définition indiquée ci-dessus, avec des composés de formule générale (IIIa)

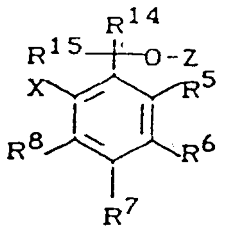

(IIIa)

dans laquelle $R^5$, $R^6$, $R^7$, $R^8$, $R^{14}$ et $R^{15}$, X et Z ont la définition indiquée ci-dessus,
avec élimination d'halogénures d'hydrogène tels que, par exemple, le bromure d'hydrogène, en composés de formule générale (IV)

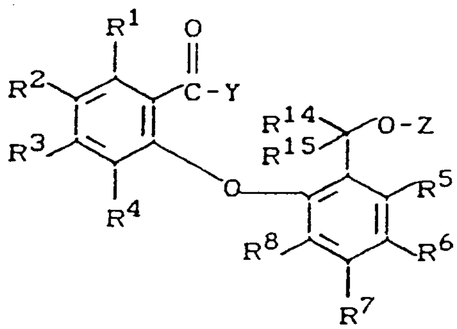

(IV)

dans laquelle $R^1$, $R^2$, $R^3$, $R^4$, $R^5$, $R^6$, $R^7$, $R^8$, $R^{14}$, $R^{15}$, Y et Z ont la définition indiquée ci-dessus
dans des solvants inertes, puis on enlève la protection du groupe hydroxyle par des moyens classiques et on effectue une cyclisation avec élimination d'eau,
les substituants $R^1$, $R^2$, $R^3$, $R^4$, $R^5$, $R^6$, $R^7$, $R^8$, $R^{14}$ et $R^{15}$ étant introduits aussi bien avant la condensation dans les composés de formules générales (II), (IIa), (III) et (IIIa) qu'après la cyclisation dans les composés de formule générale (IV) par des procédés connus, par exemple par alkylation, acylation, substitution, addition, élimination, transposition, oxydation, réaction radicalaire ou réduction et pouvant ensuite être transformés le cas échéant en d'autres groupes fonctionnels,
ou bien
[B] en partant de la substance naturelle de formule (Ib)

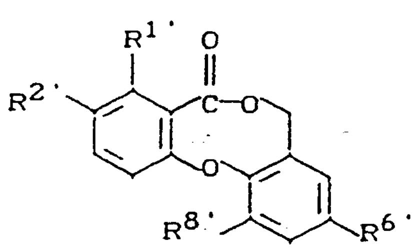

(Ib)

dans laquelle
- $R^{1'}$ est un groupe méthoxy,
- $R^{2'}$ est le groupe

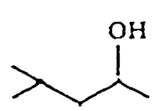

$R^{6'}$ est un groupe méthyle, et

$R^{8'}$ est un groupe hydroxy

on introduit les substituants $R^1$, $R^2$, $R^3$, $R^4$, $R^5$, $R^6$, $R^7$, $R^8$, $R^{14}$ et $R^{15}$ par les moyens classiques mentionnés pour le procédé [A], par exemple transposition, alkylation, acylation, addition, élimination, oxydation, réaction radicalaire ou réduction, dans des solvants inertes, éventuellement en présence de substances auxiliaires telles que, par exemple, des bases, des acides, des catalyseurs ou des réactifs activateurs et on transforme ces substituants ainsi que les substituants $R^{1'}$, $R^{2'}$, $R^{6'}$ et $R^{8'}$ en d'autres groupes fonctionnels.

**7.** Médicament contenant au moins un composé de formule générale (I) suivant la revendication 1.

**8.** Procédé de préparation de médicaments, caractérisé en ce qu'on fait passer sous une forme qui convient pour l'administration, des composés de formule générale (I) suivant la revendication 1, le cas échéant avec utilisation de substances auxiliaires et de supports classiques.

**9.** Le champignon de la souche Penicillium funiculosum Thom affecté du numéro de dépôt DSM 5249.

**10.** Utilisation du champignon suivant la revendication 9 pour la préparation de composés de formule générale (I) suivant la revendication 1.

**Revendications pour l'Etat contractant suivant : GR**

**1.** Procédé de production de composés de formule générale (I)

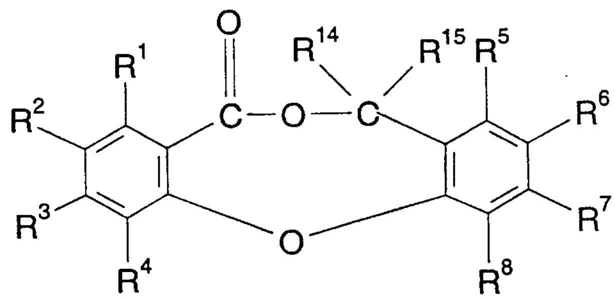

(I)

dans laquelle

$R^1$, $R^2$, $R^3$, $R^4$, $R^5$, $R^6$, $R^7$ et $R^8$ sont identiques ou différents et représentent chacun

- de l'hydrogène ou
- un groupe alkylthio, alkyle, alcényle ou alcynyle linéaire ou ramifié ayant chacun jusqu'à 12 atomes de carbone, qui sont substitués le cas échéant par un halogène, un groupe azido, imino, imino à substituant hydroxy, hydroxy, cyano, cycloalkyle ayant 3 à 8 atomes de carbone ou par un hétérocycle saturé ou non saturé de 3 à 7 chaînons ayant jusqu'à 4 hétéroatomes de la série azote, soufre ou oxygène, ou par un groupe aryle ou aryloxy ayant 6 à 10 atomes de carbone, qui peuvent être substitués de leur côté jusqu'à trois fois identiques ou différentes par un radical halogéno, nitro, phényle, phénoxy, cyano, alkyle, alkoxy ou alkoxycarbonyle linéaire ou ramifié ayant chacun jusqu'à 8 atomes de carbone, ou par un reste de formule

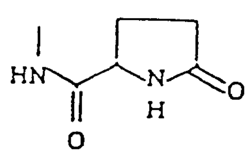

ou par un groupe de formule $-NR^9R^{10}$, $-COR^{11}$ ou $-OR^{12}$

où

$R^9$ et $R^{10}$ sont identiques ou différents et représentent chacun de l'hydrogène, un groupe alkyle ou alkoxy linéaire ou ramifié ayant jusqu'à 10 atomes de carbone, un groupe aryle ayant 6 à 10 atomes de carbone ou un groupe de formule $-S(O)_pR^{13}$,

$R^{11}$ représente l'hydrogène, un groupe hydroxy, alkyle linéaire ou ramifié ayant jusqu'à 10 atomes de carbone, phénoxy, aryle ayant 6 à 10 atomes de carbone ou le groupe $-NR^9R^{10}$,

dans lequel

$R^9$ et $R^{10}$ ont la définition indiquée ci-dessus,

$R^{12}$ est de l'hydrogène, un groupe cycloalkyle ayant 3 à 8 atomes de carbone, un groupe formyle, acyle ayant jusqu'à 8 atomes de carbone, tétrahydropyrannyle, trifluorométhoxy ou un groupe de formule $-S(O)_pR^{13}$,

dans laquelle

p est le nombre 1 ou 2,

$R^{13}$ - est un groupe alkyle linéaire ou ramifié ayant jusqu'à 8 atomes de carbone ou le groupe $-NR^9R^{10}$,

dans lequel

$R^9$ et $R^{10}$ ont la définition indiquée ci-dessus,

ou bien

$R^{12}$ est un groupe alkyle ou alcényle linéaire ou ramifié ayant jusqu'à 10 atomes de carbone, qui est substitué le cas échéant par un radical hydroxy, halogéno, cycloalkyle de 3 à 8 atomes de carbone, alkoxy ou alkoxycarbonyle ayant jusqu'à 8 atomes de carbone ou par un radical aryle ayant 6 à 10 atomes de carbone, qui peut être substitué de son côté par un halogène, un radical hydroxy, alkyle, alkoxy ou alkoxycarbonyle ayant chacun jusqu'à 8 atomes de carbone, nitro ou cyano,

ou par un hétérocycle saturé ou non saturé de 3 à 7 chaînons ayant jusqu'à 4 hétéroatomes de la série soufre, oxygène ou azote ou par un groupe de formule $-NR^9R^{10}$, $-COR^{11}$ ou $-OR^{12}$,

où

$R^9$, $R^{10}$, $R^{11}$ et $R^{12}$ ont la définition indiquée ci-dessus,

ou par un reste de formule

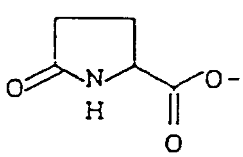 , 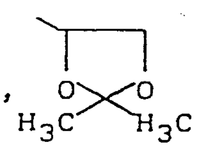 ou 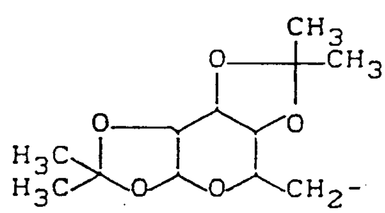

ou un reste aryle ayant 6 à 10 atomes de carbone, qui est substitué le cas échéant par un halogène, un radical hydroxy, nitro ou cyano,

ou bien

$R^1$, $R^2$, $R^3$, $R^4$, $R^5$, $R^6$, $R^7$ et $R^8$ sont identiques ou différents et représentent chacun

- un halogène, un groupe cyano, hydroxy, nitro,
- un hétérocycle de 3 à 7 chaînons ayant jusqu'à 4 hétéroatomes de la série soufre, oxygène ou azote, un groupe pyrid-3-yl-méthiodure ou
- un groupe aryle ayant 6 à 10 atomes de carbone, qui peut lui-même être substitué par un radical halogéno, cyano, phényle, nitro, alkyle ou alkoxy linéaire ou ramifié ayant chacun jusqu'à 8 atomes de carbone, ou hydroxy, ou bien
- un groupe cycloalcényle de 3 à 8 atomes de carbone,

- un groupe de formule $-NR^9R^{10}$, $-COR^{11}$ ou $-OR^{12}$, dans laquelle $R^9$, $R^{10}$, $R^{11}$ et $R^{12}$ ont la définition indiquée ci-dessus,

ou bien $R^1$ et $R^2$, $R^2$ et $R^3$, $R^3$ et $R^4$, $R^5$ et $R^6$, $R^6$ et $R^7$ ou $R^7$ et $R^8$ forment ensemble dans chaque cas un carbocycle pentagonal à heptagonal saturé ou non saturé qui est substitué le cas échéant par un radical nitro, cyano, hydroxy, alkyle linéaire ou ramifié ayant jusqu'à 8 atomes de carbone ou par un groupe de formule $-NR^9R^{10}$, $-COR^{11}$ ou $-OR^{12}$,

$R^{14}$ et $R^{15}$ sont identiques ou différents et représentent chacun

- de l'hydrogène, un groupe alkyle linéaire ou ramifié ayant jusqu'à 10 atomes de carbone, qui est substitué le cas échéant par un halogène, un radical nitro, cyano, hydroxy ou un groupe de formule $-NR^9R^{10}$, $-COR^{11}$ ou $-OR^{12}$ où $R^9$, $R^{10}$, $R^{11}$ et $R^{12}$ ont la définition indiquée ci-dessus,
- un groupe aryle de 6 à 10 atomes de carbone, qui est substitué le cas échéant par un radical nitro, cyano, halogéno, alkyle, alkoxy ou alkoxycarbonyle ayant chacun jusqu'à 8 atomes de carbone ou par un groupe de formule $-NR^9R^{10}$, où

$R^9$ et $R^{10}$ ont la définition indiquée ci-dessus, ou bien $R^{14}$ et $R^{15}$ forment ensemble un carbocycle pentagonal à heptagonal saturé ou non saturé,

et leurs sels acceptables du point de vue physiologique sous réserve que

a) $R^8$ ne puisse pas être un groupe hydroxy, méthoxy, ou acétyle lorsque $R^1$ est un groupe méthoxy, $R^3$, $R^4$, $R^5$, $R^7$, $R^{14}$ et $R^{15}$ sont de l'hydrogène, $R^6$ est un groupe méthyle et $R^2$ est le groupe

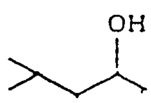

b) $R^5$ et $R^7$ ne puissent pas représenter du brome et $R^8$ ne puisse pas représenter un groupe hydroxy lorsque $R^1$ est un groupe méthoxy, $R^3$, $R^4$, $R^{14}$ et $R^{15}$ sont de l'hydrogène, $R^6$ est un groupe méthyle et $R^2$ est le groupe

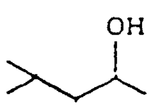

ou bien

c) $R^8$ ne puisse pas représenter un groupe méthoxy, lorsque $R^1$ est un groupe méthoxy, $R^3$, $R^4$, $R^5$, $R^7$, $R^{14}$ et $R^{15}$ représentent de l'hydrogène, $R^6$ est un groupe méthyle et $R^7$ représente le groupe

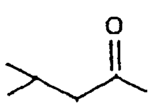

ou bien

d) $R^1$ ne puisse pas être un groupe méthoxy lorsque $R^3$, $R^4$, $R^5$, $R^6$, $R^{14}$ et $R^{15}$ représentent de l'hydrogène et $R^2$ est le groupe de formule

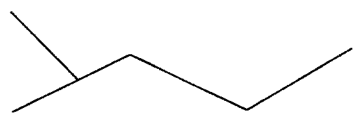

caractérisé en ce que

[A] on condense des composés de formule générale (II)

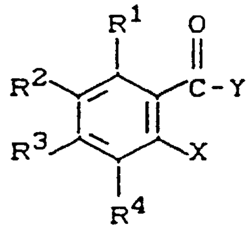

(II)

dans laquelle

$R^1$, $R^2$, $R^3$ et $R^4$ ont la définition indiquée ci-dessus,

X est du fluor, du chlore, du brome ou de l'iode

et

Y est un groupe alkoxy en $C_1$ à $C_6$ ou aryloxy ayant 6 à 10 atomes de carbone,

avec des composés de formule générale (III)

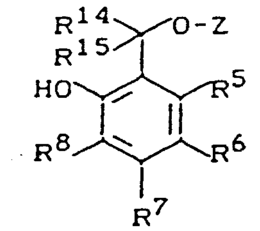

(III)

dans laquelle

$R^5$, $R^6$, $R^7$, $R^8$, $R^{14}$ et $R^{15}$ ont la définition indiquée ci-dessus

et

Z est un groupe typique protégeant la fonction hydroxyle tel que, par exemple, un groupe tétrahydropyrannyle,

ou des composés de formule générale (IIa)

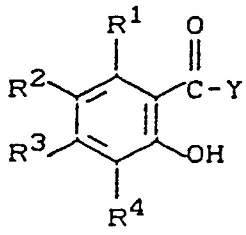

(IIa)

dans laquelle

$R^1$, $R^2$, $R^3$, $R^4$ et Y ont la définition indiquée ci-dessus, avec des composés de formule générale (IIIa)

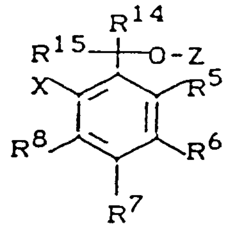

(IIIa)

dans laquelle $R^5$, $R^6$, $R^7$, $R^8$, $R^{14}$ et $R^{15}$, X et Z ont la définition indiquée ci-dessus, avec élimination d'halogénures d'hydrogène tels que, par exemple, le bromure d'hydrogène, en composés de formule générale (IV)

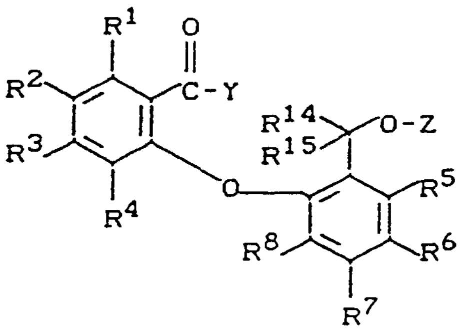

(IV)

dans laquelle
$R^1$, $R^2$, $R^3$, $R^4$, $R^5$, $R^6$, $R^7$, $R^8$, $R^{14}$, $R^{15}$, Y et Z ont la définition indiquée ci-dessus
dans des solvants inertes, puis on enlève la protection du groupe hydroxyle par des moyens classiques et on effectue une cyclisation avec élimination d'eau,
les substituants $R^1$, $R^2$, $R^3$ $R^4$, $R^5$, $R^6$, $R^7$, $R^8$, $R^{14}$ et $R^{15}$ étant introduits aussi bien avant la condensation dans les composés de formules générales (II), (IIa), (III) et (IIIa) qu'après la cyclisation dans les composés de formule générale (IV) par des procédés connus, par exemple par alkylation, acylation, substitution, addition, élimination, transposition, oxydation, réaction radicalaire ou réduction et pouvant ensuite être transformés le cas échéant en d'autres groupes fonctionnels,
ou bien
[B] en partant de la substance naturelle de formule (Ib)

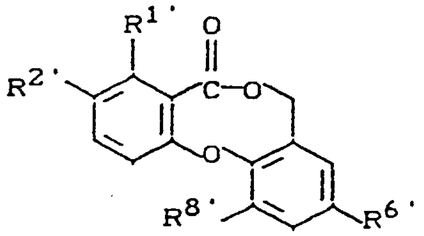

(Ib)

dans laquelle

$R^{1'}$ est un groupe méthoxy,

$R^{2'}$ est le groupe

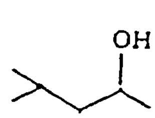

$R^{6'}$ est un groupe méthyle, et

$R^{8'}$ est un groupe hydroxy

on introduit les substituants $R^1$, $R^2$, $R^3$, $R^4$, $R^5$, $R^6$, $R^7$, $R^8$, $R^{14}$ et $R^{15}$ par les moyens classiques mentionnés pour le procédé (A), par exemple transposition, alkylation, acylation, addition, élimination, oxydation, réaction radicalaire ou réduction, dans des solvants inertes, éventuellement en présence de substances auxiliaires telles que, par exemple, des bases, des acides, des catalyseurs ou des réactifs activateurs et on transforme ces substituants ainsi que les substituants $R^{1'}$, $R^{2'}$, $R^{6'}$ et $R^{8'}$ en d'autres groupes fonctionnels.

**2.** Procédé de préparation de médicaments, caractérisé en ce qu'on fait passer sous une forme qui convient pour l'administration, des composés de formule générale (I) suivant la revendication 1, le cas échéant avec utilisation de substances auxiliaires et de supports classiques.